# EUROPEAN PATENT APPLICATION

(11) **EP 4 365 193 A1**
(43) Date of publication of application: **08.05.2024**
(21) Application number: 22832115.4
(22) Date of filing: 29.06.2022
(51) Int. Cl.: C07K 14/705, C12N 15/12, C12N 15/00, A61K 38/17, A61K 35/00

(54) **CHIMERIC POLYPEPTIDE FOR REGULATING CELL PHYSIOLOGICAL ACTIVITY**

(30) Priority: 29.06.2021 CN 202110730083; 09.09.2021 CN 202111056553; 29.01.2022 CN 202210111505
(71) Applicant: Carsgen Therapeutics Co., Ltd., Xuhui District Shanghai 200231 (CN)
(72) Inventor: WANG, Yi, Shanghai 200231 (CN); LI, Zonghai, Shanghai 200231 (CN)
(74) Representative: Lavoix
(86) International application number: PCT/CN2022/102395
(87) International publication number: WO 2023/274303

(57) **Abstract**

A chimeric polypeptide, containing a binding peptide that can specifically bind to a target molecule, a receptor regulatory domain containing one or more cleavage sites, and an intracellular domain. The receptor regulatory domain comprises an extracellular region and a transmembrane region, wherein the extracellular region and the transmembrane region are not both derived from a Notch protein. The binding of the binding peptide to the target molecule can induce the cleavage of the receptor regulatory domain, thereby releasing the intracellular domain.

## Description

### PRIORITY INFORMATION

This application claims the benefit of Chinese patent application CN202110730083.X filed on June 29, 2021, Chinese patent application CN202111056553.5 filed on September 9, 2021, and Chinese patent application CN202210111505.X filed on January 29, 2022, the entire contents of which are incorporated herein by reference in their entirety.

### SEQUENCE LISTING FILES FILED AT THE SAME TIME

The entire content of the following ASCII text file is incorporated herein by reference in its entirety: Sequence Listing in Computer Readable Format (CRF) (name: FF00617PCT-sequence listing-20220629-yzg.txt, date: 20220629, size: 232KB).

### TECHNICAL FIELD

The present application relates to a chimeric polypeptide capable of regulating cell physiological activity, and a cell expressing the chimeric polypeptide.

### BACKGROUND ART

There are many ways to regulate the physiological activities of cells such as gene expression and cell differentiation in vitro, but how to realize the space-specific regulation of cells in vivo is still a technical difficulty. For example, after some immune cells exogenously express anti-tumor molecules (such as CAR, cytokines, etc.), cells can be activated and produce strong anti-tumor effects, but at the same time, it also brings potential risks of off-target toxicity and dysfunction caused by long-term activation of cells.

Now there has been the first generation synthetic derivative of Notch receptor SynNotch, comprising the ligand binding domain, Notch core regulatory region and intracellular signaling region. The principle is to replace the intracellular region of natural Notch with artificial signaling molecules (such as specific transcription factors, etc.) by utilizing the characteristics of occurrence of shearing extracellularly and intracellularly in successive following natural Notch molecules binding to target molecules (ligands) and cutting extracellularly and intracellular, basically achieving the Ligand-dependent gene expression regulation.

However, there are still many problems in this technology: the synNotch has a background leak, that is, there is still weak gene expression in the state of no ligand activation; the level of gene expression induced by synNotch is low; the synNotch has is a long sequence, large molecule and difficult cell engineering.

### SUMMARY OF THE INVENTION

The present application relates to the following contents.

A chimeric polypeptide, comprising:
a) a binding peptide capable of specifically binding to a target molecule;
b) a receptor regulatory domain comprising one or more cleavage sites,
   wherein, the receptor regulatory domain comprises an extracellular region and a transmembrane region which are not both derived from a Notch protein; and
c) an intracellular domain,
wherein, binding of the binding peptide to the target molecule can induce cleavage of the receptor regulatory domain, thereby releasing the intracellular domain.

In an embodiment, the chimeric polypeptide is characterized in that the cleavage site is an enzymolysis site, preferably, a protease hydrolysis site.

In an embodiment, the chimeric polypeptide is characterized in that the transmembrane region of the receptor regulatory domain comprises a cleavage site, and when the binding peptide segment binds to the target molecule, cleavage and release of the intracellular domain is triggered.

In an embodiment, the chimeric polypeptide is characterized in that the transmembrane region comprises an I-CLiPs (intramembranously cleaving proteases) enzyme cleavage site; preferably, the I-CLiPs comprises a γ-secretase cleavage site, more preferably, the γ-secretase cleavage site comprises a γ-secretase cleavage site of a Gly-Val dipeptide sequence.

In an embodiment, the extracellular region of the receptor regulatory domain of the chimeric polypeptide comprises a cleavage site, and when the peptide segment specifically binding to a target molecule binds to the target molecule, cleavage of the extracellular region is triggered, and thus cleavage and release of the intracellular domain are triggered.

In an embodiment, the cleavage site of the extracellular region of the chimeric polypeptide is a sheddase protease cleavage site; preferably, the sheddase protease is selected from the group consisting of: BACE1, ADAM8, ADAM9, ADAM10, ADAM12, ADAM17 and MT1-MMP.

In an embodiment, the extracellular region of the chimeric polypeptide is derived from an extracellular region of Jagged2, EphrinB2, APLP1, APLP2, APP, CD44, CSF1R, CXCL16, CX3CL1, Delta1, E-cadherin, EphB2, EphrinB1, Growth hormone receptor, HLA-A2, IFNaR2, IL1R2, L1, LRP, LRP2, LRP6, N-cadherin, Nectin1α, NRADD, p75-NTR, Pcdh α4, Pcdh γ-C3, PTPκ, PTP-LAR, SorCS1b, SorLA, Sortilin, ApoER2, PKHD1, ErbB4, IFNaR2, VEGF-R1, or VLDLR, or a fragment of extracellular region of any one of the above proteins, or a variant of the extracellular region of any one of the above proteins.

In an embodiment, the extracellular region of the chimeric polypeptide comprises the extracellular region of Jagged2 or the extracellular region of EphrinB2, or a fragment of the extracellular region of Jagged2 or a fragment of the extracellular region of EphrinB2.

In an embodiment, the extracellular region of the chimeric polypeptide comprises an amino acid sequence having at least 80% sequence homology to any one of SEQ ID NOs: 1, 3, 5, 7, 9, 11.

In an embodiment, the intracellular domain of the chimeric polypeptide comprises a protein fragment of any protein selected from the group consisting of: a transcription activator; a transcriptional repressor; a transcriptional co-activator; a transcriptional co-repressor; a DNA-binding polypeptides; a RNA-binding polypeptides; a translation-regulating polypeptide; a hormone; a cytokine; a toxin; an antibody; a chromatin regulator; a suicide protein; an organelle-specific polypeptide (such as, nuclear pore regulator, mitochondrial regulator, endoplasmic reticulum regulator, etc.); a pro-apoptotic polypeptide; an anti-apoptotic polypeptide; other polypeptides that promote cell death by other mechanisms; a pro-proliferative polypeptide; an anti-proliferative polypeptide; an immune co-stimulatory polypeptide; a site-specific nuclease; a recombinant enzyme; an inhibitory immune receptor; an activating immune receptor; variants of Cas9 and RNA-targeting nucleases; and a DNA recognition peptide; a signal transduction polypeptide; a receptor tyrosine kinase; a non-receptor tyrosine kinase; and a polypeptide promoting differentiation;

Preferably, the intracellular domain comprises a protein fragment of any protein selected from the group consisting of: a transcription activator, a transcription repressor, a site-specific nuclease, a recombinase, an inhibitory immune receptor, and an activating immune receptor.

In an embodiment, the transcriptional activator is GLA4-VP64 or a fragment thereof; preferably, the transcriptional activator contains the sequence shown in SEQ ID NO: 27.

In an embodiment, the intracellular domain of the chimeric polypeptide is a site-specific nuclease, preferably, the site-specific nuclease is a Cas9 polypeptide.

In an embodiment, the receptor regulatory domain of the chimeric protein comprises an amino acid sequence having at least 80% sequence homology to any one of SEQ ID NOs: 28, 29, 30, 31, 32, 33, 34, 35, 36, 37, 38, 39, 40, 41, or 85.

In an embodiment, the binding peptide segment is an antibody, an antigen, a ligand, a receptor, a cell adhesion molecule, or a non-antibody molecular scaffold.

In an embodiment, the binding peptide segment is an antibody, and the antibody is a single-domain antibody, a single-chain antibody, a double-chain antibody, a triple-chain antibody, or a mini antibody.

In an embodiment, the non-antibody molecular scaffold is an avimer, a DARPin, an adnectin, an avimer, an affibody, an anticalin or an affilin.

In an embodiment, the target molecule is a ligand present in a pathological tissue and/or a normal tissue of a human.

In an embodiment, the pathological tissue is a tumor tissue, an infectious lesion tissue, or a gene mutation tissue.

In an embodiment, the tumor tissue comprises a tumor cell, an organ containing a tumor cell, and a tumor microenvironment.

In an embodiment, the target molecule is specifically expressed or highly expressed in a pathological tissue and/or a normal tissue of a specific human.

In an embodiment, the transmembrane region is a transmembrane region from notch; preferably, the transmembrane region is shown in SEQ ID NOs: 13, 14, 15, 16, 17, 18, 19, 20, 21, 102, 103, or 104, or an amino acid sequence having at least 80% sequence homology to any one of SEQ ID NOs: 13, 14, 15, 16, 17, 18, 19, 20, 21, 102, 103, or 104.

In an embodiment, the receptor regulatory domain has 50-300 amino acids.

In an embodiment, there is a linker peptide between the receptor regulatory domain and the recognition domain.

In an embodiment, the chimeric polypeptide comprises, form N-terminus to C-terminus, a binding peptide, a receptor regulatory domain and an intracellular domain.

The present application also relates to a cell expressing the chimeric polypeptide described above. In an embodiment, the cell is an immune effector cell.

In an embodiment, the cell is a T cell, a NK cell, a NKT cell, a macrophage, a CIK cell, and a stem cell-derived immune effector cell.

In an embodiment, the cell also contains a nucleic acid expressing another chimeric polypeptide or T cell receptor.

In an embodiment, said another chimeric polypeptide is a chimeric antigen receptor (CAR), a chimeric T cell receptor, or a T cell antigen coupler (TAC).

In an embodiment, the release of the intracellular domain of the chimeric polypeptide can activate expression of another chimeric polypeptide or a T cell receptor.

The present application relates to a nucleic acid, an expression vector, and a viruse for preparing of the cell of the present application.

In an embodiment, the nucleic acid is constructed in one expression vector.

The application relates to a method for activating a cell, the method comprising:
contacting the cell of the present application described above with an immobilized antigen, wherein the binding peptide segment comprising the chimeric polypeptide comprises an antibody specific for a first antigen, and wherein said contacting results in release of the transcriptional activator comprised in the chimeric polypeptide, to regulate expression of the CAR and/or TCR in the cell, wherein the CAR and/or TCR provides activation of the cell following binding to the second antigen.

In an embodiment, the first antigen and/or the second antigen have antigen expression heterogeneity.

In an embodiment, the first antigen and/or the second antigen are a tumor antigen.

In an embodiment, the method is used to treat a tumor with expression heterogeneity of the first antigen.

The application relates to a method for activating cells, the method comprising:
contacting the cell of the present application described above with the target molecule, wherein the binding peptide segment comprisingthe chimeric polypeptide comprises an antibody specific for a first target molecule, and wherein said contacting results in release of the transcriptional activator comprised in the chimeric polypeptide, to regulate expression of the CAR and/or TCR in the cell, wherein the CAR and/or TCR provides activation of the cell following binding to the second antigen.

In an embodiment, the first target molecule and/or the second target molecule have antigen expression heterogeneity.

In an embodiment, the first target molecule and/or the second target molecule are a tumor antigen.

In an embodiment, the method is used to treat a tumor with heterogeneous expression of the first target molecule.

In an embodiment, the target molecule is an insoluble molecule.

In an embodiment, the first target molecule comprises a cell membrane surface antigen.

In an embodiment, the cell include a T cell, a NK cell, a NKT cell, a macrophage, a CIK cell, a stem cell-derived immune effector cell, or a combination thereof.

The present application relates to a method for regulating secretion of cytokine IL-12, the method comprising:
In an embodiment, contacting the cell of the present application with an immobilized antigen, wherein the binding peptide segment comprising the chimeric polypeptide comprises an antibody specific for an antigen, and wherein said contacting results in release of the transcriptional activator comprised in the chimeric polypeptide, to regulate expression of the IL-12 in the cell.

In an embodiment, the cell is a T cell.

In an embodiment, the antigen comprises a tumor antigen.

The present application relates to a method for regulating secretion of cytokines IL-12, IL-7, CCL21 or a combination thereof, the method comprising:
contacting the cell of the present application with an target molecule, wherein the binding peptide segment comprising the chimeric polypeptide comprises an antibody specific for the target molecule, and wherein said contacting results in release of the transcriptional activator comprised in the chimeric polypeptide, to regulate expression of the IL-12, IL-7, CCL21 or a combination thereof in the cell.

In an embodiment, the cell comprises a T cell, a NK cell, a NKT cell, a macrophage, a CIK cell, a stem cell-derived immune effector cell, or combinations thereof.

In an embodiment, the target molecule includes a tumor antigen.

In an embodiment, the target molecule is an insoluble molecule.

In an embodiment, the target molecule includes a cell membrane surface antigen.

It should be understood that within the scope of the present application, the above-mentioned technical features of the present application and the technical features specifically described herein below (such as embodiments) may be combined with each other to form new or preferred technical solutions. Due to space limitations, they are not described herein one by one.

### BRIEF DESCRIPTION OF THE DRAWINGS

FIGs. 1A and 1B show that after co-incubation with liver cancer cells expressing GPC3, respectively, synJagged2EC, synEphrinB2EC, synEphrinB2EC-APLP2(TM) targeting GPC3 induce levels of BFP expression which are comparable to or significantly higher than that of synNotch.
FIG. 2 shows that after co-incubation of synNotch, synEphrinB2EC chimeric polypeptide with coated antigen can trigger transcriptional activity.
FIG. 3 shows that all of chimeric polypeptides targeting GPC3, comprising full-length of EphrinB2 extracellular region or a truncation thereof regulate gene expression.
FIG. 4 shows that when the antigen concentration is low (less than or equal to 0.78125 µg/mL), the cells containing the chimeric polypeptide remain quiescent, and under the stimulation of a high concentration of antigen (greater than or equal to 0.3125 µg/mL), the cells containing the chimeric polypeptide are activated.
FIG. 5 shows that after co-incubation with liver cancer cells expressing GPC3, synJagged2EC, synEphrinB2EC induce comparable or increased IL12 expression compared to synNotch.
FIG. 6 shows the expression levels of GPC3 in different liver cancer cells.
FIG. 7 shows that the expression level of synEphrinB2EC and synEphrinB2EC-APLP2(TM) in the single vector system is higher than that of synNotch.
FIG. 8 shows that synEphrinB2EC or synEphrinB2EC-APLP2(TM) induced higher transcription than synNotch after incubation with GPC3 low-expressing cells.
FIG. 9 shows that after incubation with GPC3 low-expression cells, the induced expression level of synEphrinB2EC-del3 is low, while after incubation with GPC3 high-expression cells, its induction expression level is comparable to that of synNotch.
FIG. 10 shows that synEphrinB2EC regulating the IL12 expression plays a synergistic anti-tumor effect with GPC3-CAR-T cells.
FIG. 11 shows that after co-incubation with glioma cells U87MG-EGFRvIII and U251-EGFRvIII expressing EGFRvIII, cells expressing synEphrinB2EC targeting tumor antigen EGFRvIII induce level of BFP expression close to that of cells expressing synNotch chimeric polypeptide, however, antigen-independent activation occurred in cells expressing synNotch chimeric polypeptide.
FIG. 12 shows that after human primary T cells were prepared to T cells expressing synEphrinB2EC-CAR and synNotch-CAR targeting tumor antigen EGFRvIII, the positive rate and expression level of synEphrinB2EC were higher than those of synNotch.
FIG. 13 shows that synEphrinB2EC-CAR-T cells do not express CAR and have no spontaneous CAR signal in the absence of antigen stimulation.
FIG. 14 shows that synEphrinB2EC-CAR-T cells are in a low activation and poor differentiation state in the absence of antigen stimulation.
FIG. 15 shows that synEphrinB2EC-CAR-T cells are in a low-exhaustion state in the absence of antigen stimulation.
FIG. 16 shows that synEphrinB2EC-CAR-T cells expressing targeting tumor antigen EGFRvIII can be activated by EGFRvIII and kill IL13Ra2-positive glioma cells. Compared with synNotch-CAR T cells, synEphrinB2EC-CAR-T cells are more effective in killing tumors with strong antigen express heterogeneity.
FIG. 17 shows the anti-tumor effect of IL13Ra2-CAR-T expressing EGFRvIII-synEphrinB2EC in vivo.
FIG. 18 shows that 376.96-28Z-T cells that recognize only human B7H3 have significant anti-tumor effects, while B7H3-28Z-T and B7H3-BBZ-T that recognize human and mouse B7H3 have poor efficacy. When the B7H3-28Z with low specificity is put under the regulation of EGFRvIII-synEphrinB2EC, its specific killing effect can be improved.
FIGs. 19 and 20 show that both the second and fourth generation of Claudin18.2-CAR-T cells expressing Mesothelin-synEphrinB2EC can be specifically activated by antigens to kill tumor cells.
FIG. 21 shows that Claudin18.2-CAR-T cells expressing FAP-synEphrinB2EC are anti-tumor, and the body weights of mice do not decrease significantly.
FIG. 22A and FIG. 22B show that Mesothelin-CAR-T cells containing Claudin18.2-synEphrinB2EC can be specifically activated by antigens to kill tumor cells.
FIG. 23A shows the expression levels of CLL1 and NKG2D in AML cells; FIG. 23B shows the transcriptional activity triggered by binding of chimeric polypeptides targeting CLL1.
FIG. 24 shows that NKG2D-CAR-T cells kill THP1 and HL-60 cells expressing NKG2D ligands, and CD3Z-NKG2D-CAR-T cells induced by CLL1-synEphrinB2EC have a weak killing effect on the THP-1 cells with low CLL1 expression, and can only kill HL-60 cells with high expression of both CLL1 and NKG2D ligands.
FIG. 25 shows that T cells containing CLL1-synEphrinB2EC regulating NKG2D-CAR expanded well.
FIG. 26 shows that the death ratio of T cells containing CLL1-synEphrinB2EC regulating NKG2D-CAR is basically the same as that of UTD, while the death ratio of NKG2D-CAR-T cells is significantly increased; T cells containing CLL1-synEphrinB2EC regulating NKG2D-CAR can effectively guarantee expansion and activity of T cells in culture system.

### DETAILED DESCRIPTION

The present application found a new class of chimeric polypeptides having a transcriptional regulatory activity triggered by specifically binding to a target molecule (a ligand, or referred to as a target antigen). An extracellular region and a transmembrane region of a receptor regulatory domain of the chimeric polypeptide are not both derived from Notch, and the transmembrane region comprises one or more cleavage sites. The transcription regulation activity of the chimeric polypeptide of the present application triggered by specifically binding to a target molecule (a ligand, or referred to as a target antigen) is comparable to or higher than the transcription regulation activity of synNotch triggered by specifically binding to a target molecule (a ligand, or referred to as a target antigen). The present application provides a chimeric polypeptide having a binding-triggered transcriptional switch, a nucleic acid encoding the chimeric polypeptide, and a host cell genetically modified with the nucleic acid. The present application provides a transgenic organism comprising a nucleic acid encoding a chimeric polypeptide having binding-triggered transcriptional regulatory activity. Also provided is a method of locally regulating the activity of cells using one or more chimeric polypeptides having binding-triggered transcriptional regulatory activity and a localized cell activation system using one or more chimeric polypeptides having binding-triggered transcriptional regulatory activity.

The chimeric polypeptide provided by the application comprises from N-terminus to C-terminus: (a) a binding domain capable of specifically binding a target molecule, (b) a receptor regulatory domain comprising one or more cleavage sites, an extracellular region and a transmembrane region of the receptor regulatory domain are different from the Notch receptor polypeptide, and c) the intracellular domain; wherein binding of the binding domain to the target molecule can induce cleavage of the receptor regulatory domain , thereby releasing the intracellular domain.

Exemplarily, the present application provides chimeric polypeptides synJagged2EC, synEphrinB2EC, synEphrinB2EC-APLP2(TM), nucleic acids encoding the chimeric polypeptides of the present application, and host cells genetically modified with nucleic acids. The present application provides a transgenic organism comprising a nucleic acid encoding a chimeric polypeptide synJagged2EC, synEphrinB2EC, synEphrinB2EC-APLP2(TM).

Binding of engineered cells expressing the chimeric polypeptide designed in the present application (such as synJagged2EC, synEphrinB2EC, synEphrinB2EC-APLP2(TM)) to tumor cells expressing a tumor antigen (such as GPC3, EGFRvIII, Mesothelin, FAP, Claudin18.2, CLL1, CD123) can trigger transcriptional regulatory activity. In an embodiment, expression of BFP, expression of cytokine IL12 or CAR is transcriptionally regulated. In an embodiment, levels of BFP, IL12, and CAR expression induced by cells expressing synEphrinB2EC, synJagged2EC or synEphrinB2EC-APLP2(TM) are close to or significantly higher than those induced by cells expressing synNotch chimeric polypeptide.

Pro-inflammatory cytokines such as IL12 have been proven to have a strong function of promoting tumor immunity of T cell, but the risk in clinical application is relatively high due to their potential toxic side effects. The use of the chimeric polypeptide provided by the present application to regulate the specific expression of pro-inflammatory cytokines at tumor site can effectively solve the problem of their side effects. In a specific embodiment, the level of IL12 expression induced by cells expressing synEphrinB2EC, synJagged2EC or synEphrinB2EC-APLP2(TM) is close to or significantly higher than that induced by cells expressing synNotch chimeric polypeptide. In a specific embodiment, synEphrinB2EC is more sensitive to a target molecule (ligand, or target antigen) with a low level of expression, and has a stronger ability to induce gene expression. Exemplarily, after specifically binding to the tumor antigen with a low level of expression, the ability of synEphrinB2EC to induce IL12 expression is significantly higher than that of synNotch, up to about 4-6 times. Exemplarily, the level of BFP induced by synEphrinB2EC activated by tumor antigen EGFRvIII is comparable to that of synNotch, but non-specific activation also occurs after synNotch is co-incubated with U87 or U251 cells without tumor antigen EGFRvIII, suggesting that the chimeric polypeptide synEphrinB2EC has a strong ability of gene induction, meanwhile has good antigen specificity and higher safety.

The chimeric polypeptides provided in the present application are not activated by a soluble antigen. Exemplarily, the soluble antigen is unable to activate cells expressing synEphrinB2EC, synJagged2EC, or synEphrinB2EC-APLP2(TM) chimeric polypeptides. In a specific embodiment, the soluble antigen refers to an antigen that is dissolved and free in the extracellular environment.

The receptor regulatory domain of the chimeric polypeptides provided in the present application (such as synEphrinB2EC, synJagged2EC, synEphrinB2EC-APLP2(TM)) is shorter than the corresponding domain of synNotch. When synEphrinB2EC is integrated into the same lentiviral vector with the gene to be regulated for expression, the gene regulated by synEphrinB2EC can have a larger capacity in sequence length. In a specific embodiment, the infection positive rate of T cell comprising the intracellular domain and the binding domain specifically binding to the target molecule (ligand, or referred to target antigen) prepared by the chimeric polypeptide synEphrinB2EC is about 1.5 times higher than that of synNotch, reducing the difficulty of industrial production.

The chimeric polypeptides provided in the present application (such as synEphrinB2EC, synJagged2EC, synEphrinB2EC-APLP2(TM)) improve the anti-tumor effect of CAR-T cells on the tumor with antigen expression heterogeneity or strong antigen heterogeneity by binding to trigger the regulation of CAR expression. Exemplary, after contacting cells expressing the chimeric polypeptide of the present application (such as synEphrinB2EC, synJagged2EC, synEphrinB2EC-APLP2(TM)) with target cells expressing a first target molecule (exemplary, tumor antigen EGFRvIII), the transcriptional regulation of the chimeric antigen receptor (CAR) targeting a second target molecule (exemplary, II,13Ra2) can be triggered, to achieve targeted killing of tumor cells expressing the second target molecule when and only when the first target molecule exists in the tumor microenvironment, improving the tumor specificity of CAR-T cells. Not only that, since the simultaneous expression of the first target molecule and the second target molecule in a single target cell is not an essential condition for initiating targeted killing, the present application can reduce the occurrence of target cell escape effect and cell killing in target cells expressing the second target molecule by loss or reduction of the expression of the first target molecule. In a specific embodiment, when the positive rate of the first target molecule is low, synNotch targeting the first target molecule cannot activate CAR-T cells to effectively kill tumor cells, while synEphrinB2EC, synJagged2EC, and synEphrinB2EC-APLP2(TM) targeting the first target molecule provided in the present application can still activate CAR-T cells to kill tumor cells.

For the chimeric polypeptides (such as synEphrinB2EC, synJagged2EC, synEphrinB2EC-APLP2(TM)) with binding-triggered regulation of CAR expression provided in the present application, in the absence of target molecule stimulation, the CAR is not be expressed or no phosphorylation of CAR occurs; in the inactivated state, the expression of CD25 and CD69 is maintained at a low level; in the state of low depletion, the expression of PD1, LAG3, TIM3 and CD39 is maintained at a low level; in the state of low differentiation, most cells are Tscm cells.

The chimeric polypeptides with binding-triggered regulation of CAR expression provided in the present application can realize space-specific regulation of CAR-T cells killing target cells, reduce off-target toxicity, dysfunction caused by long-term activation of cells and activation of non-specific target molecules, and reduce the difficulty of industrial preparation high-efficiency virus packaging. The present application also provides a composition and method for producing such receptors, nucleic acids encoding them, host cells genetically modified with these nucleic acids, and a method for modulating cellular activity and/or for treating various health conditions (e.g., a disease (such as a tumor)).

Unless specifically defined, all technical and scientific terms used herein have the meaning commonly understood by those skilled in the fields of cell biology, cell culture, gene therapy, biochemistry, microbiology, recombinant DNA, immunology, genetics and molecular biology. Such techniques are explained fully in the literature. See, for example, Current Protocols in Molecular Biology (FrederickM. AUSUBEL, 2000, Wiley and son Inc, Library of Congress, USA); Molecular Cloning: A Laboratory Manual, Third Edition, (Sambrooke et al, 2001, Cold Spring Harbor, New York: Cold Spring Harbor Laboratory Press); Oligonucleotide Synthesis (M.J.Gaited., 1984); Mullis et al.U.S.Pat.No.4,683,195; Nucleic Acid Hybridization (B.D.Harries & S.J.Higginseds. 1984);Transcription And Translation (B.D.Hames&S.J.Higginseds 1984); Culture Of Animal Cells (R.I.Freshney, Alan R.Liss, Inc., 1987); Immobilized Cells And Enzymes (IRL Press, 1986); B.Perbal, A Practical Guide To Molecular Cloning (1984); the series, Methods In ENZYMOLOGY (J. Abelson and M. Simon, eds.-in-chief, Academic Press, Inc., New York), especially Vols. 154 and 155 (Wuetal.eds.) and Vol. 185, "Gene Expression Technology" (D.Goeddel, ed.); Gene Transfer Vectors For Mammalian Cells (J.H.Miller and M.P.Caloseds., 1987, Cold Spring Harbor Laboratory); Immunochemical Methods In Cell And Molecular Biology (Mayer and Walker, eds., Academic Press , London, 1987); Hand book Of Experimental Immunology, Volumes I-IV (D.M. Weir and C.C. Blackwell, eds., 1986) and Manipulating the Mouse Embryo (Cold Spring Harbor Laboratory Press, Cold Spring Harbor, N.Y, 1986). All methods and materials similar or equivalent to those described herein may be used in the practice or testing of the present application, in which suitable methods and materials are described herein. All publications, patent applications, patents, and other references mentioned herein are incorporated by reference in their entirety. In case of conflict, the present specification including definitions shall prevail. In addition, the materials, methods, and examples in the present application are illustrative only and not intended to be limiting unless otherwise specified. According to the present application, those skilled in the art should appreciate that many changes and modifications may be made in the specific embodiments which are disclosed and the same or similar results are still obtained without departing from the spirit and scope of the present application. The present application is not limited in scope to the specific embodiments described herein, which are intended only as illustrations of various aspects of the present application, and functionally equivalent methods and components are within the scope of the present application. The present application includes variations and modifications of the subject of the present application for various uses and conditions.

### 1. Definition:

As used herein, "about" can indicate that it depends upon the specific situation and is known or knowable by one skilled in the art, or represents a variation within a range of up to approximately ± 1%, ± 2%, ± 3%, ± 4%, ± 5%, ± 6%, ± 7%, ± 8%, ± 9%, ± 10%, ± 11%, ± 12%, ± 13%, ± 14%, ± 15%, ± 16%, ± 17%, ± 18%, ± 19%, ± 20%, ± 25%, ± 30% of a given value. That is, the range indicated by "about" covers a given value± 1%, ± 2%, ± 3%, ± 4%, ± 5%, ± 6%, ± 7%, ± 8%, ± 9%, ± 10%, ± 11%, ± 12%, ± 13%, ± 14%, ± 15%, ± 16%, ± 17%, ± 18%, ± 19%, ± 20%, ± 25%, ± 30%. Alternatively, particularly with regard to biological systems or methods, the term may mean within an order of magnitude of a value, such as within about 5 times or within about 2 times a value.

Ranges: The description in range format is merely for convenience and brevity and should not be construed as an inflexible limitation on the scope of the application. Accordingly, the description of the range should be considered to specifically disclose all possible subranges as well as individual numerical values within that range. For example, the description of a range from 1 to 6 should be considered to specifically disclose subranges from 1 to 3, 1 to 4, 1 to 5, 2 to 4, 2 to 6, 3 to 6, etc., and the specific numerical points within these ranges, such as 1, 2, 3, 4, 5, 6. The above principles apply equally regardless of the breadth or narrowness of the numerical values stated. When a range is used for description, that range includes the endpoints of the range.

The term "receptor" is a kind of special protein or polypeptide that exists in the cell membrane or in the cell, can bind to the target molecule and activate a series of biochemical reactions in the cell, and so that the cell produce corresponding effects on external stimuli. The target molecules (also referred to as biologically active substances) that bind to receptors are collectively referred to as ligands, or target antigens.

The term "chimeric antigen receptor" or "CAR" refers to an engineered molecule that can be expressed by immune cells, including but not limited to T cells. CARs are expressed in T cells and can redirect T cells to induce specific killing of target cells determined by the chimeric receptors. CAR comprises an antigen-binding domain, a transmembrane domain, and an intracellular signaling domain. The intracellular signaling domain comprises a primary signaling domain and/or a co-stimulatory signaling domain. The extracellular binding domain of the CAR may be derived from murine, humanized or complete human monoclonal antibodies. The term CAR is not specifically limited to the CAR molecule, but also includes a CAR variant. The CAR variant comprises a split CAR in which the extracellular portion (e.g., the ligand-binding portion) and the intracellular portion (e.g., the intracellular signaling portion) of the CRA are present on two separate molecules. The CAR variant also comprises an ON-switch CAR, which is conditionally activated CAR, including, for example, the split CAR, in which conditional heterodimerization of the two portions of the split CAR is controlled by a medicine. The CAR variant also comprises a bispecific CAR that comprise a secondary CAR-binding domain that can amplify or inhibit the activity of the primary CAR. The CAR variant also include an inhibitory chimeric antigen receptor (iCAR), which can, for example, be used as a component of bispecific CAR system, where binding of the secondary CAR binding domain results in inhibition of primary CAR activation.

The term "engineering" refers to using the principles and methods of cell biology and molecular biology to change the genetic material in cells or obtain cell products at the level of cells or organelles through some engineering means. Engineered cells can also refer to as cells that contain added, deleted and/or altered genes.

The term "cell" or "engineered cell" may refer to as a human or non-human animal derived cell. The terms "individual" and "subject" are interchangeable and include a human or an animal from other species, which include, but not limited to, a humans, a mouse, a rat, a hamster and a guinea pig, a rabbit, a dog, a cat, a sheep, a pig, a goat, a cow, a horse, an ape, a monkey.

The term "transfection" refers to the introduction of exogenous nucleic acid into a eukaryotic cell. Transfection can be achieved by various means known in the art, including calcium phosphate-DNA co-precipitation, DEAE-dextran-mediated transfection, polybrene-mediated transfection, electroporation, microinjection, liposome fusion, lipofection, protoplast fusion, retrovirus infection and biolistics.

The term "nucleic acid" or "polynucleotide" or "nucleic acid molecule" refers to a single-stranded or double-stranded deoxyribonucleic acid (DNA) or ribonucleic acid (RNA) and polymers thereof, including any nucleic acid molecule encoding a polypeptide of interest or a fragment thereof. The nucleic acid molecule only needs to maintain substantial identity to the endogenous nucleic acid sequence, and does not need to have 100% homology or identity to the endogenous nucleic acid sequence. A polynucleotide having "substantial identity" to the endogenous sequence can generally hybridize with at least one strand of a double-stranded nucleic acid molecule. "Hybridization" refers to the formation of the pairing of double-stranded molecules between complementary polynucleotide sequences, or portions thereof, under various stringent conditions. The term "homology" or "identity" refers to the identity of subunit sequence between two polymer molecules, for example, between two nucleic acid molecules, such as two DNA molecules or two RNA molecules, or between two polypeptide molecules. The term "substantial identity" or "substantial homology" refers to a polypeptide or nucleic acid molecule that exhibits at least about 50% homology or identity to a reference amino acid sequence or nucleic acid sequence. In an embodiment, such a sequence has at least about 60%, 65%, 70%, 75%, 80%, 85%, 90%, 95%, 99%, or 100% homology or identity with the amino acid or nucleic acid sequence used for comparison. Sequence identity can be measured by using sequence analysis software (e.g., the BLAST, BESTFIT, GAP or PILEUP/PRETTYBOX programs). Such software matches identical or similar sequences by assigning degrees of homology to various substitutions, deletions and/or other modifications. Conservative substitutions typically include substitutions in glycine, alanine; valine, isoleucine, leucine; aspartic acid, glutamic acid, asparagine, glutamine; serine, threonine, lysine, arginine; and phenylalanine, tyrosine. In an exemplary method of determining the degree of identity, the BLAST program may be used, wherein a probability score between e-3 and e-100 is indicative of closely related sequences.

The term "isolated" means changed or removed from the natural state. For example, a nucleic acid or peptide naturally present in a living animal is not "isolated", but the same nucleic acid or peptide that is partially or completely separated from a substance co-existing in its natural state is "isolated." The isolated nucleic acid or protein may exist in a substantially purified form or may exist in a non-natural environment such as a host cell.

The term "operably linked" refers to a physical or functional linkage between two or more sequences (e.g., polypeptide sequences or polynucleotide sequences) which permits them to operate in an intended manner. For example, a promoter is operably linked to a coding sequence if the promoter affects the transcription or expression of the coding sequence. Generally, operably linked DNA sequences are contiguous and, where necessary, join two protein coding regions in the same reading frame.

The terms "peptide", "polypeptide" and "protein" are used interchangeably, and refer to as a compound consisting of amino acid residues covalently linked by peptide bonds.

The term "activation of immune cells" means a change in intracellular protein expression caused by a signal transduction pathway results in the initiation of an immune response. In an embodiment, the immune synapse formed following the CAR binding to the antigen comprises the aggregation of many molecules near the binding receptor (e.g., CD4 or CD8, CD3γ/CDδ/CDε/CDζ, etc.). This aggregation of membrane binding signaling molecules phosphorylates the ITAM motif included in the CD3 molecule. This phosphorylation in turn initiates the T cell activation pathway, ultimately activating transcription factors such as NF-κB and AP-1. These transcription factors induce the overall gene expression of T cells, including the upregulation of IL-2 production, the promotion of T cell proliferation, and thus the initiation of T cell-mediated immune response. "T cell activation" or "T cell activating" refers to the state of T cells that are stimulated to induce detectable cell proliferation, cytokine production, and/or detectable effector function. In an embodiment, the immune cells are activated after being co-incubated with cells containing a specific antigen, or the immune cells are activated after being infected with a virus.

The term "antigen" or "Ag" refers to a molecule that causes an immune response, including, but not limited to, tumor antigens and pathogen antigens. Any tumor antigen can be used in the tumor-related embodiments described herein ("example" and "embodiment" may be used interchangeably herein). The tumor antigens of the present invention include but are not limited to: thyroid stimulating hormone receptor (TSHR); CD171; CS-1; C-type lectin-like molecule-1; ganglioside GD3; Tn antigen; CD19; CD20; CD 22; CD 30; CD 70; CD 123; CD 138; CD33; CD44; CD44v7/8; CD38; CD44v6; B7H3 (CD276), B7H6; KIT (CD117); interleukin 13 receptor subunit α (IL-13Rα); interleukin 11 receptor alpha (IL-11Rα); prostate stem cell antigen (PSCA); prostate specific membrane antigen (PSMA); carcinoembryonic antigen (CEA); NY-ESO-1; HIV-1 Gag; MART-1; gp100; tyrosinase; mesothelin; EpCAM; protease serine 21 (PRSS21); vascular endothelial growth factor receptor, vascular endothelial growth factor receptor 2 (VEGFR2); Lewis (Y) antigen; CD24; platelet derived growth factor receptor β (PDGFR-β); stage-specific embryonic antigen-4 (SSEA-4); cell surface-associated mucin 1 (MUC1), MUC6; epidermal growth factor receptor family and its mutants (EGFR, EGFR2, ERBB3, ERBB4, EGFRvIII); neural cell adhesion molecule (NCAM); carbonic anhydrase IX (CAIX); LMP2; ephrin A receptor 2 (EphA2); fucosyl GM1; sialyl Lewis adhesion molecule (sLe); ganglioside GM3(aNeu5Ac(2-3)bDGalp(1-4)bDGlcp(1-1)Cer; TGS5; high molecular weight melanoma-associated antigen (HMWMAA); O-acetyl GD2 ganglioside (OAcGD2); folate receptor; tumor vascular endothelial marker 1 (TEM1/CD248); tumor vascular endothelial marker 7 related (TEM7R); Claudin 6, Claudin 18.2, Claudin 18.1; ASGPR1; CDH16; 5T4; 8H9; αvβ6 integrin; B cell maturation antigen (BCMA); CA9; kappa light chain; CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1; Sp17; SURVIVIN; TAG72; TEM1; fibronectin; tenascin; carcinoembryonic variant of tumor necrosis zone; G protein coupled receptor C class 5 group-member D (GPRC5D); chromosome X open reading frame 61 (CXORF61); CD97; CD179a; anaplastic lymphoma kinase (ALK); polysialic acid; placenta specific 1 (PLAC1); hexose part of globoH glycoceramide (GloboH); breast differentiation antigen (NY-BR-1); uroplakin 2 (UPK2); hepatitis A virus cell receptor 1 (HAVCR1); adrenergic receptor β3 (ADRB3); pannexin 3 (PANX3); G protein-coupled receptor 20 (GPR20); lymphocyte antigen 6 complex locus K9 (LY6K); olfactory receptor 51E2 (OR51E2); TCRγ alternating reading frame protein (TARP); wilms tumor protein (WT1); ETS translocation variant gene 6 (ETV6-AML); sperm protein 17 (SPA17); X antigen family member 1A (XAGE1); angiopoietin binding to cell surface receptor 2 (Tie2); melanoma cancer testis antigen-1 (MAD-CT-1); melanoma cancer testis antigen-2 (MAD-CT-2); Fos related antigen 1; p53 mutant; human telomerase reverse transcriptase (hTERT); sarcoma translocation breakpoint; melanoma inhibitor of apoptosis (ML-IAP); ERG (transmembrane protease serine 2 (TMPRSS2) ETS fusion gene); N-acetylglucosaminyl transferase V (NA17); pairing box protein Pax-3 (PAX3); androgen receptor; cyclin B1; V-myc avian myelocytomatosis virus oncogene neuroblastoma derived homolog (MYCN); Ras homolog family member C (RhoC); cytochrome P450 1B1 (CYP1B1); CCCTC binding factor (zinc finger protein)-like (BORIS); squamous cell carcinoma antigen 3 recognized by T cells (SART3); pairing box protein Pax-5 (PAX5); proacrosin binding protein sp32 (OYTES1); lymphocyte-specific protein tyrosine kinase (LCK); A kinase anchoring protein 4 (AKAP-4); synovial sarcoma X breakpoint 2 (SSX2); CD79a; CD79b; CD72; leukocyte-associated immunoglobulin-like receptor 1 (LAIR1); Fc fragment of IgA receptor (FCAR); leukocyte immunoglobulin-like receptor subfamily member 2 (LILRA2); CD300 molecular-like family member f (CD300LF); C-type lectin domain family 12 member A (CLEC12A); bone marrow stromal cell antigen 2 (BST2); EGF-like module containing mucin-like hormone receptor like 2 (EMR2); lymphocyte antigen 75 (LY75); glypican-3 (GPC3); Fc receptor-like 5 (FCRL5); immunoglobulin lambda-like polypeptide 1 (IGLL1). Pathogen antigens include but are not limited to: antigens of viruses, bacteria, fungi, protozoa, or parasite; viral antigens include but are not limited to: cytomegalovirus (CMV) antigens, Epstein-Barr virus (EBV) antigens, human immune defective virus (HIV) antigens or influenza virus antigens.

The term "antigen binding domain" refers to a molecule that specifically binds an antigenic determinant, including immunologically active portion of an immunoglobulin molecule and immunomolecule, i.e., including a molecule that contains an antigen binding site that specifically binds ("immunoreacts") to an antigen. The term "antibody" includes not only intact antibody molecules but also fragments of antibody molecules that retain antigen-binding ability. The term "antibody" is used interchangeably with the term "immunoglobulin" and "antigenic domain" in this application. The antibody includes, but not limited to monoclonal antibody, polyclonal antibody, natural antibody, bispecific antibody, chimeric antibody, Fv, Fab, Fab', Fab'-SH, F(ab')₂, linear antibody, single chain antibody (e.g. scFv), single domain antibody. In an embodiment, the antibody comprises at least two heavy (H) chains and two light (L) chains linked by disulfide bonds. Each heavy chain is composed of a heavy chain variable region (VH) and a heavy chain constant region (CH). CH consists of three domains CH1, CH2, and CH3. Each light chain consists of a light chain variable region (VL) and a light chain constant region (CL). CL consists of one domain. VH and VL may be further subdivided into hypervariable regions referred to as complementarity determining regions (CDRs), interspersed with more conserved regions referred to as framework regions (FRs). Each VH and VL consists of three CDRs and four FRs, arranged in the following order from amino-terminus to carboxy-terminus: FR1, CDR1, FR2, CDR2, FR3, CDR3, FR4. The variable regions of the heavy and light chains include binding domains that interact with antigens. The constant regions of the antibody mediate the binding of the immunoglobulins to host tissues or factors, which include various cells of the immune system (e.g., immune cells) and the first component (Clq) of the classical complement system. An antigenic domain "specifically binds" or is "immunogenic" to an antigen if the antigenic domain binds to the antigen with greater affinity (or avidity) than other reference antigens (comprising polypeptides or other substances).

The terms "therapeutically effective amount", "therapeutically effective", "effective amount" or "in an effective amount" are used interchangeably herein and refer to as the amount of a compound, preparation, substance or composition that is effective to achieve a specific biological result as described herein, such as but not limited to an amount or dose sufficient to promote T cell response. An effective amount of immune cells refers to, but is not limited to, the number of immune cells which can increase, enhance or prolong the anti-tumor activity; increase the number of anti-tumor immune cells or activated immune cells; the number of immune cells which promote IFN-γ secretion, tumor regression and tumor shrinkage and tumor necrosis.

The term "promoter" used herein is defined as a DNA sequence recognized by the synthesis mechanism of the cell required initiating the specific transcription of a polynucleotide sequence or by an introduced synthesis mechanism.

The term "endogenous" means that nucleic acid molecules or polypeptides etc. come from the organism itself.

The term "exogenous" means that a nucleic acid molecule or polypeptide is not endogenously present in the cell, or is not expressed at a level sufficient to achieve the function obtained by overexpressed; encompasses any recombinant nucleic acid molecule or polypeptide expressed in the cell, such as an exogenous, heterologous and overexpressed nucleic acid molecules and polypeptides.

The term "recognize" refers to selective binding of a target antigen. In the present application, the immune cells expressing the exogenous receptor can recognize the cells expressing the antigen to which the exogenous receptor specifically binds.

The term "specifically binds" refers to an antibody or ligand that recognizes and binds a protein of a binding partner (such as a tumor antigen) present in a sample, but the antibody or ligand does not substantially recognize or bind to other molecules in the sample.

The term "vector" used herein refers to a composition which contains an isolated nucleic acid and can be used to deliver the isolated nucleic acid to the inside of a cell. Many vectors are known in the art, including but not limited to linear polynucleotides, polynucleotides associated with ionic or amphiphilic compounds, plasmids, and viruses. Therefore, the term "vector" includes autonomously replicating plasmids or viruses, and further include non-plasmid and non-viral compounds that facilitate the transfer of nucleic acids into cells, such as transposon vectors, polylysine compounds, liposomes, and the like. Virus vectors include, but are not limited to, adenoviral vectors, adeno-associated virus vectors, retroviral vectors, and the like.

The term "disease" refers to any disorder that damages or interferes with the normal function of a cell, tissue or organ, such as a tumor (cancer) or pathogen infection. Refractory cancers include, but are not limited to, cancers that are insensitive to radiotherapy, relapsed after radiotherapy, insensitive to chemotherapy, relapsed after chemotherapy, insensitive to CAR-T therapy, or relapsed after treatment.

The term "tumor" refers to a disease characterized by the pathological hyperplasia of cells or tissues, and their subsequent migration or invasion of other tissues or organs. Tumor cell growth is often uncontrolled and progressive, characterized by migration and loss of contact inhibition. Tumors include cancers and their precancerous lesions. Tumors include hematological tumors, solid tumors, or metastatic lesions thereof.

The term "antigen expression heterogeneity or antigen heterogeneity" means that cells in a cell population express different antigens, or that the same antigen is only expressed in some cells of the cell population.

### 2. Chimeric polypeptide

The chimeric polypeptide in the present application is a receptor that regulates transcriptional activity in a target molecule (ligand, or referred to target antigen) dependent manner. The chimeric polypeptide in the present application is a recombinant, non-naturally occurring receptor, comprising a binding domain, a receptor regulatory domain and an intracellular domain.

In an embodiment, binding of the chimeric polypeptide to a target molecule triggers hydrolysis of the chimeric polypeptide, releasing the intracellular domain. In an embodiment, binding of the chimeric polypeptide to a target molecule (such as a tumor antigen) displayed on the surface of the target cell triggers hydrolysis of the chimeric polypeptide, releasing the intracellular domain. Exemplarily, after the chimeric polypeptide binds to a tumor antigen on the surface of a tumor cell, it regulates a transcription factor that tailors the transcriptional program in the cell.

The chimeric polypeptide in the present application comprises from N-terminus to C-terminus: (a) a binding domain capable of specifically binding a target molecule, (b) a receptor regulatory domain comprising one or more cleavage sites, in which an extracellular region and a transmembrane region of the receptor regulatory domain are not both derived from Notch, and c) an intracellular domain; in which, the binding of the binding domain to the target molecule can induce the cleavage of the receptor regulatory domain, releasing the intracellular domain. The binding domain and intracellular domain that can specifically bind to a target molecule are heterologous to a Notch receptor polypeptide.

The chimeric polypeptides in the present application have better regulatory transcriptional activity than existing SynNotch receptors and provide a more modular platform for engineering. Existing SynNotch receptors can be engineered by ligand binding domains such as single-chain antibodies and nanobodies, but it is difficult to use the native extracellular domains of receptors/ligands on SynNotch receptors. The chimeric polypeptides in the present application are suitable for use with other types of ligand binding domains, thereby expanding the prospect of being able to target diseases and tissues.

### 2.1 Receptor regulatory domain

The receptor regulatory domain of the chimeric polypeptide comprises an extracellular region and a transmembrane region (also referred to a transmembrane domain). The receptor regulatory domain comprises one or more ligand-induced proteolytic cleavage sites, and the cleavage site is selected from an I-CLiPs (intramembranously cleaving proteases) cleavage site or a sheddase protease cleavage site. I-CLiPs are transmembrane cleaving proteases that can catalyze the hydrolysis of specific sites on the transmembrane region of transmembrane proteins. In an embodiment, the I-CLiPs comprise a γ-secretase cleavage site. In an embodiment, the γ-secretase cleavage site comprises a γ-secretase cleavage site of a Gly-Val dipeptide sequence. In an embodiment, the sheddase protease is selected from BACE1, ADAM8, ADAM9, ADAM10, ADAM12, ADAM17, MT1-MMP or combinations thereof.

In an embodiment, one or more ligand-induced proteolytic cleavage sites comprised in the receptor regulatory domain are located in the transmembrane region, and the cleavage sites are selected from I-CLiPs cleavage sites. In an embodiment, the extracellular region comprises one or more ligand-induced sheddase proteolytic cleavage sites, and the transmembrane region comprises the I-CLiPs enzyme cleavage site. In an embodiment, the cleavage site of the extracellular region is the sheddase protease cleavage site. In an embodiment, the transmembrane region comprises the I-CLiPs enzyme cleavage site.

In an embodiment, the extracellular region comprises one or more cleavage sites. In an embodiment, the transmembrane region comprises one or more cleavage sites. In an embodiment, the extracellular region comprises one or more cleavage sites and the transmembrane region comprises one or more cleavage sites. In an embodiment, the extracellular region does not comprise cleavage sites. In an embodiment, the transmembrane region comprises one or more cleavage sites. In an embodiment, the extracellular region does not comprise cleavage sites, and the transmembrane region comprises one or more cleavage sites.

### 2.1.1 Transmembrane region

In an embodiment, the transmembrane region is a single-transmembrane receptor transmembrane region comprising at least one γ-secretase cleavage site. In an embodiment, the transmembrane region includes but is not limited to CLSTN1, CLSTN2, APLP1, APLP2, LRP8, APP, BTC, TGBR3, SPN, CD44, CSF1R, CXCL16, CX3CL1, DCC, DLL1, DSG2, DAG1, CDH1, EPCAM, EPHA4, EPHB2, EFNB1, EFNB2, ErbB4, GHR, HLA-A and IFNAR2 transmembrane regions, in which the transmembrane region comprise at least one γ-secretase cleavage site. In an embodiment, transmembrane region includes but is not limited to IL1R1, IL1R2, IL6R, INSR, ERN1, ERN2, JAG2, KCNE1, KCNE2, KCNE3, KCNE4, KL, CHL1, PTPRF, SCN1B, SCN3B, NPR3, NGFR, PLXDC2, PAM, AGR, ROBO1, SORCS3, SORCS1, SORL1, SDC1, SDC2, SPN, TYR, TYRP1, DCT, VASN, FLT1, CDH5, PKHD1, NECTIN1, PCDHGC3, NRG1, LRP1B, CDH2, NRG2, PTPRK, SCN2B, Nradd and PTPRM transmembrane regions.

In an embodiment, the transmembrane region comprises a Notch1 transmembrane region, a Notch2 transmembrane region, a Notch3 transmembrane region, or a Notch4 transmembrane region from a human or non-human animal (e.g., mouse, zebrafish, Drosophila, Xenopus laevis, or Gallus). In an embodiment, the transmembrane region comprises a fragment having at least 70%, 75%, 80%, 85%, 90%, 95%, 96%, 97%, 98%, 99% or 100% homology or identity to the amino acid sequence as shown in SEQ ID NO:13, 14, 15, 16, 17, 18, 19, 20, 102, 103, or 104, and/or optionally comprises at most 1, 2, 3, 4, 5 or more amino acid residues substituted with different amino acid residues. In an embodiment, amino acid residues other than "GV" in SEQ ID NO: 13, 14, 15, 17, 18, 19, 102, 103 or 104 are replaced by different amino acid residues.

In an embodiment, the transmembrane region comprises APLP1 transmembrane region or APLP2 transmembrane region from a human or non-human animal. In an embodiment, the transmembrane region comprises a fragment having at least 70%, 75%, 80%, 85%, 90%, 95%, 96%, 97%, 98%, 99% or 100% homology or identity to the amino acid sequence as shown in SEQ ID No: 21, and/or optionally comprises at most 1, 2, 3, 4, 5 or more amino acid residues substituted with different amino acid residues.

In an embodiment, the carboxyl terminal of the transmembrane region comprises a stop transfer sequence (STS). The STS connects the intracellular domain of the chimeric polypeptide and prevents it from entering the endoplasmic reticulum lumen. In an embodiment, The STS comprises about 4 to 10 residues, such as 4, 5, 6, 7, 8, 9, or 10 amino acid residues. In an embodiment, the STS comprises a sequence having at least 70%, 75%, 80%, 85%, 90%, 95%, 96%, 97%, 98%, 99% or 100% identity to the STS of Notch1, Notch2, Notch3, Notch4, CLSTN1, CLSTN2, CSF1R, CXCL16, DAG1, GHR, PTPRF, AGR, KL, NRG1, LRP1B, Jag2, EPCAM, KCNE3, CDH2, NRG2, PTPRK, BTC, EPHA3, IL1R2 or PTPRM. In an embodiment, the STS comprises a sequence that contain only Lys(K) or Arg(R) of the first four residues. In an embodiment, the STS comprises a fragment having at least 70%, 75%, 80%, 85%, 90%, 95%, 96%, 97%, 98%, 99%, or 100% homology or identity to the amino acid sequence as shown in SEQ ID NOs: 22, 23, 24, 25, or 26, and/or optionally comprises at most 1, 2, 3, 4, 5 or more amino acid residues replaced with different amino acid residues.

### 2.1.2 Extracellular region

In an embodiment, the extracellular region comprises extracellular full length of Jagged2, EphrinB2, APLP1, APLP2, APP, CD44, CSF1R, CXCL16, CX3CL1, Delta1, E-cadherin, EphB2, EphrinB1, Growth hormone receptor, HLA-A2, IFNaR2, IL1R2, L1, LRP, LRP2, LRP6, N-cadherin, Nectin1α, NRADD, p75-NTR, Pcdhα4, Pcdhγ-C3, PTPκ, PTP-LAR, SorCS1b, SorLA, Sortilin, ApoER2, PKHD1, ErbB4, IFNaR2, VEGF-R1, or VLDLR, or a fragment of the extracellular region of any one of the above proteins or a truncation or truncated structure thereof, or a variant of the extracellular region of any one of the above proteins. In an embodiment, the extracellular region of the above protein is full length. In an embodiment, the extracellular region of the receptor regulatory domain of the chimeric polypeptide comprises the full length of the extracellular region of the above-mentioned EphrinB2EC or Jagged2 respectively, and the chimeric polypeptide each can be triggered to cleave and release the intracellular domain after specifically binding to the target molecule,

The fragment of the extracellular region of the protein mentioned in the present application does not comprise the complete extracellular region, but only comprises part of the extracellular region of the protein. In an embodiment, the chimeric polypeptide comprising a fragment of the extracellular domain of EphrinB2 is triggered to cleave and release the intracellular domain of the chimeric polypeptide after specifically binding to a target molecule. In an embodiment, the fragment of the extracellular region of EphrinB2 comprises a fragment with the potential ADAM10 cleavage site removed from the extracellular region of EphrinB2, such as EphrinB2EC-del23 and EphrinB2EC-del3. In an embodiment, the fragment of the extracellular region of EphrinB2 comprises a fragment with near-N-terminum removed from the extracellular region of EphrinB2, such as EphrinB2EC-del1. In an embodiment, the fragment of the extracellular region of EphrinB2 comprises a fragment with near-C-terminum removed from the extracellular region of EphrinB2, such as EphrinB2EC-del3. In an embodiment, the fragment of the extracellular region of EphrinB2 comprises a fragment in which the middle sequence of the extracellular region of EphrinB2 is removed, such as EphrinB2EC-del2. In an embodiment, the chimeric polypeptides respectively comprising the extracellular region of the receptor regulatory domain composed of the above-mentioned EphrinB2EC-del1, EphrinB2EC-del2, EphrinB2EC-del3 or EphrinB2EC-del23 all can be triggered to cleave and release the intracellular domain after specifically binding to the target molecule.

The variant of the extracellular region of the protein mentioned in the present application means some amino acid sequences in all the amino acids of the extracellular region of the protein are mutated or deleted or increased. In an embodiment, the chimeric polypeptide comprising the extracellular region of the receptor regulatory domain composed of a variant of the extracellular region of EphrinB2 is triggered to cleave and release the intracellular domain after specifically binding to the target molecule. In an embodiment, the chimeric polypeptide comprising the extracellular region of the receptor regulatory domain composed of a variant of the extracellular region of Jagged2 is triggered to cleave and release the intracellular domain after specifically binding to the target molecule.

The truncated structure or truncated form of the extracellular region of the protein mentioned in the present application means that some amino acid fragments are truncated from all the amino acid fragments of the extracellular region of the protein. In an embodiment, all of the chimeric polypeptides respectively comprising the extracellular region of the receptor regulatory domain composed of truncations EphrinB2EC-del1, EphrinB2EC-del2, EphrinB2EC-del3 or EphrinB2EC-del23 can be triggered to cleave and release the intracellular domain after specifically binding to the target molecule.

In the present application, the fragment, variant or truncated structure (also referred to as the truncated form or truncation) of the extracellular region of the protein can sometimes be mixed for use, and the sequence identity of the fragment, variant or truncated structure (also referred to as the truncated form or truncation) of the extracellular region of the protein compared to the extracellular region of the protein is at least about 60%, 70%, 80%, 85%, 90%, 95%, 96%, 97%, 98%, 99%. In an embodiment, the amino acid sequence of the truncated structure is shown in SEQ ID NOs: 5, 7, 9, 11.

In a specific embodiment of the present application, the fragment, variant or truncated structure (also referred to as the truncated form) of the extracellular region of the protein is a potential site for cleavage by ADAM10 of the extracellular region of EphrinB2 removed or mutated.

In an embodiment, the extracellular region comprises the full length of EphrinB2 extracellular domain of a human or non-human animal (e.g., a white-cheeked gibbon, a bonobo, a Sumatran orangutan, a chimpanzee, a gorilla, a rabbit, a peruvian night monkey, a marmoset, a coronal lemur, a small-eared greater Galago, a mouse, a rat, a cow, an xenopus laevis) or a truncation thereof. In an embodiment, the extracellular region comprises the full length of Jagged2 extracellular region or a truncation thereof. In an embodiment, the extracellular domain comprises an EphrinB2 extracellular domain comprising no cleavage site. In an embodiment, the extracellular region comprises an EphrinB2 extracellular region that does not include a cleavage site. In an embodiment, the extracellular region comprises a fragment having at least 60%, 65%, 70%, 75%, 80%, 85%, 90% homology or identity to the amino acid sequence shown in SEQ ID NOs: 1, 3, 5, 7, 9 or 11, and/or optionally comprises at most 1, 2, 3, 4, 5 or more amino acid residues replaced with different amino acid residues.

In an embodiment, the receptor regulatory domain comprises EphrinB2 extracellular region/ Notch1 transmembrane region (Notch1STS), EphrinB2 extracellular region/Notch1 transmembrane region (Notch2STS), EphrinB2 extracellular region/Notch1 transmembrane region (Notch3STS), EphrinB2 extracellular region/Notch1 transmembrane region (Notch4STS), EphrinB2 extracellular region/Notch2 transmembrane region (Notch1STS), EphrinB2 extracellular region/Notch2 transmembrane region (Notch2STS), EphrinB2 extracellular region /Notch2 transmembrane region (Notch3STS), EphrinB2 extracellular region/Notch2 transmembrane region (Notch4STS), EphrinB2 extracellular region/Notch3 transmembrane region (Notch1STS), EphrinB2 extracellular region/Notch3 transmembrane region (Notch2STS), EphrinB2 extracellular region/Notch3 transmembrane region (Notch3STS), EphrinB2 extracellular region/Notch3 transmembrane region (Notch4STS), EphrinB2 extracellular region/Notch4 transmembrane region (Notch1STS), EphrinB2 extracellular region/Notch4 transmembrane region (Notch2STS), EphrinB2 extracellular region/Notch4 transmembrane region (Notch3STS), EphrinB2 extracellular region/Notch4 transmembrane region (Notch4STS), EphrinB2 extracellular region/APLP2 transmembrane region (APLP2STS), EphrinB2 extracellular region/APLP2 transmembrane region (Notch1STS), EphrinB2 extracellular region/APLP2 transmembrane region (Notch2STS), Jagged2 extracellular region/Notch1 transmembrane region (Notch1STS), Jagged2 extracellular region/Notch1 transmembrane region (Notch2STS), Jagged2 extracellular region/APLP2 transmembrane region (APLP2STS), Jagged2 extracellular region/APLP2 transmembrane region (Notch1STS), Jagged2 extracellular region/APLP2 transmembrane region (Notch2STS). In this text, the transmembrane region is the Notch1 transmembrane region (Notch1STS), which means that the transmembrane region comprises the Notch1 transmembrane region and the STS part of Notch1, the transmembrane region is the Notch1 transmembrane region (Notch2STS), which means that the transmembrane region comprises the Notch1 transmembrane region and Notch2STS is used in the STS part, and the transmembrane region is Notch4 transmembrane region (Notch1STS), which means that the transmembrane region comprises the Notch4 transmembrane region and Notch1STS, and the rest of the description is extrapolated in order.

Exemplarily, the chimeric polypeptide synJagged2EC comprises a receptor regulatory domain composed of a fragment of Jagged2 extracellular region (SEQ ID No: 1) and a Notch1 transmembrane region (SEQ ID No: 13). In an embodiment, the receptor regulatory domain of the chimeric polypeptide synJagged2EC comprises the sequence as shown in SEQ ID No: 28.

Exemplarily, the chimeric polypeptide synEphrinB2EC comprises a receptor regulatory domain composed of EphrinB2 extracellular region (SEQ ID No: 1, 3, 5, 7, 9 or 11) and Notch1 transmembrane region (SEQ ID No: 13), or EphrinB2 extracellular region (SEQ ID No:3 or 4) and Notch1 transmembrane domain (Notch2STS) (SEQ ID No:102), or EphrinB2 extracellular region (SEQ ID No:1, 3, 5, 7, 9 or 11 ) and Notch1 transmembrane domain (Notch3STS) (SEQ ID No: 103), or EphrinB2 extracellular region (SEQ ID No: 1, 3, 5, 7, 9 or 11) and Notch1 transmembrane domain (Notch4STS) (SEQ ID No:104), or EphrinB2 extracellular region (SEQ ID No:1, 3, 5, 7, 9 or 11) and Notch2 transmembrane domain (SEQ ID No:14), or EphrinB2 extracellular region (SEQ ID No: 1, 3, 5, 7, 9 or 11) and Notch3 transmembrane domain (SEQ ID No: 15), or EphrinB2 extracellular region (SEQ ID No: 1, 3, 5, 7, 9 or 11) and Notch4 transmembrane domain (SEQ ID No: 16), or EphrinB2 extracellular region (SEQ ID No: 1, 3, 5, 7, 9 or 11) and human Notch1 transmembrane domain (SEQ ID No: 17), or EphrinB2 extracellular region region (SEQ ID No: 1, 3, 5, 7, 9 or 11) and human Notch2 transmembrane domain (SEQ ID No: 18), or EphrinB2 extracellular region (SEQ ID No: 1, 3, 5, 7, 9 or 11) and human Notch3 transmembrane domain (SEQ ID No: 19), or EphrinB2 extracellular region (SEQ ID No:1, 3, 5, 7, 9 or 11) and human Notch4 transmembrane domain (SEQ ID No: 20), or the receptor regulatory domain composed of EphrinB2 extracellular region (SEQ ID No: 1, 3, 5, 7, 9 or 11) and human APLP2 transmembrane domain (SEQ ID No: 21). In an embodiment, the receptor regulatory domain of the chimeric polypeptide synEphrinB2EC comprises the sequence as shown in SEQ ID No: 29, 30, 31, 32, 33, 34, 35, 37, 38, 39, 40, 41 or 85.

The chimeric polypeptide synEphrinB2EC-APLP2(TM) comprises a receptor regulatory domain composed of a fragment of EphrinB2 extracellular region (SEQ ID No: 3) and an APLP2 transmembrane region (SEQ ID No: 21). In an embodiment, the receptor regulatory domain of the chimeric polypeptide synEphrinB2EC-APLP2(TM) comprises the sequence as shown in SEQ ID No: 36.

In an embodiment, the receptor regulatory domain comprised in the chimeric polypeptide comprises an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identity or homology to any one of sequences as shown in SEQ ID No: 28, 29, 30, 31, 32, 33, 34, 35, 36, 37, 38, 39, 40, 41 or 85.

The receptor regulatory domain of the chimeric polypeptide is short, which is conducive to virus packaging and expression, and reduces the difficulty of industrial production preparation. The full length of the coding sequence of each of the chimeric polypeptides synEphrinB2EC, synEphrinB2EC-del, synJagged2EC, or synEphrinB2EC-APLP2(TM) is shorter than that of the synNotch, and the ultimately prepared vector comprising the complete chimeric polypeptide have a high transduction efficiency, which reduces the difficulty of industrial production preparation.

In an embodiment, the chimeric polypeptide synEphrinB2EC, synEphrinB2EC-del, synJagged2EC or synEphrinB2EC-APLP2(TM) and its binding-triggered regulation gene are not located in the same vector, that is, a dual vector system. In an embodiment, the chimeric polypeptide synEphrinB2EC, synEphrinB2EC-del, synJagged2EC, synEphrinB2EC-APLP2(TM) and its binding-triggered regulation gene are located in the same vector, that is, a single vector system. In an embodiment, the infection efficiency of the synEphrinB2EC, synEphrinB2EC-del, synJagged2EC, and synEphrinB2EC-APLP2(TM) single vector system is higher than that of synNotch, which reduces the difficulty of industrial production preparation. In an embodiment, the infection efficiency of the synEphrinB2EC single-vector system is about 1.5 times higher than that of synNotch, which reduces the difficulty of industrial production.

Compared with synNotch, the chimeric polypeptide synEphrinB2EC-del induced significantly lower leaky expression level of transcriptional regulation. Compared with synNotch, the chimeric polypeptide synEphinB2EC-Del3 has both stringency of induced expression and strong inducibility. The chimeric polypeptide synEphinB2EC-Del3 can better distinguish the different expression levels of the same target molecule, and is suitable for identifying some target molecules that are low expressed in normal tissues but highly expressed in tumor tissues, and are less likely to cause off-target effects in normal tissues. Compared with synNotch, the chimeric polypeptide synEphinB2EC-Del3 regulates gene expression more strictly, and is less sensitive to low-expressed target molecules. When targeting some target molecules highly expressed in tumors and low expressed in normal tissues, synEphinB2EC-Del3 has more strong selectivity to tumor tissues with higher security.

### 2.2 Intracellular domain

The chimeric polypeptide in the present application comprises an intracellular domain that is released by hydrolysis after binding of the chimeric polypeptide to a target molecule.

The intracellular domain of the chimeric polypeptide in the present application comprises a fragment of a protein selected from a transcription factor (including a transcriptional activator, a transcriptional repressor), a transcriptional coactivator, a transcriptional co-repressor, a DNA-binding polypeptide, a RNA-binding polypeptide, a translation-regulating polypeptide, a hormone, a cytokine, a toxin, an antibody, a chromatin regulator, a suicide protein, an organelle-specific polypeptide (e.g., a nuclear pore regulator, a mitochondrial regulator, an endoplasmic reticulum regulator, etc.), a pro-apoptotic polypeptide, an anti-apoptotic polypeptide, other polypeptides that promote cell death through other mechanisms, a pro-proliferative polypeptide, an anti-proliferative polypeptide, an immune co-stimulatory polypeptide, a site-specific nuclease, a recombinase, an inhibitory immune receptor, an activating immune receptor, variants of Cas9 and RNA-targeting nucleases, a DNA recognition peptide, a signaling peptide, a receptor tyrosine kinase, a non-receptor tyrosine kinase, a differentiation-promoting peptides, or a combination thereof.

The intracellular domain of the chimeric polypeptide in the present application comprises a transcriptional activator that promotes or represses the transcription of a DNA sequence driven by a promoter. In an embodiment, the transcription factor directly regulates cell differentiation. In an embodiment, the transcription factor indirectly regulates cell differentiation by regulating the expression of a second transcription factor. The transcription factor is the transcriptional activator or the transcriptional repressor. In an embodiment, the transcription factor is the transcriptional repressor. In an embodiment, the transcription factor is the transcriptional activator. In an embodiment, the transcription factor also includes a nuclear localization signal. In an embodiment, the transcription factor is selected from the group consisting of Gal4-VP16, Gal4-VP64, tetR-VP64, ZFHD1-VP64, Gal4-KRAB, and HAP1-VP16. In an embodiment, the transcription factor is Gal4. In an embodiment, the transcription factor is Gal4-VP64.

In some cases, the antibody induced by the intracellular domain of the chimeric polypeptide in the present application is a therapeutic antibody for treating a disease (including immune disease, tumor).

In an embodiment, the intracellular domain comprises a fragment of a protein selected from a transcriptional activator, a transcriptional repressor, a site-specific nuclease, a recombinase, an inhibitory immune receptor, an activating immune receptor, or a combination thereof. In an embodiment, the transcriptional activator comprises GLA4, GLA4-VP64 or a fragment thereof. In an embodiment, the transcriptional activator protein comprises an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identity or homology to the amino acid sequence shown in SEQ ID NO: 27.

In an embodiment, the site-specific nuclease is a Cas9 polypeptide. In an embodiment, the intracellular domain is a recombinase. In an embodiment, the intracellular domain is an inhibitory immune receptor. In an embodiment, the intracellular domain is an activating immune receptor.

In an embodiment, the chimeric polypeptide comprises an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identity or homology to any one of the amino acid sequences shown by respectively connecting SEQ ID No: 28, 29, 30, 31, 32, 33, 34, 35, 36, 37, 38, 39, 40, 41 or 85 with SEQ ID No: 27.

### 2.3 Binding domain (also referred to as binding peptide segment)

The chimeric polypeptide comprises a binding domain that specifically binds to a target molecule.

In an embodiment, the binding domain of the chimeric receptor disclosed herein specifically binds to one or more target molecules. In an embodiment, the chimeric polypeptide comprises a linker interposed between the binding domain and the receptor regulatory domain. The binding domain comprises an antibody, an antigen, a ligand, a receptor, a target (e.g., tag FLAG), a Fc receptor, an extracellular matrix component, a cell adhesion molecule, a non-antibody molecular scaffold, or a combination thereof.

In an embodiment, the binding domain of the chimeric polypeptide comprises an antigen binding domain. In an embodiment, the antigen binding domain is selected from an antibody, a receptor, a cell adhesion molecule, a non-antibody molecular scaffold, or a combination thereof. In an embodiment, the antigen binding domain comprises an antibody based recognition scaffold. In an embodiment, the antigen binding domain comprises an antibody. In an embodiment, the antigen binding domain comprises an antibody that specifically binds to a tumor antigen, a disease-associated antigen, or an extracellular matrix component. In an embodiment, the antigen binding domain comprises an antibody that specifically binds to a cell surface antigen, a soluble antigen, or an antigen immobilized on an insoluble substrate. In an embodiment, the antigen binding domain comprises a single chain antibody Fv (scFv). In an embodiment, the antigen binding domain comprises an antibody that can specifically bind to multiple antigens. In an embodiment, the antigen binding domain comprises a nanobody, a single domain antibody, a diabody, a triabody, or a minibody, or a combination thereof. In an embodiment, the antigen binding domain is a non-antibody based recognition scaffold, such as an avimer, a DARPin, an adnectin, an avimer, an affibody, an anticalin or an affilin. In an embodiment, the antibody is a single domain antibody, a single chain antibody, a diabody, a triabody, a minibody, a F(ab')₂ fragment, a F(ab)v fragment, a scFv, a single domain antibody (sdAb) and a functional fragment thereof, or a combination thereof. In an embodiment, the binding domain comprises an antigen, e.g., an endogenous antigen, an exogenous antigen. In an embodiment, the binding domain comprises a ligand for the receptor. In an embodiment, the binding domain comprises a receptor. In an embodiment, the binding domain comprise a cell adhesion molecule (e.g., all or a portion of the extracellular region of the cell adhesion molecule). In an embodiment, the binding domain comprises a partial domain of the polymerization domain.

In an embodiment, the binding domain specifically binds to the tumor antigen and/or pathogen antigen described in the term "antigen" in the part of "1. Definitions". In an embodiment, the binding domain comprises an antibody that specifically binds to the tumor antigen and/or pathogen antigen in the term "antigen" in the part of "1. Definitions". In an embodiment, the binding domain comprises an antibody that specifically binds to Mesothelin, FAP, Claudin18.2, CLL1, CD19, GPC3, WT1, HER2, EGFR, EGFRvIII, BCMA, CD123 or a combination thereof. In an embodiment, the binding domain comprises an antibody VH or VL or scFV, which specifically binds to Mesothelin, FAP, Claudin18.2, CLL1, CD19, GPC3, WT1, HER2, EGFR, EGFRvIII, BCMA, CD123 or a combination thereof. In an embodiment, the binding domain comprises an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identity or homology to any one of the amino acid sequences shown in SEQ ID NO: 42, 43, 44, 45, 46, 47, 86, 89, 90, 91, 92, 93, 94, 95, 96, 97, 98, 99, 100 or 101.

In an embodiment, the chimeric polypeptide comprises an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identity or homology to any one of the amino acid sequences shown in SEQ ID NO: 50, 51, 52, 53, 54, 55, 56, 57, 61, 73, 87, 105, 106, 108 or 110.

### 2.4 Target molecule

The target molecule that binds to the chimeric polypeptide of the present application is also refered to as a ligand, or a target antigen.

The target molecule can be membrane bound. The target molecule may be present on the surface of the cell. The target molecule may be immobilized on an insoluble substrate (e.g., polyethylene, polystyrene, polyvinylpyrrolidone, polycarbonate, nitrocellulose, etc.). The target molecules may be soluble. The target molecule may be present in the extracellular environment (e.g., the extracellular matrix). The target molecule may be present in an artificial matrix. The target molecule may be present in an acellular environment. The target molecule is present on an insoluble support which can take a variety of forms, e.g., a plate, an tissue culture dish, a column, and the like. The target molecule may be present in the extracellular matrix (ECM) (e.g., the antigen is an ECM component). The target molecule may be present in an artificial matrix. The target molecule may be present in an acellular environment. The target molecule comprises a polypeptide, a nucleic acid, a glycoprotein, a small molecule, a carbohydrate, a lipid, a glycolipid, a lipoprotein, and a lipopolysaccharide. In an embodiment, the target molecule is selected from the group consisting of a differentiation marker cluster, a cell surface receptor, an adhesion protein, an integrin, a mucin, a lectin, a tumor antigen.

In an embodiment, the target molecule is a differentiation (CD) marker cluster. In some embodiments, the CD marker is selected from the group consisting of: CD1, CD1a, CD1b, CD1c, CD1d, CD1e, CD2, CD3d, CD3e, CD3g, CD4, CD5, CD7, CD8a, CD8b, CD19, CD20, CD21 , CD22, CD23, CD25, CD27, CD28, CD33, CD34, CD40, CD45, CD48, CD52, CD59, CD66, CD70, CD71, CD72, CD73, CD79A, CD79B, CD80 (B7.1), CD86 (B7.2), CD94, CD95, CD134, CD140 (PDGFR4), CD152, CD154, CD158, CD178, CD181 (CXCR1), CD182 (CXCR2), CD183 (CXCR3), CD210, CD246, CD252, CD253, CD261, CD262, CD273 (PD-L2), CD274 (PD-L1), CD276 (B7H3), CD279, CD295, CD339 (JAG1), CD340 (HER2), EGFR, FGFR2, CEA, AFP, CA125, MUC-1, MAGE, alkaline phosphatase, placenta-like 2 (ALPPL2), B cell maturation antigen (BCMA), blue fluorescent protein (BFP), green fluorescent protein (GFP), enhanced green fluorescent protein (EGFP), and signal regulatory protein alpha (SIRPα).

In an embodiment, the target molecule is an antigen. In an embodiment, the target molecule comprises a tumor antigen and/or a pathogen antigen. In an embodiment, the tumor antigen is selected from the group consisting of: Mesothelin, FAP, Claudin18.2, CLL1, CD19, GPC3, WT1, EGFR, BCMA, CD7, NKG2D-Ligand, CD19, B7H3, ALPPL2, CD123, CD171, CD179a, CD20 , CD213A2, CD22, CD24, CD246, CD272, CD30, CD33, CD38, CD44v6, CD46, CD71, CD97, CEA, CLDN6, CLECL1, CS-1, EGFR, EGFRvIII, ELF2M, EpCAM, EphA2, FLT3, GD2, GD3 , GM3, GPRC5D, HER2 (ERBB2), IGLL1, IL 11Ra, IL13Ra2, CD 117, MUC1, NCAM, PAP, PDGFR-b, PRSS21, PSCA, PSMA, ROR1, SIRPa, SSEA-4, TAG72, TEM1/CD248, TEM7R, TSHR, VEGFR2, ALPI, cMet and Axl.

In an embodiment, the chimeric polypeptide and the transcriptional regulation expressed CAR recognize different tumor antigens respectively.

In the treatment of tumors with antigenic heterogeneity, the proportion of positive cells (i.e., positive rate) for the first target molecule (e.g., EGFRvIII) is low, and the positive rate for the second target molecule (e.g., II,13Ra2) is high. Such tumor cells will escape the treatment targeting the first target molecule, thereby reducing the curative effect. Due to the widespread expression of the second target molecule, the treatment targeting the second target molecule may result in off-target toxicity. In an embodiment, the present application constructs a chimeric polypeptide binding to the first target molecule to trigger transcriptional regulation of the expression of an exogenous receptor recognizing the second target molecule, thereby achieving specific and extensive killing of tumor cells expressing the second target molecule and partially the first target molecule. In an embodiment, the killing effect of the CAR targeting the second target molecule depends on the triggered transcriptional regulatory activity of the chimeric polypeptide binding to the first target molecule, that is, the killing effect requires the presence of the first target molecule and the second target molecule in the environment. Compared with synNotch, the use of chimeric polypeptide synEphrinB2EC, synJagged2EC, synEphrinB2EC-APLP2(TM) binding to the first target molecule to trigger regulation of the expression of the exogenous receptor (such as CAR) targeting the second target molecule, can significantly improve the anti-tumor effect of CAR-T cells transcription-regulated by chimeric polypeptide on tumors with the expression heterogeneity of the first target molecule _{∘}

In the treatment of tumors with target molecule heterogeneity, the first target molecule (exemplarily, Mesothelin) is highly expressed, and also expressed in normal tissues, and the second target molecule (exemplarily, Claudin18.2) is highly expressed, also expressed in other normal tissues; targeting either the first target molecule or the second target molecule alone can lead to off-target toxicity. In an embodiment, compared with synNotch, the use of chimeric polypeptide synEphrinB2EC, synJagged2EC, synEphrinB2EC-APLP2(TM) specifically binding to the first target molecule to trigger transcriptional regulation of the expression of the exogenous receptor targeting the second target molecule, can significantly improve the anti-tumor effect of the CAR-T cells with the chimeric polypeptide binding triggered transcriptional regulation on tumors with expression heterogeneity of the first and second target molecules. In an embodiment, the use of chimeric polypeptides synEphrinB2EC, synJagged2EC, synEphrinB2EC-APLP2(TM) specifically binding to the second target molecule to trigger regulation of the expression of the exogenous receptor targeting the first target molecule, compared to synNotch, can significantly improve the anti-tumor effect of the CAR-T cells with the chimeric polypeptide binding triggered transcriptional regulation on tumors with expression heterogeneity of the first and second target molecules.

In an embodiment, the cells for treatment express the second target molecule targeted by CAR, and then the treatment targeting the second target molecule will cause the cells to kill each other during the culture process, affecting the cell viability and yield. In an embodiment, using the chimeric polypeptide that specifically binds to the first target molecule to trigger transcriptional regulation of the expression of the CAR targeting the second target molecule, the cell specifically binds to the first target molecule on a tumor cell to trigger transcriptional regulation of expression of the exogenous receptor targeting the second target molecule in the cells, thereby killing tumor cells expressing the second target molecule, and/or attacking host immune cells expressing the second target molecule to increase the survival and expansion of the cells, to further improve the anti-tumor activity. In an embodiment, the second target molecule comprises a NK cell marker, which is selected from the group consisting of: NKG2 receptor family, such as NKG2A, NKG2D, NKG2C, etc.; killer immunoglobulin-like receptor (KIR) family, such as KIR2DL1, KIR2DL2/3. KIR2DL4, KIR2DL5, KIR3DL1, 15KIR3DL2, KIR2DS1, KIR2DS2/S3, KIR2DS4, KIR2DS5, KIR3DS1, etc.; natural cytotoxic receptors (NCR), such as NKP30, NKP44, NKP46, NKp80, etc.; and other NK cell-specific expressed antigens such as CD159a, CD159c, CD94, CD158, CD56, LIR/ILT2, CD244, CD226, CD2, CD16, CD161, TIGIT, CS1. In an embodiment, the chimeric polypeptide that specifically binds to the first target molecule of leukemia cells (such as CLL1, CD123) is used to trigger transcriptional regulation of the expression of the exogenous receptor targeting NKG2D, thereby killing tumor cells.

In an embodiment, the target molecule is an inflammatory disease-associated molecule, including but not limited to: AOC3 (VAP-1), CAM-3001, CCL11 (eotaxin-1), CD125, CD147 (basigin), CD154 (CD40L), CD2, CD20, CD23 (IgE receptor), CD25 (α chain of IL-2 receptor), CD3, CD4, CD5, IFN-α, IFN-γ, IgE, IgE Fc region, IL -1, IL-12, IL-23, IL-13, IL-17, IL-17A, IL-22, IL-4, IL-5, IL-5, IL-6, IL-6 receptor, integrin α4, integrin α4β7, LFA-1 (CD11a), myostatin, OX-40, scleroscin, SOST, TGFβ1, TNF-α, and VEGF-A.

### 2.5. Chimeric polypeptide binding triggered regulation gene

The chimeric polypeptide binding triggered regulation gene is operably linked to a transcriptional control element that is activated or repressed by the intracellular domain of the chimeric polypeptide. In an embodiment, the regulated gene is regulated for expression by a GAL-4, tetR, ZFHD1, HNF1A or HAP1 modulated promoter. In an embodiment, the regulated gene expression product is selected from the group consisting of: a non-coding RNA, a cytokine, a cytotoxin, a chemokine, an immunomodulator, a pro-apoptotic factor, an anti-apoptotic factor, a hormone, a differentiation factor, a dedifferentiation factor, a modifying TCR, a CAR, a reporter gene, or a combination thereof.

In an embodiment, the chimeric polypeptide binding triggered regulation genes includes but not limited to: a chemokine, a chemokine receptor, a cytokine, a cytokine receptor, a differentiation factor, a growth factor, a growth factor receptor, a hormone, a metabolic enzyme, a proliferation inducer, a receptor, a small molecule 2nd messenger synthesis enzyme, a T cell receptor, a transcription activator, a transcription repressor, a transcriptional activator, a transcriptional repressor, a translation regulator, a translational activator, a translational repressor, an activating immunoreceptor, an apoptosis inhibitor, an apoptosis inducer, an immunoactivator, an immunoinhibitor and an inhibiting immunoreceptor.

In an embodiment, the chimeric polypeptide binding triggered regulation gene includes but not limited to: a transcriptional activator, a transcriptional repressor, a CAR, a second chimeric polypeptide, a translational regulator, a cytokine, a hormone, a chemokine or an antibody molecule. In an embodiment, a nucleic acid sequence encoding the transcriptional activator, the transcriptional repressor, foreign receptor, the second chimeric polypeptide, the translation regulator, the cytokine, the hormone, the chemokine, the antibody molecule or a combination thereof is operably linked to a transcriptional control element that is activated or repressed by the intracellular domain of the chimeric polypeptide. In an embodiment, the transcriptional control element includes UAS (upstream activating sequence), the sequence of which is shown in SEQ ID NO: 48. In an embodiment, the intracellular domain of the chimeric polypeptide comprises GAL4-VP64, the sequence of which is shown in SEQ ID NO: 27; the nucleic acid sequence of the chimeric polypeptide binding triggered regulation gene is operably linked to UAS, the sequence of which is shown in SEQ ID NO: 48. In an embodiment, the intracellular domain of the chimeric polypeptide comprises GAL4-VP64, the sequence of which is shown in SEQ ID NO: 27; the nucleic acid sequence of the chimeric polypeptide binding triggered regulation gene is operably connected to the UAS-CMV promoter, and the sequence of which is shown in SEQ ID NO:49.

In an embodiment, the chimeric polypeptide binding triggered regulation gene comprises: an exogenous cytokine or a CAR. In an embodiment, the nucleic acid sequence of the exogenous cytokine is operably linked to a transcriptional control element that is activated or repressed by the intracellular domain of the chimeric polypeptide. In an embodiment, after the chimeric polypeptide in the cell binds to the target molecule, the receptor is triggered to hydrolyse so that the receptor cleaves and releases the intracellular domain, to induce the cells to express cytokines or chemokines including: an interferon (α- Interferon, β-interferon, γ-interferon), an interleukin (IL-1, IL-1α, IL-2, IL-3, IL-4, IL-5, IL-6, IL-7, IL-8. IL-9, IL-10, IL-11, IL-12; IL-13, IL-14, IL-15, IL-16, IL-17, IL-17A, IL-18, IL-19, IL-20, IL-24), a tumor necrosis factor (TNF-α), a transforming growth factor-β, TRAIL, MIP-1, MIP-1β, MCP-1, RANTES, IP10, CCL2, CCL3, CCL5, CCL17, CCL19, CCL21, CCR7, CXCL9, CXCL10, CXCL11, CXCL16, MCSF. In an embodiment, the cytokine comprises IL-2, IL-7, IL-9, IL-12, IL-15, IL-18, CCL21 or a combination thereof.

In an embodiment, the nucleic acid sequence of the cytokine IL12 is operably linked to a transcriptional control element which is activated by the intracellular domain of the chimeric polypeptide. The IL12 has subunit P35 of GENE ID: 3592, and subunit P40 of GENE ID: 3593. In an embodiment, IL12 comprises the sequence as shown in SEQ ID NO: 58.

In an embodiment, after the chimeric polypeptide binds to the target molecule in the cell, the receptor is triggered to hydrolyse so that the receptor cleaves and releases the intracellular domain, to induce the cells to express CAR or modifying TCR. The nucleic acid sequence of the CAR or modifying TCR is operably linked to a transcriptional control element that is activated or repressed by the intracellular domain of the chimeric polypeptide. In an embodiment, the CAR or modifying TCR specifically recognizes: a tumor antigen, a cancer cell-associated antigen, a hematological malignancy antigen, a solid tumor antigen, a cell surface antigen, an intracellular antigen, and the like.

In an embodiment, the first target molecule and the second target molecule are different, and are respectively selected from the group consisting of 1) blood tumor antigens: CD19 (expressed in B cells), CD20 (expressed in B cells), CD22 (expressed in B cells) , CD30 (expressed in B cells), CD33 (expressed in bone marrow cells), CD70 (expressed in B cells/T cells), CD123 (expressed in bone marrow cells), κ (expressed in B cells), Lewis Y (expressed in bone marrow cells), NKG2D ligand (expressed in myeloid cells), ROR1 (expressed in B cells), SLAMF7/CS1 (expressed in myeloma cells, natural killer cells, T cells and most B cells), CD138 (expressed in malignant plasma cells in multiple myeloma), CD56 (expressed in myeloma cells, neural cells, natural killer cells, T cells and trabecular osteoblasts), CD38 (expressed in B cells/T cells), and CD160 (expressed in NK cells/T cells), and the like; 2) solid tumor antigens: B7H3 (expressed in sarcomma, glioma), CAIX (expressed in kidney), CD44v6/v7 (expressed in cervix), CD171 (expressed in neuroblastoma), CEA (expressed in colon), EGFRvIII (expressed in glioma), EGP2 (expressed in cancer), EGP40 (expressed in colon), EphA2 (expressed in glioma, lung), ErbB2 (HER2) (expressed in breast, lung, prostate, glioma), ErbB receptor family (expressed in breast, lung, prostate, glioma), ErbB3/4 (expressed in breast, ovary), HLA-A1/MAGE1 (expressed in melanoma), HLA-A2/NY-ESO-1 (expressed in sarcoma, melanoma), FR-a (expressed in ovary), FAR (expressed in rhabdomyosarcoma), GD2 (expressed in neuroblastoma, sarcoma, melanoma), GD3 (expressed in melanoma, lung cancer), HMW-MAA (expressed in melanoma), IL11Ra (expressed in osteosarcoma), IL13Ra2 (expressed in glioma), Lewis Y (expressed in breast/ovary/pancreas), mesothelin (expressed in mesothelioma, breast, pancreas), Muc1 (expressed in ovary, breast, prostate), NCAM (expressed in neuroblastoma, colon rectum), NKG2D ligand (expressed in ovary, sarcomata), PSCA (expressed in prostate, pancreas), PSMA (expressed in prostate), TAG72 (expressed in colon), VEGFR-2 (expressed in tumor vasculature), Axl (expressed in lung cancer), Met (expressed in lung cancer), α5β3 (expressed in tumor vasculature), α5β1 (expressed in tumor vasculature), TRAII,-R1/TRAII,-R2 (expressed in solid tumors (colon, lung, pancreas) and hematological malignancies), RANKL (expressed in prostate cancer and bone metastases), tenascin (expressed in glioma, epithelial tumors (breast, prostate)), EpCAM (expressed in epithelial tumors (breast, colon, lung)), CEA (expressed in epithelial tumors (breast, colon, lung)), gpA33 (expressed in colorectal cancer), mucin (expressed in epithelial tumors (breast, colon, lung, ovary)), TAG-72 (expressed in epithelial tumors (breast, colon, lung)), EphA3 (expressed in lung, kidney, melanoma, glioma, hematological malignancies) and IGF1R (expressed in lung, breast, head and neck, prostate, thyroid, glioma). Examples of surface and intracellular antigens include, e.g., Her2 (ERBB2), MAGE-A1 (MAGEA1), MART-1 (MLANA), NY-ESO (CTAG1), WT1, MUC17, and MUC13. In an embodiment, the first and second target molecules are respectively selected from the group consisting of: BCMA, B7H6, CAIX, CD123, CD138, CD171, CD19, CD20, CD22, CD30, CD33, CD38, CD44, CEA, CS1, EGFRvIII, EGP2, EGP40, Erb family members (ERBB1, ERBB2, ERBB3, ERBB4), FAP, fetal acetylcholine receptor (AChR), folate receptor alpha (FOLR1), folate receptor beta (FOLR2), GD2, GD3, GPC3, IL-13Ra2 (IL13RA2), kappa light chain (IGK), Lewis-Y, mesothelin (MSLN), mucin-1 (MUC1), mucin-16 (MUC16), NKG2D ligand, prostate-specific membrane antigen (PSMA), prostate stem cell antigen (PSCA), receptor tyrosine kinase-like orphan receptor 1 (ROR1), and anaplastic lymphoma receptor tyrosine kinase (ALK).

In an embodiment, the binding domain of the chimeric polypeptide specifically binds to the first target molecule, and the CAR specifically binds to a second target molecule different from the first target molecule, the first and second target molecules are respectively selected from the group consisting of: Mesothelin, FAP, Claudin18.2, CLL1 , CD19, GPC3, WT1, EGFR, EGFRvIII, BCMA, CD7, NKG2D-Ligand, MOG, CD19, B7H3(CD276), BCMA(CD269), ALPPL2, CD123, CD171, CD179a, CD20, CD213A2, CD22, CD24, CD246 , CD272, CD30, CD33, CD38, CD44v6, CD46, CD71, CD97, CEA, CLDN6, CLECL1, CS-1, EGFR, EGFRvIII, ELF2M, EpCAM, EphA2, Ephrin B2, FAP, FLT3, GD2, GD3, GM3, GPRCSD, HER2(ERBB2/neu), IGLL1, IL 11Ra, IL13Ra2, CD 117, MUC1, NCAM, PAP, PDGFR-b, PRSS21, PSCA, PSMA, ROR1, SIRPa, SSEA-4, TAG72, TEM1/CD248, TEM7R, TSHR, VEGFR2, ALPI, citrulline vimentin, cMet and Axl.

In an embodiment, the transcription factor triggered by the chimeric polypeptide in response to the first target molecule drives the expression of the CAR in response to the second target molecule, so that the CAR is active only in the presence of the first and second target molecules, to activate T cells. In an embodiment, the first target molecule and the second target molecule are ASGR1 and GPC3, EGFRvIII and IL13Ra2, EGFRvIII and B7H3, Mesothelin and Claudin18.2, Claudin18.2 and Mesothelin, FAP and Claudin18.2, CLL1 and NKG2D, CD123 and NKG2D, respectively. In an embodiment, the chimeric polypeptide and the CAR respectively comprise the sequence shown in SEQ ID NO: 61, 62; or the sequences shown in SEQ ID NO: 61, 64; or the sequences shown in SEQ ID NO: 61, 65; or the sequences shown in SEQ ID NO: 61, 66; or the sequences shown in SEQ ID NO: 105, 67; or the sequences shown in SEQ ID NO: 106, 67; or the sequences shown in SEQ ID NO: 108, 107; or the sequences shown in NO: 73, 109; or the sequences shown in SEQ ID NO: 50, 109.

U87 and U251 cells express endogenous IL13Ra2 but not EGFRvIII. The killing of T cells containing the chimeric peptide that modulates CAR expression against cells expressing at least 2 or more target molecules with different positive rates is detected. In an embodiment, EGFRvIII-positive and EGFRvIII-negative cells were mixed in different proportions to simulate IL13Ra2-positive and only partially EGFRvIII-positive gliomas under natural conditions. Gastric cancer cell HGC-27 expresses endogenous Mesothelin but not Claudin18.2. HGC-27-A2 is HGC-27 cells overexpressing human Claudin18.2.

In an embodiment, the nucleic acid sequence encoding the CAR is operably linked to a transcriptional control element which is regulated by the intracellular domain of the chimeric polypeptide. In an embodiment, the nucleic acid sequence encoding the CAR is operably linked to the UAS-CMV promoter, and the intracellular domain of the chimeric polypeptide includes GLA4-VP64. In an embodiment, the extracellular antigen binding region of the CAR comprises an antibody that recognizes Mesothelin, FAP, Claudin18.2, CLL1, CD19, GPC3, WT1, HER2, EGFR, EGFRvIII, BCMA, CD7, CD123, NKG2D-Ligand or a combination thereof. In an embodiment, the extracellular antigen binding region of the CAR comprises an antibody scFV that recognizes Mesothelin, FAP, Claudin18.2, CLL1, CD19, GPC3, WT1, HER2, EGFR, EGFRvIII, BCMA, CD7, CD123, NKG2D-Ligand or a combination thereof. In an embodiment, the extracellular antigen binding region of the CAR comprises an amino acid sequence having at least 75%, 76%, 77%, 78%, 79%, 80%, 81%, 82%, 83%, 84%, 85%, 86%, 87%, 88 %, 89%, 90%, 91%, 92%, 93%, 94%, 95%, 96%, 97%, 98%, 99%, or 100% identity or homology to the amino acid sequence shown in SEQ ID NO: 42, 43, 44, 45, 46, 47, 86, 89, 90, 91, 92, 93, 94, 95, 96, 97, 98, 99, 100 or 101. In an embodiment, the IL13Ra2-CAR, B7H3-CAR, Claudin18.2-CAR, Mesothelin-CAR or NKG2D-CAR regulated for expression by the chimeric polypeptide is operably linked to a transcriptional control element, which is regulated by the intracellular domain regulation of the chimeric polypeptide. In an embodiment, the IL13Ra2-CAR, B7H3-CAR, Claudin18.2-CAR, Mesothelin-CAR or NKG2D-CAR regulated for expression by the chimeric polypeptide is inserted downstream of the UAS-CMV promoter, and the intracellular domain of the chimeric polypeptide comprises Gal4-VP64. The "Z-CAR-T expressing X-chimeric polypeptide Y" or "X-chimeric polypeptide Y-Z-CAR-T" described in the examples of the present application is used to describe T cells which comprise chimeric polypeptide Y targeting antigen X that modulates the expression of CAR recognizing antigen Z, the Z-CAR is inserted downstream of the UAS-CMV promoter, and the intracellular domain of the chimeric polypeptide Y comprises Gal4-VP64. In the above, the symbols X and Z are different tumor antigen, that is, the chimeric polypeptide Y can target the antigen X, and the CAR can recognize the antigen Z. When the chimeric polypeptide Y and CAR are combined and used in T cells, the expression of the CAR is regulated by the intracellular domain of the chimeric polypeptide Y

Gastric cancer highly expresses Mesothelin and Claudin18.2 at the same time. In order to prevent Claudin18.2-CAR-T cells from killing normal tissues expressing Claudin18.2, the killing effect of Claudin18.2-CAR-T cells is limited to the tumor area. In an embodiment, Claudin18.2-CAR-T cells express Mesothelin-synEphrinB2EC.

In an embodiment, the CAR extracellular antigen binding region is linked directly or by a hinge to the transmembrane region. In an embodiment, the hinge comprises a CD8 hinge, e.g., a sequence having 95-100% identity to SEQ ID NO: 75. In an embodiment, the upstream of the nucleic acid molecule encoding the CAR includes a polynucleotide encoding a signal peptide. In an embodiment, the signal peptide comprises a CD8 signal peptide, e.g., a sequence having 95-100% identity to SEQ ID NO: 81.

The transmembrane domain of the CAR molecule in the present application comprises a CD28 or CD8 transmembrane domain. In an embodiment, the CAR includes the CD8 transmembrane domain, e.g., a sequence having 95-100% identity to SEQ ID NO: 76. In an embodiment, the CAR comprises the CD28 transmembrane domain, e.g., a sequence having 95-100% identity to SEQ ID NO: 77.

In an embodiment, the CAR comprises an intracellular signaling domain: a primary signaling domain and/or a costimulatory signaling domain. In an embodiment, the primary signaling domain comprises a CD3ζ intracellular domain, e.g., a sequence having 95-100% identity to SEQ ID NO: 80. In an embodiment, the costimulatory signaling domain comprises a CD28 or 4-1BB intracellular domain.

In an embodiment, the CAR comprises a 4-1BB intracellular domain, e.g., a sequence having 95-100% identity to SEQ ID NO: 79. In an embodiment, the CAR comprises a CD28 intracellular domain, e.g., a sequence having 95-100% identity to SEQ ID NO: 78.

In an embodiment, the intracellular signaling domain of the CAR comprises a human CD3ζ intracellular domain. In an embodiment, the intracellular signaling domain of the CAR comprises a human CD3ζ intracellular domain and a CD28 intracellular domain. In an embodiment, the intracellular signaling domain of the CAR comprises a human CD3ζ intracellular domain and a 4-1BB intracellular domain. In an embodiment, the intracellular signaling domain of the CAR comprises a CD3ζ intracellular domain, a CD28 intracellular domain, and a 4-1BB intracellular domain. In an embodiment, the cell expresses a CAR and a cytokine.

Exemplarily, the CAR expressed by the transcription regulation of the chimeric polypeptide in the present application comprises a sequence in which the sequence shown in SEQ ID NO: 42, 43, 44, 45, 46, 47, 86, 89, 90, 91, 92, 93, 94, 95, 96, 97, 98, 99, 100 or 101 is sequentially connected with SEQ ID NO: 82, 83 or 84, respectively.

In an embodiment, the chimeric polypeptide binding triggered regulation genes include but not limited to: Trastuzumab (Herceptin, commercially available from Chugai Pharmaceutical Co., Ltd.); bevacizumab (Avastin, commercially available from Genentech, Inc.); infliximab (Remicade); rituximab (Rituxan, commercially available from Biogen Idec Inc.); adalimumab (Humira). In an embodiment, the above therapeutic antibody is inserted behind the UAS-CMV promoter regulated by Gal4-VP64.

### 2.6 Other sequences

In an embodiment, the chimeric polypeptide in the present application further comprises one or more other domains, comprising: a signal peptide, an epitope tag, an affinity domain, a nuclear localization signal (NLS), and a polypeptide that produces a detectable signal.

### 3. Nucleic acids and vectors

The present application provides a nucleic acid and an expression vector encoding the chimeric polypeptide of the present application and/or chimeric polypeptide binding triggered transcriptional regulation gene.

In an embodiment, the nucleotide sequence encoding the chimeric polypeptide of the present application is operably linked to a transcriptional control element (e.g., promoter, enhancer, etc.). In an embodiment, the transcriptional control element is inducible. In an embodiment, transcriptional control elements are constitutive. In an embodiment, the promoter is functional in eukaryotic cells. In an embodiment, the promoter is a cell type-specific promoter. In an embodiment, the promoter is a tissue-specific promoter.

In an embodiment, the expression vector is a viral vector, e.g., adeno-associated viral (AAV) vector, adenoviral vector, lentiviral vector, retroviral vector, and the like. In an embodiment, retroviral vectors (gamma-retroviruses or lentiviruses) are used to introduce nucleic acid molecules into cells. Non-viral vectors can also be used. Any suitable viral vector or non-viral delivery system can be used for transduction. In the specific examples of the present application, the pRRLSIN vector is constructed to express the chimeric polypeptide. Chimeric polypeptides or CARs can be constructed with accessory molecules (e.g., cytokines) in a single polycistronic expression cassette, multiple expression cassettes in a single vector, or in multiple vectors. Examples of elements for generating polycistronic expression cassettes include, but are not limited to, various viral and non-viral internal ribosome entry sites (IRES, e.g., FGF-1 IRES, FGF-2 IRES, VEGF IRES, IGF-II IRES, NF-κB IRES, RUNX1 IRES, p53 IRES, hepatitis A IRES, hepatitis C IRES, pestivirus IRES, baculovirus IRES, picornavirus IRES, poliovirus IRES, and encephalomyocarditis virus IRES) and cleavable linkers (For example, 2A peptides, such as P2A, T2A, E2A and F2A peptides).

Other viral vectors that may be used comprise, for example, adenovirus, lentivirus and adeno-associated viral vectors, vaccinia virus, bovine papilloma virus or herpes viruses such as Epstein-Barr virus.

Non-viral methods may also be used for the genetic modification of immune cells. For example, nucleic acid molecules can be introduced into immune cells by microinjection under lipofection, asialoorosomucoid-polylysine coupling, or surgical conditions. Other non-viral methods of gene transfer comprise in vitro transfection using transposons, liposomes, calcium phosphate, DEAE-dextran, electroporation and protoplast fusion. It is also possible to firstly transfer the nucleic acid molecule into the type of cell that may be cultured in vitro (for example, an autologous or allogeneic primary cell or its progeny), and then inject the cell (or its progeny) modified by the nucleic acid molecule into the target tissue or the whole body of the subject.

### 4. Engineered cells

The application provides an engineered cell genetically modified with the nucleic acid of the present application: an engineered cell genetically modified with the nucleic acid encoding the chimeric polypeptide of the present application; an engineered cell genetically modified with nucleic acids, comprising the chimeric polypeptide and the chimeric polypeptide binding triggered regulation gene of the present application. The present application provides a method for modulating the activity of a cell expressing a chimeric polypeptide of the present application. The method generally comprises contacting the engineered cell with a target molecule to induce cleavage of the chimeric polypeptide, thereby releasing the intracellular domain, which modulates the activity of the cell.

In an embodiment, engineered cells are genetically modified to express the chimeric polypeptide of the present application, and are further genetically modified to express a CAR. For example, engineered cells are genetically modified with a nucleic acid comprising a nucleotide sequence encoding the CAR, and the intracellular domain of the chimeric polypeptide is a transcriptional activator, and the nucleotide sequence encoding the CAR is operably linked to a transcriptional control element activated by the intracellular domain the chimeric polypeptide. Many CAR polypeptides have been described in the art, and any one of them are suitable for use in the present application.

In an embodiment, the engineered cells are immune cells, neurons, epithelial cells, endothelial cells or stem cells. Stem cells comprise human pluripotent stem cells (comprising human induced pluripotent stem cells (iPSC) and human embryonic stem cells). In an embodiment, the cells comprise immune cells. In an embodiment, the cells are primary cells. In an embodiment, the immune cells are B cells, monocytes, natural killer cells, basophils, eosinophils, neutrophils, dendritic cells, macrophages, regulatory T cells, helper T cells, cytotoxic T cells, other T cells, or combinations thereof. In an embodiment, the engineered cell comprises a nucleic acid sequence having at least 80% sequence homology to any one of SEQ ID NO: 59, 63, 68, 69, 70, 71, 72, 74, 88, or an amino acid sequence translated therefrom.

The immune cells of the present application may be cells of lymphoid lineage. The lymphoid lineage comprising B, T, and natural killer (NK) cells is provided for production of the antibody, regulation of the cellular immune system, detection of exogenous agents in the blood, detection of foreign cells of the host, etc. Non-limiting examples of immune cells of the lymphoid lineage comprise T cells, natural killer T (NKT) cells and precursors thereof, comprising embryonic stem cells and pluripotent stem cells (e.g., stem cells or pluripotent stem cells that are differentiated into lymphoid cells). T cells may be mature lymphocytes in the thymus and are primarily responsible for cell-mediated immunity. T cells are involved in the adaptive immune system. T cells can be any type of T cells, including but not limited to helper T cells, cytotoxic T cells, memory T cells (including central memory T cells, stem-like memory T cells (or stem-like memory T cells), and two effector memory T cells, e.g., TEM cells and TEMRA cells), regulatory T cells (also referred to as suppressor T cells), natural killer T cells, mucosa-associated invariant T cells, γδ T cells or αβ T cells. Cytotoxic T cells (CTL or killer T cells) are T lymphocytes capable of inducing the death of infected somatic or tumor cells. The subject's own T cells can be engineered to express the chimeric polypeptide of the present application and the CAR with the chimeric polypeptide binding triggered transcriptional activity. In an embodiment, the cells of the present application are selected from T cells, NK cells, cytotoxic T cells, NKT cells, macrophages, CIK cells, and stem cell-derived immune cells or combinations thereof. In an embodiment, the immune cells are T cells. In an embodiment, the T cells may be CD4+ T cells and/or CD8+ T cells. In an embodiment, the immune cells are CD3+ T cells. In an embodiment, the cells in the composition of the present application comprise a cell population collected from PBMC cells stimulated by CD3 magnetic beads.

The cells (e.g., T cells) of the present application can be autologous, non-autologous (e.g., allogeneic), or derived in vitro from engineered progenitor or stem cells. It can be obtained from a number of sources, including peripheral blood mononuclear cells (PBMC), bone marrow, lymph node tissue, umbilical cord blood, thymus tissue, tissue from the infected site, ascites, pleural effusion, spleen tissue, and tumors.

In certain aspects of the present application, T cells can be obtained from a blood sample collected from a subject using any number of techniques known to those of skill in the art, such as the Ficoll^{™} separation technique. In a preferred aspect, the cells from the circulating blood of the individual are obtained by apheresis. Products of apheresis usually comprise lymphocytes, comprising T cells, monocytes, granulocytes, B cells, other nucleated white blood cells, red blood cells, and platelets. In one aspect, cells collected by apheresis may be washed to remove the plasma fraction and placed in an appropriate buffer or culture medium for subsequent processing steps. Multiple rounds of selection may also be used in the context of the present application. In some aspects, it may be necessary to perform a selection procedure and use "unselected" cells during activation and expansion. "Unselected" cells can also undergo other rounds of selection.

The cells of the present application are capable of modulating the tumor microenvironment.

The source of unpurified CTL can be any source known in the art, such as bone marrow, fetal, neonatal or adult or other source of hematopoietic cells, such as fetal liver, peripheral blood or umbilical cord blood. Cells can be isolated using various techniques. For example, non-CTLs can be initially removed by negative selection. The mAb is particularly useful for identifying markers associated with specific cell lineages and/or differentiation stages of positive and negative selection. Most of the terminally differentiated cells can be removed initially by relatively rough separation. For example, magnetic bead separation may be used initially to remove large numbers of irrelevant cells. In certain embodiments, at least about 80%, usually at least about 70%, of the total hematopoietic cells will be removed prior to isolating the cells.

Separation procedures include, but are not limited to, density gradient centrifugation; resetting; coupling to particles altering cell density; magnetic separation with antibody-coated magnetic beads; affinity chromatography; using cytotoxic agents combined with or in combination with mAb, including but not limited to complement and cytotoxins; and panning with antibodies attached to a solid substrate (e.g., plate, chip, elutriation) or any other convenient technique.

Techniques for separation and analysis include, but are not limited to, flow cytometry, which can have varying degrees of sophistication, such as multiple color channels, low- and obtuse-angle light-scattering detection channels, and impedance channels.

Cells can be selected for dead cells by using dyes associated with dead cells, such as propidium iodide (PI). In certain embodiments, cells are harvested in medium comprising 2% fetal calf serum (FCS) or 0.2% bovine serum albumin (BSA), or any other suitable medium such as sterile isotonic medium.

### 5. Transgenic organisms

The present application provides a non-human transgenic organism comprising a nucleic acid encoding the chimeric polypeptide of the present application. The non-human transgenic organism of the present application comprises a genome that has been genetically modified to comprise the nucleic acid encoding the chimeric polypeptide of the present application.

### 6. Method

The present application provides a method for regulating an activity of an engineered cell expressing the chimeric polypeptide of the present application, comprising: contacting the engineered cell comprising the chimeric polypeptide of the present application with a target molecule, the binding of the target molecule to the binding domain of the chimeric polypeptide inducing cleavage of the protease cleavage site of the receptor regulatory domain of the chimeric polypeptide, releasing the intracellular domain, wherein release of the intracellular domain regulates the activity of the engineered cell. In an embodiment, the contacting is performed in vivo, ex vivo or in vitro. In an embodiment, the target molecule is on the surface of the target cell, immobilized on an insoluble substrate, present in an extracellular matrix, present in an artificial matrix, or soluble. In an embodiment, release of the intracellular domain regulates proliferation of engineered cells. In an embodiment, release of the intracellular domain regulates apoptosis of the engineered cell. In an embodiment, release of the intracellular domain induces cell death by a mechanism other than apoptosis. In an embodiment, release of the intracellular domain regulates gene expression in engineered cells through transcriptional regulation, chromatin regulation, translation, transport, or post-translational processing. In an embodiment, release of the intracellular domain regulates differentiation of engineered cells. In an embodiment, release of the intracellular domain regulates migration of engineered cells. In an embodiment, the release of the intracellular domain regulates the expression and secretion of the molecule from the engineered cell. In an embodiment, the release of the intracellular domain regulates the adhesion of the engineered cell to the target cell or to the extracellular matrix. In an embodiment, release of the intracellular domain induces re-expression of the gene product in the engineered cell. Release of the intracellular domain induces re-expression of the gene product in engineered cells. In an embodiment, the gene product is a transcriptional activator, a transcriptional repressor, a chimeric antigen receptor, a translational regulator, a cytokine, a hormone, a chemokine, or an antibody. In an embodiment, the released transcription factors regulate the differentiation of the engineered cells, which are immune cells, stem cells, progenitor cells or precursor cells. In an embodiment, the method is used to treat tumors. The application provides a method for activating T cells, comprising: contacting T cells as described herein (wherein the T cells are genetically modified with one or more nucleic acids comprising a nucleotide sequence encoding: i) a chimeric polypeptide involved in the present application; and ii) a CAR) with an immobilized antigen, wherein the domain of the chimeric polypeptide comprises an antibody specific for a first target molecule, and wherein the contacting results in release of the intracellular domain (e.g., a transcriptional activator) of the chimeric polypeptide, and expression of the CAR polypeptide in T cells, wherein the CAR polypeptide provides activation of the T cells following binding of a second target molecule. In an embodiment, the first target molecule and the second target molecule are different tumor antigens; or the first target molecule is a tissue-specific molecule and the second target molecule is a tumor antigen.

The present disclosure provides a method for regulating an activity of an engineered cell, comprising: contacting the engineered cell with an antigen immobilized on the surface, wherein the engineered cell expressing the chimeric polypeptide of the present disclosure can result in release of the intracellular domain and the activity of the engineered cell. In some cases, the intracellular domain is a transcription factor that regulates cell differentiation.

The present disclosure provides a method for locally regulating an activity of an engineered cell, comprising: expressing in the cell the chimeric polypeptide of the present application; after the engineered cell contacts with a target molecule, releasing the intracellular domain to regulate the activity of the engineered cell: expression of gene products of cells, proliferation of cells, apoptosis of cells, non-apoptotic death of cells, differentiation of cells, dedifferentiation of cells, migration of cells, secretion of molecules from cells, and cell adhesion.

In an embodiment, the present application provides a method for treating a tumor in a subject suffering from the tumor, comprising: i) genetically modifying T lymphocytes or NKT cells obtained from an individual with a vector comprising a nucleic acid encoding a chimeric polypeptide of the present application, or a chimeric polypeptide and it binding triggered transcriptional activity CAR, wherein the chimeric polypeptide is specific to an antigen on a tumor cell in the subject; ii) introducing the genetically modified T lymphocytes or NKT cells into the subject, wherein the genetically modified T lymphocytes or NKT cells recognize and kill tumor cells, thereby treating the tumor.

In an embodiment, the present application provides a method for inhibiting an activity of a target cell in a subject, comprising: administering to the subject a therapeutically effective amount of engineered cells expressing the chimeric polypeptide of the present application, the engineered cells inhibiting the activity of the target cell in the subject. In an embodiment, the target cells are tumor cells. In an embodiment, the target cells are acute myeloma leukemia cells, anaplastic lymphoma cells, astrocytoma cells, B cell cancer cells, breast cancer cells, colon cancer cells, ependymoma cells, esophageal cancer cells, glioblastoma cells, glioma cells, leiomyosarcoma cells, liposarcoma cells, liver cancer cells, lung cancer cells, mantle cell lymphoma cells, melanoma cells, neuroblastoma cells, non-small cell lung cancer cells, oligodendroglioma cells, ovarian cancer cells, pancreatic cancer cells, peripheral T-cell lymphoma cells, kidney cancer cells, sarcoma cells, gastric cancer cells, liver cancer cells, mesothelioma cells or sarcoma cells. In an embodiment, the target cell expresses a low level of the target molecule. In an embodiment, the engineered cell further comprises CAR, modifying TCR, an exogenous cytokine and/or a therapeutic monoclonal antibody triggered by the chimeric peptide binding target molecule for transcriptional activation. In an embodiment, the chimeric polypeptide specifically binds to a first target molecule, and the CAR and/or modifying TCR specifically binds to a second target molecule; the first and second target molecules in the target cell have expression heterogeneity, or the first target molecule is a tissue-specific molecule and the second target molecule is a tumor antigen. In an embodiment, the first target molecule is not a tumor antigen but has tissue-specific expression, and the second target molecule is a tumor antigen. In an embodiment, the positive rate of the first target molecule is lower than that of the second target molecule. In an embodiment, the method can improve the anti-tumor specificity of the engineered cells.

### 7. Administration

A composition comprising the chimeric polypeptide of the present application may be provided systemically or directly to a subject to induce and/or enhance an immune response to an antigen and/or treat and/or prevent a tumor, a pathogenic infection or an infectious disease. In an embodiment, the composition of the present application is injected directly into an organ of interest (e.g., an organ affected by a tumor). Alternatively, the composition of the present application is provided to the organ of interest indirectly, e.g., by administration to the circulatory system (e.g., vein, tumor vasculature). Expansion and differentiation agents may be provided before, simultaneously with or after administration of the composition to increase the production of T cells, NKT cells or CTL cells in vitro or in vivo.

Immune cells in the composition of the present application may include purified cell populations. Those skilled in the art can readily determine the percentage of immune cells of the present application in the population using various well-known methods, such as fluorescence activated cell sorting (FACS). Suitable ranges for purity are about 50% to about 55%, about 5% to about 60%, and about 65% to about 70% in the population comprising the immune cells of the present application. In certain embodiments, the purity is about 70% to about 75%, about 75% to about 80%, or about 80% to about 85%. In certain embodiments, the purity is about 85% to about 90%, about 90% to about 95%, and about 95% to about 100%. Dosages can be readily adjusted by those skilled in the art (e.g., decreased purity may require increased dosages). Cells may be introduced by injection, catheter, and the like.

The composition of the present application may be a pharmaceutical composition comprising the immune cells of the present application or progenitor cells and a pharmaceutically acceptable carrier thereof. Administration may be autologous or allogeneic. For example, immune cells or progenitor cells may be obtained from one subject and administered to the same subject or to a different compatible subject. Peripheral blood-derived immune cells or progeny thereof (e.g., in vivo, ex vivo, or in vitro sources) may be administered by local injection, including catheter administration, systemic injection, local injection, intravenous injection, or parenteral administration. The composition of the present application may be formulated in unit dose of injectable forms (solutions, suspensions, emulsions, etc.) for being administered.

### 8. Dosage Form

The composition comprising the chimeric polypeptide of the present application may conveniently be provided in the form of sterile liquid preparations, such as isotonic aqueous solutions, suspensions, emulsions, dispersions or viscous compositions, which may be buffered to a selected pH. Liquid formulations are generally easier to be prepared than gels, other viscous compositions, and solid compositions. Additionally, liquid compositions are somewhat more convenient for administration, especially by injection. On the other hand, viscous compositions can be formulated within an appropriate viscosity range to provide a longer contact time with a specific tissue. Liquid or viscous compositions may comprise a carrier, which may be a solvent or dispersion medium, comprising, for example, water, saline, phosphate-buffered saline, polyols (e.g., glycerol, propylene glycol, liquid polyethylene glycol, etc.), and a suitable mixture thereof.

Sterile injectable solutions can be prepared by incorporating the immune cells in the composition of the present application in the required amount of an appropriate solvent, and incorporating different amounts of other ingredients as required. Such composition may be mixed with suitable carriers, diluents or excipients such as sterile water, physiological saline, glucose, dextrose and the like. The composition may also be lyophilized. The composition may comprise auxiliary substances such as wetting, dispersing or emulsifying agents (e.g., methylcellulose), pH buffering agents, gelling or viscosity-increasing agents, preservatives, flavoring agents, pigments, etc., depending on the administration route and desired formulation.

Various additives, including antimicrobial preservatives, antioxidants, chelating agents, and buffering agents, may be added to enhance the stability and sterility of the compositions. Prevention of the action of microorganisms can be ensured by various antibacterial and antifungal agents, for example, parabens, chlorobutanol, phenol, sorbic acid, and the like. Absorption of the injectable pharmaceutical forms can be prolonged by the use of agents which delay absorption, for example, aluminum monostearate and gelatin. However, any vehicle, diluent or additive used will have to be compatible with the genetically modified immune cells or progenitors thereof.

The compositions may be isotonic, i.e. may have the same osmotic pressure as blood and/or tear fluid. The desired isotonicity of the compositions can be achieved by using sodium chloride or a pharmaceutically acceptable agent thereof, such as dextrose, boric acid, sodium tartrate, propylene glycol or other inorganic or organic solutes. Sodium chloride may be particularly suitable for buffers containing sodium ions.

If desired, a pharmaceutically acceptable thickener may be used to maintain the viscosity of the composition at a selected level. For example, methylcellulose is readily and economically available and easy to use. Other suitable thickeners include, for example, xanthan gum, carboxymethylcellulose, hydroxypropylcellulose, carbomer, and the like. The concentration of the thickener can depend on the reagent chosen. It is important to use the amount that will achieve the chosen viscosity. Obviously, the selection of suitable carriers and other additives will depend on the exact administration route and the nature of the specific dosage form, e.g., liquid dosage form (e.g., whether the composition is formulated as a solution, suspension, gel, or other liquid form, e.g. time-release or liquid-filled form).

The number of cells in the composition to be administered will vary for the subject being treated. More potent cells may be administered in smaller numbers. The precise determination of an effective dose can be determined according to each subject's individual factors, including its size, age, sex, weight and the state of the subject. Dosages can be readily determined by those skilled in the art from the present application and knowledge in the art.

Those skilled in the art can readily determine the amount of cells and optional additives, vehicles and/or carriers in the composition and to be administered in the method. Typically, any additive (other than one or more active cells and/or one or more reagents) is present in 0.001% to 50% by weight of the solution in phosphate-buffered saline, and the active ingredient is present in order of micrograms to milligram, for example, about 0.0001 wt% to about 5 wt%, about 0.0001 wt% to 1 wt%, about 0.0001 wt% to about 0.05 wt%, or about 0.001 wt% to about 20 wt%, about 0.01 wt% to about 10 wt% % or about 0.05 wt% to about 5 wt%. For any composition to be administered to animals or humans, the following results may be determined: toxicity, for example by determining the lethal dose (LD) and LD50 in a suitable animal model, e.g. rodents such as mice; the dose of the composition, wherein the concentration of the components and the time of application of the composition elicit an appropriate response.

### 9. Treatment method

The present application provides a method for inducing and/or increasing an immune response in a subject in need of a composition of the present application. The composition comprising the chimeric polypeptide of the present application may be used to treat and/or prevent a tumor in the subject. The compositions of the present application may be used to prolong the survival of the subject suffering from the tumor. The composition of the present application may also be used to treat and/or prevent a pathogen infection or other infectious diseases in the subject, such as the immunocompromised human. Such method comprises administering an effective amount of the composition of the present application to achieve a desired effect, whether alleviating an existing disorder or preventing recurrence. For treatment, the amount administered is an amount that is effective to produce the desired effect. The effective amount may be provided in one or more administrations. The effective amount may be provided in large doses or by continuous infusion.

In an embodiment, the composition comprising the chimeric polypeptide of the present application may be used to treat the subject with low surface antigen expression levels of tumor cells, e.g., due to recurrence of the disease, wherein the subject has undergone treatment that resulted in residual tumor cells. In certain embodiments, the tumor cell has a low density of the target molecule on the surface of the tumor cell.

In an embodiment, the composition comprising the chimeric polypeptide of the present application may be used to treat a subject suffering with recurrence of disease, wherein the subject has undergone immune cells (e.g., T cells) comprising alone administrated CAR. In an embodiment, the tumor cells have a low density of tumor-specific antigens on the surface of the tumor cells. Such methods comprise administering an effective amount of a composition of the present application to achieve the desired effect, alleviation of an existing disorder or prevention of recurrence.

An "effective amount" (or "therapeutically effective amount") is an amount sufficient to produce beneficial or desired clinical results following treatment. The effective amount may be administered to a subject in one or more doses. For treatment, the effective amount is an amount sufficient to alleviate, ameliorate, stabilize, reverse or slow the progression of the disease or otherwise reduce the pathological consequences of the disease. The effective amount is generally determined by a physician on a case-by-case basis and is within the competence of those skilled in the art. Several factors are generally considered when determining a suitable dosage to achieve the effective amount.

These factors comprise the age, sex and weight of the subject, the disease being treated, the severity of the disease, and the form and effective concentration of the composition of the present application being administered.

For adoptive immunotherapy using antigen-specific T cells, cell doses in the range of about 10⁶ to 10¹⁰ are typically infused. After the immune cells of the present application are administered to a host and then differentiated, T cells specific for a specific antigen are induced. The compositions of the present application may be administered by any method known in the art, including but not limited to intravenous, subcutaneous, intranodal, intratumoral, intrathecal, intrapleural, intraperitoneal administration, and direct administration to the thymus.

The present application provides a method for treating and/or preventing a tumor in a subject. The method may comprise administering to the subject suffering from the tumor an effective amount of a composition of the present application.

Non-limiting examples of tumors include blood cancers (such as leukemia, lymphoma, and myeloma), ovarian cancer, breast cancer, bladder cancer, brain cancer, colon cancer, intestinal cancer, liver cancer, lung cancer, pancreatic cancer, prostate cancer, skin cancer, gastric cancer, glioblastoma, laryngeal cancer, melanoma, neuroblastoma, adenocarcinoma, glioma, soft tissue sarcomas, and various carcinomas (including prostate carcinoma and small cell lung carcinoma). Non-limiting examples of tumors include, but are not limited to, astrocytoma, fibrosarcoma, myxosarcoma, liposarcoma, oligodendroglioma, ependymoma, medulloblastoma, primitive neuroectodermal tumor (PNET), chondrosarcoma, osteosarcoma, pancreatic ductal adenocarcinoma, small and large cell lung adenocarcinoma, chordoma, angiosarcoma, endothelial sarcoma, squamous cell carcinoma, bronchoalveolar carcinoma, epithelial adenocarcinoma and its liver metastases, lymphatic sarcoma, lymphangioendothelial sarcoma, liver cancer, cholangiocarcinoma, synovial tumor, mesothelioma, Ewing's sarcoma, rhabdomyosarcoma, colon cancer, basal cell carcinoma, sweat gland carcinoma, papillary carcinoma, sebaceous gland carcinoma, thyroid carcinoma, cyst gland carcinoma, medullary carcinoma, bronchial carcinoma, renal cell carcinoma, cholangiolar carcinoma, choriocarcinoma, seminoma, embryonal carcinoma, Wilms' tumor, testicular tumor, medulloblastoma, craniopharyngioma, ependymoma, pineal tumor, hemangioblastoma, acoustic neuroma, oligodendroglioma, meningioma, neuroblastoma, retinoblastoma, leukemia, multiple myeloma, Waldenstrom's macroglobulinemia and heavy chain diseases, breast tumors such as ductal and lobular adenocarcinomas, squamous and adenocarcinomas of the cervix, epithelial carcinomas of the uterus and ovaries, prostate adenocarcinomas, transitional squamous cell carcinomas of the bladder, B and T-cell lymphomas (nodular and diffuse) plasmacytoma, acute and chronic leukemia, malignant melanoma, soft tissue sarcoma, and leiomyosarcoma. In certain embodiments, the tumor is selected from the group consisting of blood cancers (e.g., leukemia, lymphoma, and myeloma), ovarian cancer, prostate cancer, breast cancer, bladder cancer, brain cancer, colon cancer, intestinal cancer, liver cancer, lung cancer, pancreatic cancer, prostate cancer, skin cancer, stomach cancer, glioblastoma, and throat cancer. In an embodiment, the composition of the present application may be used for the treatment and/or prevention of conventional treatment unsuitable or relapsed refractory solid tumors, such as liver cancer, lung cancer, breast cancer, ovarian cancer, kidney cancer, thyroid cancer, gastric cancer, colorectal cancer. In an embodiment, the tumor is a hematological tumor.

The therapeutic goals of the composition of the present application may comprise alleviating or reversing disease progression and/or alleviating side effects, or the therapeutic goals include reducing or delaying the risk of recurrence.

The present application provides a method for treating and/or preventing a pathogen infection (e.g., viral, bacterial, fungal, parasitic, or protozoan infection) in, e.g., an immunocompromised subject. The method may comprise administering an effective amount of a composition of the present application to a subject suffering from the pathogenic infection. Exemplary viral infections that are amenable to treatment include, but are not limited to, cytomegalovirus, Epstein-Barr virus, human immunodeficiency virus, and influenza virus infections.

The term "enhancing" refers to as ability which allowing a subject or a tumor cell to improve the respond to the treatments disclosed herein. For example, enhanced response can include 5%, 10%, 15%, 20%, 25%, 30%, 35%, 40%, 45%, 50%, 55%, 60%, 65%, 70%, 75%, 80%, 85%, 90%, 95%, or 98% or more increase in responsiveness. As used herein, "enhancing" may also refer to as increasing the number of subjects who respond to treatment, e.g., immune cell therapy. For example, enhanced response may refer to the total percentage of subjects who respond to treatment, wherein the percentage is 5%, 10%, 15%, 20%, 25%, 30%, 35%, 40%, 45%, 50%, 55%, 60%, 65%, 70%, 75%, 80%, 85%, 90%, 95%, or 98% or more.

### 10. Kit

The present application provides a kit for inducing and/or enhancing immune response and/or treating and/or preventing tumor or pathogen infection in a subject. In an embodiment, the kit comprises an effective amount composition and pharmaceutical composition of the chimeric polypeptide of the present application. In an embodiment, the kit comprises a sterile container; such container may be a box, an ampoule, a bottle, a vial, a tube, a bag, a sachet, a blister pack, or other suitable forms of container known in the art. Such container may be made of plastic, glass, laminated paper, metal foil, or other materials suitable for containing the medicine. In an embodiment, the kit comprises a nucleic acid molecule encoding the CAR of the present application, which recognizes an antigen of interest in an expressible form, and may optionally be included in one or more vectors.

In an embodiment, a composition and/or nucleic acid molecule of the present application are provided together with an instruction manual for administering the composition or nucleic acid molecule to a subject suffering from a tumor or a pathogen or an immune disease or developing a tumor or a pathogen or an immune disease. The instruction manual generally comprises information about the use of the composition in the treatment and/or prevention of the tumor or the pathogenic infection. In an embodiment, the instruction manual comprises at least one of the following: a description of the therapeutic agent; a dose table and administration for the treatment or prevention of the tumors, the pathogenic infection, or the immune disease or a symptom thereof; precautions; warnings; indications; incompatibility; medication information; adverse reactions; animal pharmacology; clinical studies; and/or references. These instruction manuals may be printed directly on the container, or affixed to the containers as labels, or provided within or together with the container as separate sheets, booklets, cards or file folders.

### 11. Therapeutic or pharmaceutical use

In the present application, the methods described in the present application can also be interpreted as therapeutic use, that is, the methods described in the present application can be considered as pharmaceutical uses of the composition, nucleic acid molecule or engineered cell of the present application in treatment or in the preparation of corresponding therapeutic medicine. In an embodiment, the present application relates to the use of the above-mentioned engineered cells or nucleic acid molecules in regulating the activity of engineered cells or the use of the above-mentioned engineered cells in the preparation of a medicine for regulating the activity of engineered cells; the present application relates to use of the above-mentioned engineered cells or nucleic acid molecules in activation of engineered cells or use of above-mentioned engineered cells in the preparation of a medicine for activating engineered cells; the present application relates to the use of the above-mentioned engineered cells or nucleic acid molecules in inhibiting the activity of target cells in a subject or in the preparation of a medicine for inhibiting the activity of target cells in a subject; the present application relates to use of the above-mentioned engineered cells or nucleic acid molecules in improving or treating the health condition of a subject in need thereof or in the preparation of a medicine for improving or treating the health condition of a subject in need thereof. All that is described above in the present application is equally applicable for therapeutic or pharmaceutical use.

The present application comprises, for example, CAR T cells, preparation methods and antibodies thereof disclosed in the Chinese patent application publicationnumber CN107058354A , CN107460201A, CN105194661A, CN105315375A, CN105713881A, CN106146666A, CN106519037A, CN106554414A, CN105331585A, CN106397593A, CN106467573A, CN104140974A, CN108884459A, CN107893052A, CN108866003A, CN108853144A, CN109385403A, CN109385400A, CN109468279A, CN109503715A, CN109908176A, CN109880803A, CN110055275A, CN110123837A, CN110438082A, CN110468105A, and international patent application publicationnumber WO2017186121A1, WO2018006882A1, WO2015172339A8. WO2018/018958A1, WO2014180306A1, WO2015197016A1, WO2016008405A1, WO2016086813A1, WO2016150400A1, WO2017032293A1, WO2017080377A1, WO2017186121A1, WO2018045811A1, WO2018108106A1, WO 2018/219299 , WO2018/210279, WO2019/024933, WO2019/114751, WO2019/114762, WO2019/141270, WO2019/149279, WO2019/170147A1, WO 2019/210863, WO2019/219029.

### Example 1: Construction of chimeric polypeptides synJagged2EC, synEphrinB2EC, synEphrinB2EC-APLP2(TM)

This example constructs compositions of various exemplary chimeric polypeptide of chimeric polypeptides used in the present application (Table 1).

**Table 1**

| Name of vector/chimeric polypeptide | Binding domain | Receptor regulatory domain | | Intracellular |
|---|---|---|---|---|
| | | extracellular region | transmembrane region (STS) | |
| GPC3-synJagged2EC | antiGPC3-scFv | Jagged2 | Notch1 (Notch1) | GAL4-VP64 |
| GPC3-synEphrinB2EC | antiGPC3-scFv | EphrinB2 | Notch1 (Notch1) | GAL4-VP64 |
| GPC3-synEphrinB2EC-2 | antiGPC3-scFv | EphrinB2 | Notch1 (Notch2) | GAL4-VP64 |
| GPC3-synEphrinB2EC-3 | antiGPC3-scFv | EphrinB2 | Notch1 (Notch3) | GAL4-VP64 |
| GPC3-synEphrinB2EC-4 | antiGPC3-scFv | EphrinB2 | Notch1 (Notch4) | GAL4-VP64 |
| GPC3-synEphrinB2EC-5 | antiGPC3-scFv | EphrinB2 | Notch2 (Notch2) | GAL4-VP64 |
| GPC3-synEphrinB2EC-6 | antiGPC3-scFv | EphrinB2 | Notch3 (Notch3) | GAL4-VP64 |
| GPC3-synEphrinB2EC-7 | antiGPC3-scFv | EphrinB2 | Notch4 (Notch4) | GAL4-VP64 |
| GPC3-synEphrinB2EC-8 | antiGPC3-scFv | EphrinB2 | huNotch1 (huNotch1) | GAL4-VP64 |
| GPC3-synEphrinB2EC-APLP2T M | antiGPC3-scFv | EphrinB2 | APLP2 (APLP2) | GAL4-VP64 |
| GPC3-synEphrinB2EC-del1 | antiGPC3-scFv | EphrinB2-del1 | Notch1 (Notch1) | GAL4-VP64 |
| GPC3-synEphrinB2EC-del2 | antiGPC3-scFv | EphrinB2-del2 | Notch1 (Notch1) | GAL4-VP64 |
| GPC3-synEphrinB2EC-del3 | antiGPC3-scFv | EphrinB2-del3 | Notch1 (Notch1) | GAL4-VP64 |
| GPC3-synEphrinB2EC-del3-2 | antiGPC3-scFv | EphrinB2-del3 | Notch1 (Notch2) | GAL4-VP64 |
| GPC3-synEphrinB2EC-del23 | antiGPC3-scFv | EphrinB2-del23 | Notch1 (Notch1) | GAL4-VP64 |
| EGFRvIII-synEphrinB2EC | antiEGFR vIII -scFv | EphrinB2 | Notch1 (Notch1) | GAL4-VP64 |
| Mesothelin-synEphrinB2EC | antiMesothelin-scFv | EphrinB2 | Notch1 (Notch1) | GAL4-VP64 |
| FAP-synEphrinB2EC | antiFAP-scFv | EphrinB2 | Notch1 (Notch1) | GAL4-VP64 |
| Claudin18.2-synEphrinB2EC | antiClaudin18.2-scFv | EphrinB2 | Notch1 (Notch1) | GAL4-VP64 |
| CLL1-synEphrinB2EC | antiCLL1-scFv | EphrinB2 | Notch1 (Notch1) | GAL4-VP64 |
| CLL1-synEphrinB2EC-APLP2T M | antiCLL1-scFv | EphrinB2 | APLP2 (APLP2) | GAL4-VP64 |
| CD123-synEphrinB2EC | AntiCD123-scFv | EphrinB2 | Notch1 (Notch1) | GAL4-VP64 |

Exemplarily, the fragment sequences in Table 1 above are as follows: antiGPC3-scFv (SEQ ID No: 42), antiEGFRvIII-scFv (SEQ ID No: 43), antiMesothelin-scFv (SEQ ID No: 44), antiFAP-scFv (SEQ ID No: 45), antiClaudin18.2-scFv (SEQ ID NO: 46), antiCLL1-scFv (SEQ ID No: 47), Jagged2 extracellular region (SEQ ID No: 1, 2), EphrinB2 extracellular region (SEQ ID No:3, 4), EphrinB2-del1 extracellular region (SEQ ID No:5, 6), EphrinB2-del2 extracellular region (SEQ ID No:7, 8), EphrinB2-del3 extracellular region (SEQ ID No: 9, 10), EphrinB2-del23 extracellular region (SEQ ID No:11, 12), mouse Notch1 (Notch1STS) transmembrane region (SEQ ID No:13), mouse Notch2 transmembrane region (Notch2STS) (SEQ ID No: 14), mouse Notch3 transmembrane region (Notch3STS) (SEQ ID No: 15), mouse Notch4 transmembrane region (Notch4STS) (SEQ ID No: 16), human Notch1 transmembrane region (Notch1STS) (SEQ ID No: 17) , human Notch2 transmembrane region (Notch2STS) (SEQ ID No: 18), human Notch3 transmembrane region (Notch3STS) (SEQ ID No: 19), human Notch4 transmembrane region (Notch4STS) (SEQ ID No: 20), APLP2 transmembrane region (APLP2STS) (SEQ ID No: 21), Notch1STS (SEQ ID No: 22), Notch2STS (SEQ ID No: 23), Notch3STS (SEQ ID No: 24), Notch4STS (SEQ ID No: 25), STS of APLP2 (SEQ ID No: 26) and GAL4-VP64 (SEQ ID No: 27), Notch of mouse is represented by the English symbol Notch, and Notch of human is represented by the English symbol huNotch.

After the above chimeric polypeptides were introduced into Jurkat cells (purchased from the Cell Bank of the Chinese Academy of Sciences), the expression was detected by flow cytometry using biotin-labeled antigen polypeptide+PE-labeled streptavidin. The results showed that the above chimeric polypeptides were stably expressed on the cell membrane..

In this example, BFP (genebank ID: QJR97815.1) was inserted behind the UAS-CMV promoter (SEQ ID NO: 49) regulated by Gal4-VP64 in Table 1, to act as a reporter gene activated by the chimeric polypeptide. In this example, green fluorescent protein (GFP) regulated by the pGK promoter (Addgene #79120, 7721-8220 bp) was inserted downstream of BFP, to act as a transduction positive marker. The GFP protein sequence is shown in genebank ID: UDY80669.1. In this example, the UAS-BFP-PGK-GFP fragment comprising UAS-CMV, BFP, pGK, and GFP nucleic acid sequences in sequence connection was transducted into Jurkat cells to establish Jurkat response cells.

### Example 2: synJagged2EC, synEphrinB2EC, synEphrinB2EC-APLP2(TM) regulating gene expression

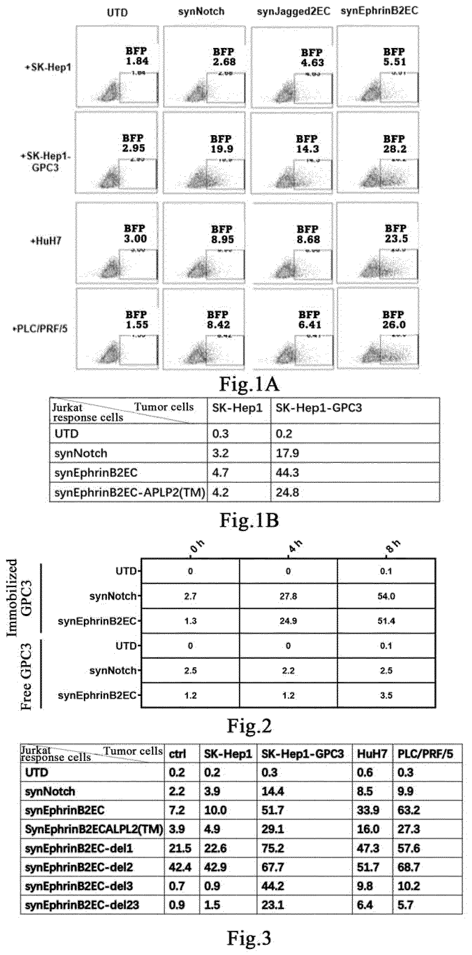

The Jurkat response cells introduced with the chimeric polypeptides targeting GPC3 respectively were mixed with liver cancer cells at 1:1 (wherein, SK-Hep1 was negative for GPC3, SK-Hep1-GPC3 was exogenously overexpressed GPC3 protein, HuH7 and PLC/PRF/5 were positive for GPC3) and were co-incubated for 24 hours, and BFP was detected by flow cytometry. FIGs. 1A and 1B (values indicated the percentage of BFP-positive cells) showed that after co-incubation with target cells, compared to synNotch (modified from plasmid Addgene #79125, the antiCD19 scFv sequence was replaced by antiGPC3-scFv sequence), synJagged2EC (SEQ ID No: 51), synEphrinB2EC (SEQ ID No: 52), and synEphrinB2EC-APLP2 (TM) (SEQ ID No: 53) induced comparable or significantly increased level of BFP expression.

### Example 3: Antigen co-incubation experiment

Coating group: GPC3 antigen was pre-coated in 96-well plate (5 µg/mL overnight at 4°C), and 50,000 Jurkat response cells expressing chimeric polypeptide targeting GPC3 were plated in each well. Lysis group: 50,000 Jurkat response cells expressing chimeric polypeptide targeting GPC3 were plated in each well and GPC3 antigen (5 µg/mL) was directly added to the culture solution. BFP was detected by flow cytometry at 0, 4, 8, 24, and 32 hours of co-incubation the antigen with the cells. FIG. 2 showed that after co-incubation with the coated antigen, the expression of BFP in the cells of each group was comparable. Cells incubated with lysed antigen had almost no detectable BFP.

### Example 4. SynEphrinB2EC-del comprising a truncation of the extracellular region of EphrinB2 regulating gene expression

Referring to Examples 1 and 2, Jurkat response cells expressing GPC3-synEphrinB2EC-del1 (SEQ ID No: 54), GPC3-synEphrinB2EC-del2 (SEQ ID No: 55), GPC3-synEphrinB2EC-del3 (SEQ ID No: 56), GPC3- synEphrinB2EC-del23 (SEQ ID No: 57) were constructed, and BFP expression was detected after GPC3 stimulation. FIG. 3 showed that all of the chimeric polypeptides targeting GPC3, comprising the full-length of the extracellular region of EphrinB2 or a truncation thereof, regulate gene expression. Leakage of synEphrinB2EC-del3 or synEphrinB2EC-del23 induced expression was significantly reduced.

### Example 5. Response of synEphrinB2EC, synEphrinB2EC-APLP2(TM) to different concentrations of antigen stimulation

Biotin-labeled GPC3 antigen polypeptide was dissolved in PBS buffer (10 µg/mL), and diluted twice with PBS to a concentration of 0.019531 µg/mL, and 50 µL of each concentration of the antigen was added to a 96-well plate coated with streptavidin (Thermo 15500), incubated at room temperature for 2h for GPC3 antigen coating.

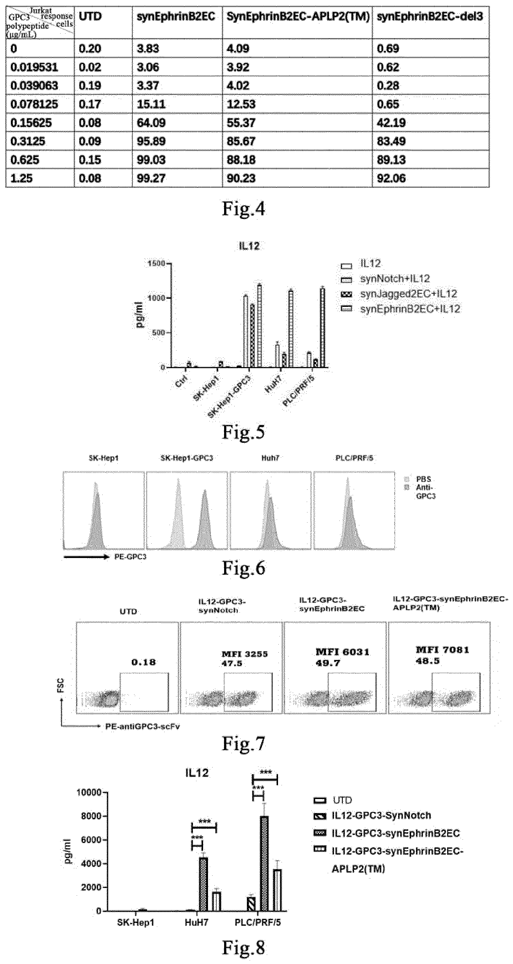

40,000 Jurkat response cells expressing chimeric polypeptide targeting GPC3 were added to the wells coated with different concentrations of GPC3 antigen, and cultured at 37°C for 24 hours, and BFP was detected by flow cytometry (FIG. 4).

### Example 6: synJagged2EC, synEphrinB2EC regulating IL12 expression

IL12 was inserted into downstream of the UAS-CMV promoter, and GFP regulated by the pGK promoter was connected with the downstream of IL12. The obtained fragment UAS-IL12-PGK-GFP was introduced into T cells simultaneously or successively with the chimeric polypeptide targeting GPC3, and then respectively mixed and co-incubated with liver cancer cells at 1:1 for 24 hours. IL12 in the culture supernatant was detected by ELISA. FIG. 5 shows that synJagged2EC, synEphrinB2EC induce comparable or increased IL12 expression compared to synNotch after co-incubation with target cells. Combined with FIG. 6, it can be seen that synEphrinB2EC induces increased transcription after incubation with GPC3 low-expressing cells.

### Example 7. The single vector system of synEphrinB2EC, synEphrinB2EC-APLP2(TM) regulating IL12 expression

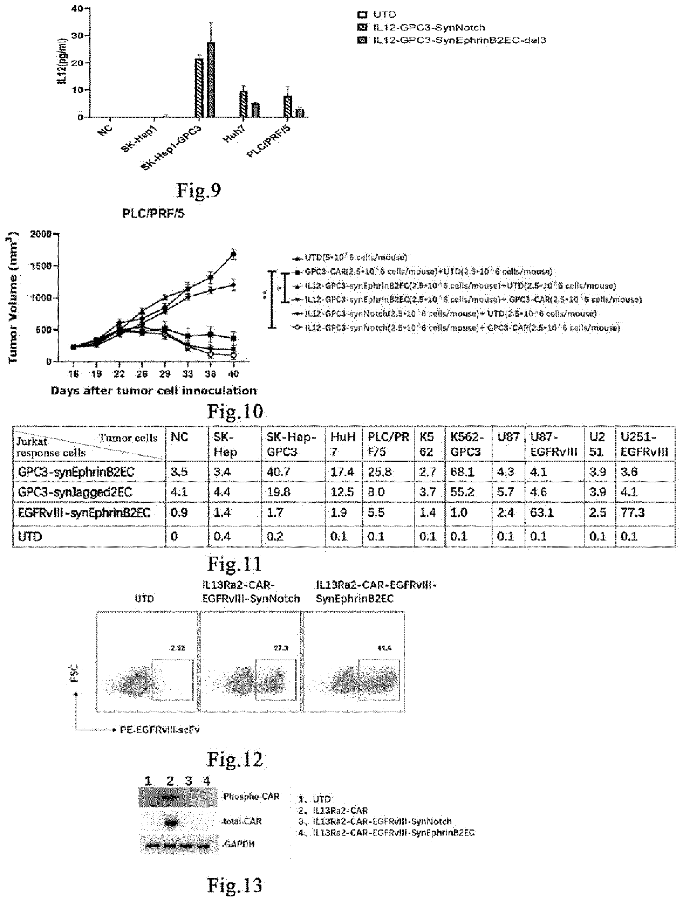

In Example 6, the chimeric polypeptide and the regulated expression gene were placed in two different vectors, and double virus infection was required to realize the regulation of the target gene by the chimeric polypeptide. In order to simplify the operation and improve the transduction efficiency, IL12 was inserted downstream of the UAS-CMV promoter, and then integrated into the expression vector of the chimeric polypeptide to transduce T cells. FIG. 7 showed that the expression level of synEphrinB2EC and synEphrinB2EC-APLP2(TM) in the single vector system was higher than that of synNotch. It can be seen from Fig. 8 combined with FIG. 6 that after incubation with GPC3 low-expressing cells, synEphrinB2EC or synEphrinB2EC-APLP2(TM) induced higher transcription than synNotch. FIG. 9 showed that after incubation with GPC3 low-expressing cells, the induced expression level of synEphrinB2EC-del3 was low, but after incubation with GPC3 high-expressing cells, its induced expression level was comparable to that of synNotch, indicating that synEphrinB2EC-del3 can more effectively distinguish low expression and high expression of antigen.

### Example 8. GPC3-synEphrinB2EC regulating IL12 expression in synergy with CAR-T cells to treat tumors

In vivo anti-tumor experiments using the T cells of GPC3-synEphrinB2EC that regulates the expression of IL12 prepared in Example 7, and the T cells expressing GPC3-CAR (SEQ ID NO: 60).

NPG mice were subcutaneously inoculated with PLC/PRF/5 liver cancer cells, and were divided into groups as illustrated after tumor formation and given the corresponding number of cells. The tumor volume and body weight were measured. FIG. 10 shows that synEphrinB2EC, which regulates the expression of IL12, plays an anti-tumor effect in synergy with GPC3-CAR-T cells.

### Example 9: EGFRvIII-synEphrinB2EC regulating gene expression

The Jurkat response cells comprising the chimeric polypeptide targeting EGFRvIII (SEQ ID NO: 61) were co-incubated with glioma cells at a ratio of 1:1 for 24 hours, and BFP was detected by flow cytometry (FIG. 11).

### Example 10. CAR-T Cells comprising synEphrinB2EC regulating CAR Expression

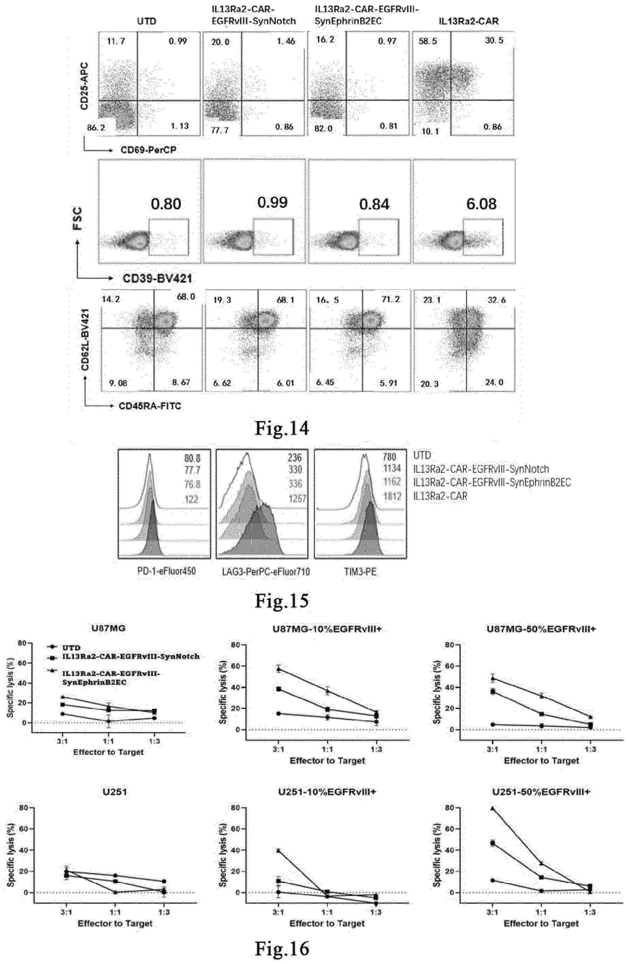

IL13Ra2-CAR (SEQ ID NO: 62) was inserted downstream of the UAS-CMV promoter, and then integrated into chimeric polypeptide expressing vectors, respectively, to transduce T cells, to prepare T cells with regulation of IL13Ra-CAR expression by chimeric polypeptide targeting EGFRvIII(FIG. 12), which were cultured in vitro for 14 days. CAR expression and phosphorylation levels were detected by Western blot (FIG. 13); cells were labeled with CD25, CD69, PD1, LAG3, TIM3 and CD39, CD45RA, CD62L antibodies, respectively, and detected by flow cytometry (FIG. 14, 15).

### Example 11. In vitro and in vivo killing of T cells comprising EGFRvIII-synEphrinB2EC regulating IL13Ra2-CAR expression

Target cells: U87, U87-10% EGFRvIII (90% U87+10% U87-EGFRvIII), U87-50% EGFRvIII (50% U87+50% U87-EGFRvIII), U251, U251-10% EGFRvIII (90% U251+10% U251-EGFRvIII), U251-50% EGFRvIII (50% U251+50% U251-EGFRvIII).

Co-culture was performed at an effect-to-target ratio of 3:1, 1:1, 1:3 respectively, and LDH in the supernatant was detected or target cell killing was monitored in real time by xCELLigence RTCA . FIG. 16 shows that IL13Ra2-CAR-T cells expressing EGFRvIII-synEphrinB2EC can kill EGFRvIII antigen heterogeneous tumors, and also have a killing effect on tumor cell populations with low EGFRvIII positive rate.

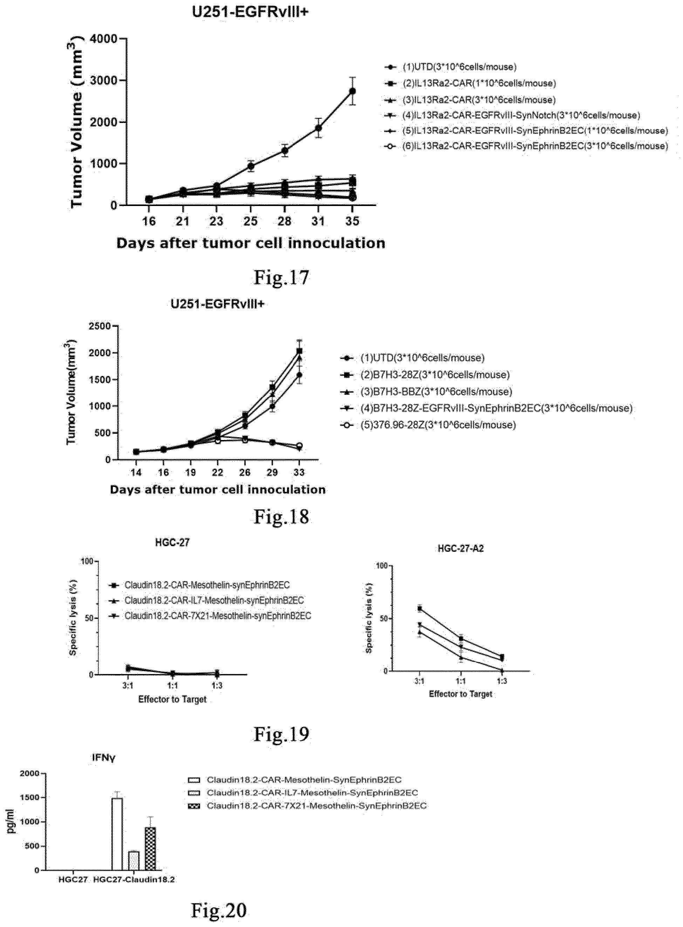

NPG mice were subcutaneously inoculated with U251-EGFRvIII cells, and were divided into groups as illustrated after tumor formation and given the corresponding number of cells. The tumor volume and body weight were measured. FIG. 17 showed the anti-tumor effect of IL13Ra2-CAR-T expressing EGFRvIII-synEphrinB2EC in vivo.

### Example 12. In vivo killing of B7H3-CAR-T cells expressing EGFRvIII-synEphrinB2EC chimeric polypeptide

T cells expressing B7H3-28Z (SEQ NO: 64), B7H3-BBZ (SEQ NO: 65), and 376.96-28Z (SEQ NO: 66) were constructed respectively. Referring to Example 10, T cells containing EGFRvIII-synEphrinB2EC regulating B7H3-28Z were constructed.

NPG mice were subcutaneously inoculated with U251-EGFRvIII cells, and were divided into groups as illustrated after tumor formation and given the corresponding number of cells. The tumor volume and body weight were measured. FIG. 18 showed that 376.96-28Z-T cells that recognize only human B7H3 have significant anti-tumor effects, while B7H3-28Z CAR-T and B7H3-BBZ CAR-T that recognize human and mouse B7H3 have poor efficacy. When the B7H3-28Z with low specificity was put under the regulation of EGFRvIII-synEphrinB2EC, its specific killing effect can be improved.

### Example 13. In vitro killing of Claudin18.2-CAR-T cells expressing Mesothelin-synEphrinB2EC

Refer to Examples 1 and 10, T cells with the chimeric polypeptide targeting Mesothelin (SEQ NO: 105) regulating the expression of Claudin18.2-CAR (SEQ NO: 67) or the co-expression of Claudin18.2-CAR with IL7 and CCL21 were prepared, wherein, the positive rates of Mesothelin synEphrinB2EC regulating the expression of Claudin18.2-CAR (SEQ NO: 68), Claudin18.2-CAR and IL7 (SEQ NO: 69), and Claudin18.2-CARwith IL7 and CCL21 (SEQ NO: 70) were 40.1%, 31.1%, and 33.8%, respectively. Co-culture was performed at different effect-to-target ratios, respectively, and LDH in the supernatant was detected. FIGs. 19 and 20 showed that both of the second and fourth generations of Claudin18.2-CAR-T cells expressing Mesothelin-synEphrinB2EC can be specifically activated by antigens to kill tumor cells.

### Example 14. In vivo killing of Claudin18.2-CAR-T cells expressing FAP-synEphrinB2EC

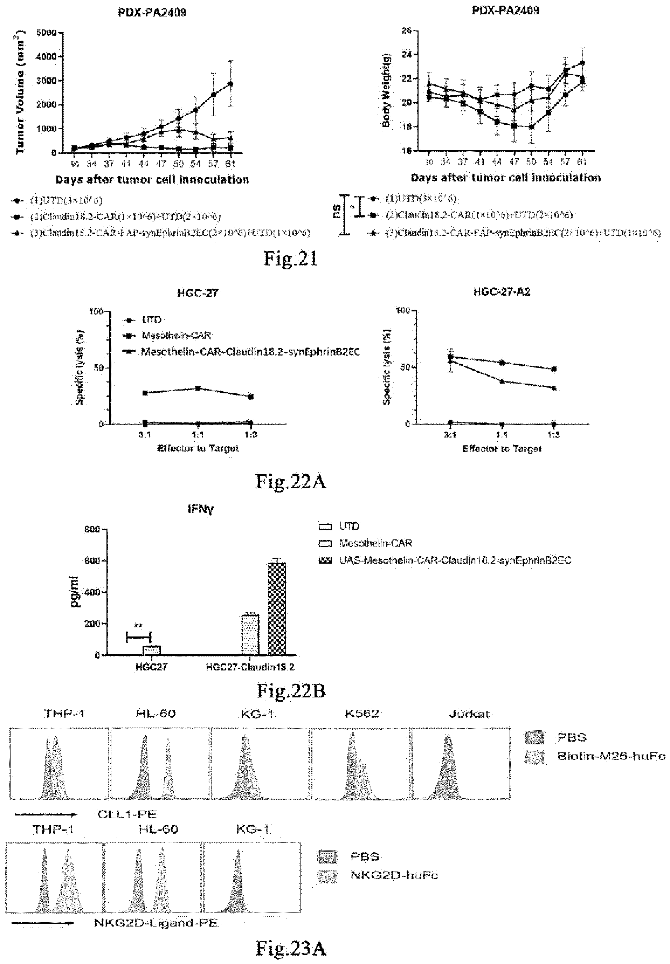

Referring to Examples 1 and 10, T cells containing FAP-synEphrinB2EC (SEQ NO: 106) regulating Claudin18.2-CAR (SEQ NO: 67) were constructed. Pancreatic cancer PDX model was divided into groups as illustrated after tumor formation, which were given the corresponding number of CAR-T cells. The tumor volume and body weight were measured. FIG. 21 showed that Claudin18.2-CAR-T cells expressing FAP-synEphrinB2EC were anti-tumor, and the body weight of mice did not decrease significantly.

### Example 15. In vitro killing of Mesothelin-CAR-T cells expressing Claudin18.2-synEphrinB2EC

Referring to Examples 1 and 10, T cells containing Claudin18.2-synEphrinB2EC (SEQ NO: 108) regulating Mesothelin-CAR (SEQ NO: 107) were constructed. Co-culture was performed at the effect-to-target ratio of 3:1, 1:1, and 1:3, respectively, and LDH in the supernatant was detected. FIG. 22A and FIG. 22B showed that Mesothelin-CAR-T cells containing Claudin18.2-synEphrinB2EC can be specifically activated by antigens to kill tumor cells.

### Example 16. CLL1-synEphrinB2EC regulating gene expression

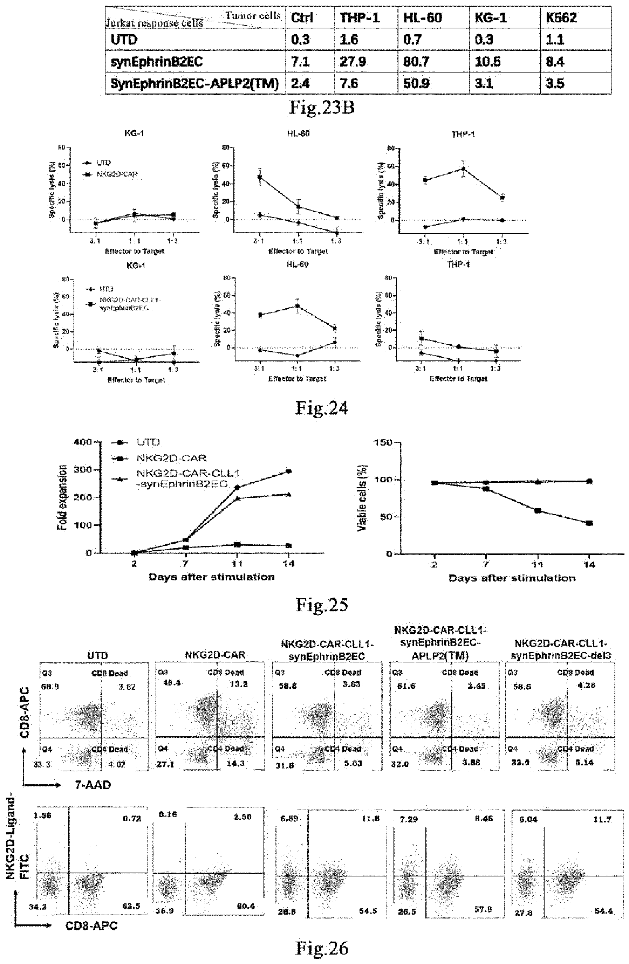

Referring to Examples 1 and 2, Jurkat response cells containing chimeric polypeptides targeting CLL1 (SEQ NO: 73, 110) were constructed, and respectively incubated with AML cell lines (FIG. 23A) at a ratio of 1:1 for 24 hours. BFP was detected by flow cytometry (FIG. 23B).

### Example 17. In vitro killing of NKG2D-CAR-T cells expressing CLL1-synEphrinB2EC

Referring to Example 10, T cells containing CLL1-synEphrinB2EC (SEQ NO: 73) regulating NKG2D-CAR (SEQ NO: 109) were constructed. Co-culture was performed at different effect-to-target ratios, and LDH in the supernatant was detected. The results showed that NKG2D-CAR-T cells killed THP1 and HL-60 cells expressing NKG2D ligands, and CD3Z-NKG2D-CAR-T cells induced by CLL1-synEphrinB2EC had a weak killing effect on THP-1 cells with low CLL1 expression, and can only kill HL-60 cells with high expression of both CLL1 and NKG2D ligands (FIGs. 23A and 24). This result is consistent with the experiment of BFP induced expression, indicating that CLL1-synEphrinB2EC further enhanced the specificity of NKG2D-CAR-T cells targeting AML cells.

### Example 18. Expansion and activity of NKG2D-CAR-T cells cultured in vitro

The CAR-T cells cultured in vitro were collected for cell counting and viability determination. FIG. 25 showed that T cells containing CLL1-synEphrinB2EC regulating NKG2D-CAR expanded well. The CAR-T cells cultured in vitro were collected, labeled with antibodies, stained with 7-AAD, and detected by flow cytometry. FIG. 26 showed that the death ratio of T cells containing CLL1-synEphrinB2EC regulating NKG2D-CAR is basically the same as that of UTD, while the death ratio of NKG2D-CAR-T cells was significantly increased; T cells containing CLL1-synEphrinB2EC regulating NKG2D-CAR can effectively guarantee expansion and activity of T cells in culture system.

### Example 19. Inducement of NKG2D-CAR expression by chimeric peptides targeting other AML therapeutic targets

Referring to Examples 16-18, taking CD123 as an example, CD123-synEphrinB2EC chimeric polypeptide (SEQ NO: 50) regulating NKG2D-CAR (SEQ NO: 109) T cells were constructed, which can enhance the specificity of NKG2D-CAR T cells targeting AML cells and effectively ensure the expansion and activity of T cells in the culture system.

### Example 20. Chimeric polypeptide inducing expression of therapeutic monoclonal antibody

The genes regulated by the chimeric polypeptides GPC3-synJagged2EC, GPC3-synEphrinB2EC, and GPC3-synEphrinB2EC-APLP2(TM) of the present application are replaced by the coding sequences of trastuzumab, bevacizumab, infliximab, and rituximab, adalimumab. When co-incubated with GPC3-positive tumor cells, synJagged2EC, synEphrinB2EC, synEphrinB2EC-APLP2(TM) induced comparable or significantly increased levels of trastuzumab, bevacizumab, infliximab, rituximab, and adalimumab expression, compared with synNotch.

Examples described herein include the example as any single example or in combination with any other example or portion thereof. In addition, it should be understood that after reading the above teaching content of the present application, those skilled in the art can make various changes or modifications to the present application, and these equivalent forms also fall within the scope defined by the appended claims of the present application.

### Sequence information

| No. | Name | Sequence |
|---|---|---|
| 1 | Amino acid of Jagged2 extracellular region | evigfgrscwsrgtpfphgsswvedcnscrcldgrrdcskvwcgwkpcllagqpealsaqcplgqrclekapggqclrppcea wgecgaeeppstpclprsghldnncarltlhfnrdhvpqgttvgaicsgirslpatravardrllvllcdrassgasavevavsfsp ardlpdsslliqggaahaivaaitqrgnsslllavtevkvetvvtggsstg |
| 2 | Nucleotide of Jagged2 extracellular region | gaagtgatcgggttcgggagatcctgctggtcccggggcactccgttcccacacggaagctcctgggtggaagactgcaacagc tgccgctgcctggatggccgccgtgactgcagcaaggtgtggtgcggatggaagccttgtctgctggccggccagcccgaggc cctgagcgcccagtgcccactggggcaaaggtgcctggagaaggccccaggccagtgtctgcgaccaccctgtgaggcctgg ggggagtgcggcgcagaagagccaccgagcaccccctgcctgccacgctccggccacctggacaataactgtgcccgcctca ccttgcatttcaaccgtgaccacgtgccccagggcaccacggtgggcgccatttgctccgggatccgctccctgccagccacaag ggctgtggcacgggaccgcctgctggtgttgctttgcgaccgggcgtcctcgggggccagtgctgtggaggtggccgtgtccttc agccctgccagggacctgcctgacagcagcctgatccagggcgcggcccacgccatcgtggccgccatcacccagcggggga acagctcactgctcctggctgtcaccgaggtcaaggtggagacggttgttacgggcggctcttccacaggt |
| 3 | Amino acid of EphrinB2 extracellular region (full length) | gqdassagstrnkdptrrpeleagtngrssttspfvkpnpgsstdgnsaghsgnnilgsevalfa |
| 4 | Nucleotide of EphrinB2 extracellular region (full length) | ggacaagatgcaagttctgctggatcaaccaggaataaagatccaacaagacgtccagaactagaagctggtacaaatggaaga agttcgacaacaagtccctttgtaaaaccaaatccaggttctagcacagacggcaacagcgccggacattcggggaacaacatcc tcggttccgaagtggccttatttgca |
| 5 | Amino acid of EphrinB2-del1 extracellular region | gqdatrrpeleagtngrssttspfvkpnpgsstdgnsaghsgnnilgsevalfa |
| 6 | Nucleotide of EphrinB2-del1 extracellular region | ggacaagatgcaacaagacgtccagaactagaagctggtacaaatggaagaagttcgacaacaagtccctttgtaaaaccaaatc caggttctagcacagacggcaacagcgccggacattcggggaacaacatcctcggttccgaagtggccttatttgca |
| 7 | Amino acid of EphrinB2-del2 extracellular region | gqdassagstrnkdptrrpspfvkpnpgsstdgnsaghsgnnilgsevalfa |
| 8 | Nucleotide of EphrinB2-del2 extracellular region | ggacaagatgcaagttctgctggatcaaccaggaataaagatccaacaagacgtccaagtccctttgtaaaaccaaatccaggttct agcacagacggcaacagcgccggacattcggggaacaacatcctcggttccgaagtggccttatttgca |
| 9 | Amino acid of EphrinB2-del3 extracellular region | gqdassagstrnkdptrrpeleagtngrssttspsevalfa |
| 10 | Nucleotide of EphrinB2-del3 extracellular region | ggacaagatgcaagttctgctggatcaaccaggaataaagatccaacaagacgtccagaactagaagctggtacaaatggaaga agttcgacaacaagtccctccgaagtggccttatttgca |
| 11 | Amino acid of EphrinB2-del23 extracellular region | gqdassagstrnkdptrrpsevalfa |
| 12 | Nucleotide of EphrinB2-del23 extracellular region | ggacaagatgcaagttctgctggatcaaccaggaataaagatccaacaagacgtccatccgaagtggccttatttgca |
| 13 | Mouse Notch1 transmembrane region | lmyvaaaafvllffvgcgvllsrkrrr |
| 14 | Mouse Notch2 transmembrane region | llyllavavviilffillgvimakrkrkh |
| 15 | Mouse Notch3 transmembrane region | llpllvagavflliifilgvmvarrkreh |
| 16 | Mouse Notch4 transmembrane region | pilcspvvgvlllalgallvlqlirrrrreh |
| 17 | Human Notch1 transmembrane region | fmyvaaaafvllffvgcgvllsrkrrr |
| 18 | Human Notch2 transmembrane region | llyllavavviilfiillgvimakrkrkh |
| 19 | Human Notch3 transmembrane region | lpllvagavlllvilvlgvmvarrkreh |
| 20 | Human Notch4 transmembrane region | pvlcspvagvillalgallvlqlirrrrreh |
| 21 | APLP2 transmembrane region | Saligllviavaiatvivislvmlrkrq |
| 22 | Notch1STS | rkrrr |
| 23 | Notch2STS | krkrkh |
| 24 | Notch3STS | rrkreh |
| 25 | Notch4STS | rrrrreh |
| 26 | STS ofAPLP2 | rkrq |
| 27 | GAL4-VP64 | mkllssieqacdicrlkklkcskekpkcakclknnwecryspktkrspltrahltevesrlerleqlfllifpredldmilkmdslq dikalltglfvqdnvnkdavtdrlasvetdmpltlrqhrisatssseessnkgqrqltvsaaaggsggsggsdalddfdldmlgs dalddfdldmlgsdalddfdldmlgsdalddfdldmlgs |
| 28 | Receptor regulatory domain of synJagged2EC | evigfgrscwsrgtpfphgsswvedcnscrcldgrrdcskvwcgwkpcllagqpealsaqcplgqrclekapgqclrppcea wgecgaeeppstpclprsghldnncarltlhfnrdhvpqgttvgaicsgirslpatravardrllvllcdrassgasavevavsfsp ardlpdssliqgaahaivaaitqrgnsslllavtevkvetvvtggsstglmyvaaaafvllffvgcgvllsrkrrr |
| 29 | Receptor regulatory domain of synEphrinB2EC | gqdassagstrnkdptrrpeleagtngrssttspfvkpnpgsstdgnsaghsgnnilgsevalfalmyvaaaafvllffvgcgvll srkrrr |
| 30 | Receptor regulatory domain of synEphrinB2EC-2 | gqdassagstrnkdptrrpeleagtngrssttspfvkpnpgsstdgnsaghsgnnilgsevalfalmyvaaaafvllffvgcgvll skrkrkh |
| 31 | Receptor regulatory domain of synEphrinB2EC-3 | gqdassagstrnkdptrrpeleagtngrssttspfvkpnpgsstdgnsaghsgnnilgsevalfalmyvaaaafvllffvgcgvll srrkreh |
| 32 | Receptor regulatory domain of synEphrinB2EC-4 | gqdassagstrnkdptrrpeleagtngrssttspfvkpnpgsstdgnsaghsgnnilgsevalfalmyvaaaafvllffvgcgvll srrrrreh |
| 33 | Receptor regulatory domain of synEphrinB2EC-5 | gqdassagstrnkdptrrpeleagtngrssttspfvkpnpgsstdgnsaghsgnnilgsevalfallyllavavviilfiillgvima krkrkh |
| 34 | Receptor regulatory domain of synEphrinB2EC-6 | gqdassagstrnkdptrrpeleagtngrssttspfvkpnpgsstdgnsaghsgnnilgsevalfalpllvagavlllvilvlgvmv arrkreh |
| 35 | Receptor regulatory domain of synEphrinB2EC-7 | gqdassagstrnkdptrrpeleagtngrssttspfvkpnpgsstdgnsaghsgnnilgsevalfapvlcspvagvillalgallvlq lirrrrreh |
| 36 | Receptor regulatory domain of synEphrinB2EC-APLP 2TM | gqdassagstrnkdptrrpeleagtngrssttspfvkpnpgsstdgnsaghsgnnilgsevalfasaligllviavaiatvivislv mlrkrq |
| 37 | Receptor regulatory domain of synEphrinB2EC-del1 | gqdatrrpeleagtngrssttspfvkpnpgsstdgnsaghsgnnilgsevalfalmyvaaaafvllffvgcgvllsrkrrr |
| 38 | Receptor regulatory domain of synEphrinB2EC-del2 | gqdassagstrnkdptrrpspfvkpnpgsstdgnsaghsgnnilgsevalfalmyvaaaafvllffvgcgvllsrkrrr |
| 39 | Receptor regulatory domain of synEphrinB2EC-del3 | gqdassagstrnkdptrrpeleagtngrssttspsevalfalmyvaaaafvllffvgcgvllsrkrrr |
| 40 | Receptor regulatory domain of synEphrinB2EC-del3-2 | gqdassagstrnkdptrrpeleagtngrssttspsevalfalmyvaaaafvllffvgcgvllskrkrkh |
| 41 | Receptor regulatory domain of synEphrinB2EC-del23 | gqdassagstrnkdptrrpsevalfalmyvaaaafvllffvgcgvllsrkrrr |
| 42 | antiGPC3-scFv | evqlvqsgaevkkpgasvkvsckasgytfsdyemhwvrqapgqglewmgaihpgsgdtaynqrfkgrvtitadkststay melsslrsedtavyycarfysyaywgqgtlvtvsaggggsggggsggggsdivmtqtplslpvtpgepasiscrssqslvhsn gntylqwylqkpgqspqlliykvsnrfsgvpdrfsgsgsgtdftlkisrveaedvgvyycsqsiyvpytfgqgtkleikr |
| 43 | antiEGFR vIII -scFv | diqmtqspsslsasvgdrvtitchasqdinvnigwlqqkpgkafkgliyhgknledgvpsrfsgsgsgtdftltisslqpedfaty ycnqyenipltfgqgtkveikrggggsggggsggggsdvqlvesggglvqpggslrlscavsgysitsdyawnwirqapgk glewlgyisyrgrtqynpslksrisitrdnskntfflqlnslraedtavyycarmgknwdywgqgtlvtvss |
| 44 | antiMesothelin-scFv | qmqlvqsgaevkkpgasvkvsckasgytftsyymhwvrqapgqglewmgiinpsggstsyaqkfqgrvtmtrdtststvy melsslrsedtavyycassrsgttvvnhdafdiwgkgttvtvssggggsggggsggggsdiqltqspsslsasvgdrvtitcras qvisralawyqqtpgkppklliydasnlqsgvpsrfsgsgsgtdftltisrlqpedfatyycqqfnsypltfgggtkleikr |
| 45 | antiFAP-scFv | qvqlvqsgaevkkpgssvkvsckasggtfssyaiswvrqapgqglewmggiipifgtanyaqkfqgrvtitadkststayme lsslrsedtavyycardaadrdywgqgttvtvssggggsggggsggggsdivmtqsplslpvtpgepasiscrsssqslllhpngf nhlywylqkpgqspqlliyvggnrasgvpdrfsgsgsgtdftlkisrveaedvgvyycqqrnnknrtfgqgtkveikr |
| 46 | anti Claudin18.2 scFv | qvqlqesgpglikpsqtlsltctvsggsissgynwhwirqppgkglewigyihytgstnynpalrsrvtisvdtsknqfslklssv taadtaiyycariyngnsfpywgqgttvtvssggggsggggsggggsdivmtqspdslavslgeratinckssqslfnsgnqk nyltwyqqkpgqppklliywastresgvpdrfsgsgsgtdftltisslqaedvavyycqnaysfpytfgggtkleikr |
| 47 | antiCLL1-scFv | evqlvqsgaevkkpgasvkvsckasgytfnsyymhwvrqapgqglewmgifnpgggstsyaqkfqgrvtmtrdtststvy melsslrsedtavyycargttsdafdiwgqgtmvtvssggggsggggsggggsdiqmtqspsslsasvgdrvtitcrasqsiss ylnwyqqkpgkapklliydvasslqsgvpsrfsgsgsgtdftltisslqpedfatyycqqsystpltfgggtkveikr |
| 48 | UAS | aagcttgatatcgaattcggagcactgtcctccgaacgtcggagcactgtcctccgaacgtcggagcactgtcctccgaacgtcgg agcactgtcctccgaacggagcatgtcctccgaacgtcggagcactgtcctccgaacg |
| 49 | UAS-CMV promoter | aagcttgatatcgaattcggagcactgtcctccgaacgtcggagcactgtcctccgaacgtcggagcactgtcctccgaacgtcgg agcactgtcctccgaacggagcatgtcctccgaacgtcggagcactgtcctccgaacgactagttaggcgtgtacggtgggaggc ctatataagcagagctcgtttagtgaaccgtcagatcgcctggagacgccatccacgctgttttgacctccatagaagacaccggg accgatccagcctctcgacattcgttggatc |
| 50 | Amino acid sequence of CD123-synEphrinB2E C | evqllesggglvqpggslrlscaasgftfssyamswvrqapgkglewvsaisgsggstyyadsvkgrftisrdnskntlylqm nslraedtavyycakddsfyggfdywgqgtlvtvssggggsggggsggggsdiqmtqspstlsasvgdrvtitcrasqsissw lawyqqkpgkapklliydasslesgvpsrfsgsgsgteftltisslqpddfatyycqqyssykytfgqgtkveikrgqdassagst rnkdptrrpeleagtngrssttspfvkpnpgsstdgnsaghsgnnilgsevalfalmyvaaaafvllffvgcgvllsrkrrrmklls sieqacdicrlkklkcskekpkcakclknnwecryspktkrspltrahltevesrlerleqlfllifpredldmilkmdslqdikall tglfvqdnvnkdavtdrlasvetdmpltlrqhrisatssseessnkgqrqltvsaaaggsggsggsdalddfdldmlgsdalddf dldmlgsdalddfdldmlgsdalddfdldmlgs |
| 51 | Amino acid sequence of GPC3-synJagged2EC | eqkliseedlevqlvqsgaevkkpgasvkvsckasgytfsdyemhwvrqapgqglewmgaihpgsgdtaynqrfkgrvti tadkststaymelsslrsedtavyycarfysyaywgqgtlvtvsaggggsggggsggggsdivmtqtplslpvtpgepasiscr ssqslvhsngntylqwylqkpgqspqlliykvsnrfsgvpdrfsgsgsgtdftlkisrveaedvgvyycsqsiyvpytfgqgtkl |
| | | eikrevigfgrscwsrgtpfphgsswvedcnscrcldgrrdcskvwcgwkpcllagqpealsaqcplgqrclekapgqclrp pceawgecgaeeppstpclprsghldnncarltlhfnrdhvpqgttvgaicsgirslpatravardrllvllcdrassgasaveva vsfspardlpdssliqgaahaivaaitqrgnsslllavtevkvetvvtggsstglmyvaaaafvllffvgcgvllsrkrrrmkllssi eqacdicrlkklkcskekpkcakclknnwecryspktkrspltrahltevesrlerleqlfllifpredldmilkmdslqdikallt glfvqdnvnkdavtdrlasvetdmpltlrqhrisatssseessnkgqrqltvsaaaggsggsggsdalddfdldmlgsdalddf dldmlgsdalddfdldmlgsdalddfdldmlgs |
| 52 | Amino acid sequence of GPC3-EphrinB2EC | eqkliseedlevqlvqsgaevkkpgasvkvsckasgytfsdyemhwvrqapgqglewmgaihpgsgdtaynqrfkgrvti tadkststaymelsslrsedtavyycarfysyaywgqgtlvtvsaggggsggggsggggsdivmtqtplslpvtpgepasiscr ssqslvhsngntylqwylqkpgqspqlliykvsnrfsgvpdrfsgsgsgtdftlkisrveaedvgvyycsqsiyvpytfgqgtkl eikrgqdassagstrnkdptrrpeleagtngrssttspfvkpnpgsstdgnsaghsgnnilgsevalfalmyvaaaafvllffvgc gvllsrkrrrmkllssieqacdicrlkklkcskekpkcakclknnwecryspktkrspltrahltevesrlerleqlfllifpredld milkmdslqdikalltglfvqdnvnkdavtdrlasvetdmpltlrqhrisatssseessnkgqrqltvsaaaggsggsggsdald dfdldmlgsdalddfdldmlgsdalddfdldmlgsdalddfdldmlgs |
| 53 | Amino acid sequence of GPC3-synEphrinB2EC-APLP 2 (TM) | eqkliseedlevqlvqsgaevkkpgasvkvsckasgytfsdyemhwvrqapgqglewmgaihpgsgdtaynqrfkgrvti tadkststaymelsslrsedtavyycarfysyaywgqgtlvtvsaggggsggggsggggsdivmtqtplslpvtpgepasiscr ssqslvhsngntylqwylqkpgqspqlliykvsnrfsgvpdrfsgsgsgtdftlkisrveaedvgvyycsqsiyvpytfgqgtkl eikrgqdassagstrnkdptrrpeleagtngrssttspfvkpnpgsstdgnsaghsgnnilgsevalfasaligllviavaiatvivi slvmlrkrqmkllssieqacdicrlkklkcskekpkcakclknnwecryspktkrspltrahltevesrlerleqlfllifpredld milkmdslqdikalltglfvqdnvnkdavtdrlasvetdmpltlrqhrisatssseessnkgqrqltvsaaaggsggsggsdald dfdldmlgsdalddfdldmlgsdalddfdldmlgsdalddfdldmlgs |
| 54 | Amino acid sequence of GPC3-synEphrinB2EC -dell | eqkliseedlevqlvqsgaevkkpgasvkvsckasgytfsdyemhwvrqapgqglewmgaihpgsgdtaynqrfkgrvti tadkststaymelsslrsedtavyycarfysyaywgqgtlvtvsaggggsggggsggggsdivmtqtplslpvtpgepasiscr ssqslvhsngntylqwylqkpgqspqlliykvsnrfsgvpdrfsgsgsgtdftlkisrveaedvgvyycsqsiyvpytfgqgtkl eikrgqdatrrpeleagtngrssttspfvkpnpgsstdgnsaghsgnnilgsevalfalmyvaaaafvllffvgcgvllsrkrrrm kllssieqacdicrlkklkcskekpkcakclknnwecryspktkrspltrahltevesrlerleqlfllifpredldmilkmdslqdi kalltglfvqdnvnkdavtdrlasvetdmpltlrqhrisatssseessnkgqrqltvsaaaggsggsggsdalddfdldmlgsdal ddfdldmlgsdalddfdldmlgsdalddfdldmlgs |
| 55 | Amino acid sequence of GPC3-synEphrinB2EC -del2 | eqkliseedlevqlvqsgaevkkpgasvkvsckasgytfsdyemhwvrqapgqglewmgaihpgsgdtaynqrfkgrvti tadkststaymelsslrsedtavyycarfysyaywgqgtlvtvsaggggsggggsggggsdivmtqtplslpvtpgepasiscr ssqslvhsngntylqwylqkpgqspqlliykvsnrfsgvpdrfsgsgsgtdftlkisrveaedvgvyycsqsiyvpytfgqgtkl eikrgqdassagstrnkdptrrpspfvkpnpgsstdgnsaghsgnnilgsevalfalmyvaaaafvllffvgcgvllsrkrrrmk llssieqacdicrlkklkcskekpkcakclknnwecryspktkrspltrahltevesrlerleqlfllifpredldmilkmdslqdik alltglfvqdnvnkdavtdrlasvetdmpltlrqhrisatssseessnkgqrqltvsaaaggsggsggsdalddfdldmlgsdald dfdldmlgsdalddfdldmlgsdalddfdldmlgs |
| 56 | Amino acid sequence of GPC3-synEphrinB2EC -del3 | eqkliseedlevqlvqsgaevkkpgasvkvsckasgytfsdyemhwvrqapgqglewmgaihpgsgdtaynqrfkgrvti tadkststaymelsslrsedtavyycarfysyaywgqgtlvtvsaggggsggggsggggsdivmtqtplslpvtpgepasiscr ssqslvhsngntylqwylqkpgqspqlliykvsnrfsgvpdrfsgsgsgtdftlkisrveaedvgvyycsqsiyvpytfgqgtkl eikrgqdassagstrnkdptrrpeleagtngrssttspsevalfalmyvaaaafvllffvgcgvllsrkrrrmkllssieqacdicrlk klkcskekpkcakclknnwecryspktkrspltrahltevesrlerleqlfllifpredldmilkmdslqdikalltglfvqdnvnk davtdrlasvetdmpltlrqhrisatssseessnkgqrqltvsaaaggsggsggsdalddfdldmlgsdalddfdldmlgsdald dfdldmlgsdalddfdldmlgs |
| 57 | Amino acid sequence of GPC3-synEphrinB2EC | eqkliseedlevqlvqsgaevkkpgasvkvsckasgytfsdyemhwvrqapgqglewmgaihpgsgdtaynqrfkgrvti tadkststaymelsslrsedtavyycarfysyaywgqgtlvtvsaggggsggggsggggsdivmtqtplslpvtpgepasiscr ssqslvhsngntylqwylqkpgqspqlliykvsnrfsgvpdrfsgsgsgtdftlkisrveaedvgvyycsqsiyvpytfgqgtkl |
| | -del23 | eikrgqdassagstrnkdptrrpsevalfalmyvaaaafvllffvgcgvllsrkrrrmkllssieqacdicrlkklkcskekpkcak clknnwecryspktkrspltrahltevesrlerleqlfllifpredldmilkmdslqdikalltglfvqdnvnkdavtdrlasvetd mpltlrqhrisatssseessnkgqrqltvsaaaggsggsggsdalddfdldmlgsdalddfdldmlgsdalddfdldmlgsdal ddfdldmlgs |
| 58 | IL12 ( including two subunits P35and P40) | mchqqlviswfslvflasplvaiwelkkdvyvveldwypdapgemvvltcdtpeedgitwtldqssevlgsgktltiqvkef gdagqytchkggevlshsllllhkkedgiwstdilkdqkepknktflrceaknysgrftcwwlttistdltfsvkssrgssdpqgv tcgaatlsaervrgdnkeyeysvecqedsacpaaeeslpievmvdavhklkyenytssffirdiikpdppknlqlkplknsrqv evsweypdtwstphsyfsltfcvqvqgkskrekkdrvftdktsatvicrknasisvraqdryysssswsewasvpcsggggsg gggsggggsrnlpvatpdpgmfpclhhsqnllravsnmlqkarqtlefypctseeidheditkdktstveaclpleltknescln sretsfitngsclasrktsfmmalclssiyedlkmyqvefktmnakllmdpkrqifldqnmlavidelmqalnfnsetvpqks sleepdfyktkiklcillhafriravtidrvmsylnas |
| 59 | Nucleic acid of IL12-GPC3-synEphrin B2EC-del3 | aagcttgatatcgaattcggagcactgtcctccgaacgtcggagcactgtcctccgaacgtcggagcactgtcctccgaacgtcgg agcactgtcctccgaacggagcatgtcctccgaacgtcggagcactgtcctccgaacgactagttaggcgtgtacggtgggaggc ctatataagcagagctcgtttagtgaaccgtcagatcgcctggagacgccatccacgctgttttgacctccatagaagacaccggg accgatccagcctctcgacattcgttggatcatgtgtcaccagcagttggtcatctcttggttttccctggtttttctggcatctcccctcg tggccatatgggaactgaagaaagatgtttatgtcgtagaattggattggtatccggatgcccctggagaaatggtggtcctcacctg tgacacccctgaagaagatggtatcacctggaccttggaccagagcagtgaggtcttaggctctggcaaaaccctgaccatccaa gtcaaagagtttggagatgctggccagtacacctgtcacaaaggaggcgaggttctaagccattcgctcctgctgcttcacaaaaa ggaagatggaatttggtccactgatattttaaaggaccagaaagaacccaaaaataagacctttctaagatgcgaggccaagaatta ttctggacgtttcacctgctggtggctgacgacaatcagtactgatttgacattcagtgtcaaaagcagcagaggctcttctgacccc caaggggtgacgtgcggagctgctacactctctgcagagagagtcagaggggacaacaaggagtatgagtactcagtggagtg ccaggaggacagtgcctgcccagctgctgaggagagtctgcccattgaggtcatggtggatgccgttcacaagctcaagtatgaa aactacaccagcagcttcttcatcagggacatcatcaaacctgacccacccaagaacttgcagctgaagccattaaagaattctcgg caggtggaggtcagctgggagtaccctgacacctggagtactccacattcctacttctccctgacattctgcgttcaggtccagggc aagagcaagagagaaaagaaagatagagtcttcacggacaagacctcagccacggtcatctgccgcaaaaatgccagcattagc gtgcgggcccaggaccgctactatagctcatcttggagcgaatgggcatctgtgccctgcagtggtggcggtggctcgggcggt ggtgggtcgggtggcggcggatctagaaacctccccgtggccactccagacccaggaatgttcccatgccttcaccactcccaaa acctgctgagggccgtcagcaacatgctccagaaggccagacaaactctagaattttacccttgcacttctgaagagattgatcatg aagatatcacaaaagataaaaccagcacagtggaggcctgtttaccattggaattaaccaagaatgagagttgcctaaattccaga gagacctctttcataactaatgggagttgcctggcctccagaaagacctcttttatgatggccctgtgccttagtagtatttatgaagac ttgaagatgtaccaggtggagttcaagaccatgaatgcaaagcttctgatggatcctaagaggcagatctttctagatcaaaacatgc tggcagttattgatgagctgatgcaggccctgaatttcaacagtgagactgtgccacaaaaatcctcccttgaagaaccggatttttat aaaactaaaatcaagctctgcatacttcttcatgctttcagaattcgggcagtgactattgatagagtgatgagctatctgaatgcttcct aaacgcgtggctccggtgcccgtcagtgggcagagcgcacatcgcccacagtccccgagaagttggggggaggggtcggcaa ttgaaccggtgcctagagaaggtggcgcggggtaaactgggaaagtgatgtcgtgtactggctccgcctttttcccgagggtggg ggagaaccgtatataagtgcagtagtcgccgtgaacgttctttttcgcaacgggtttgccgccagaacacaggtgtcgtgacgcgg atccaggcctaagcttacgcgtgccaccatggcgctccctgtcaccgcactgcttcttccgctggcactgctgctgcacgctgcac ggcctgagcaaaaacttatctctgaagaggacctcgaggtgcagctggtgcagagcggcgccgaggtgaagaagcccggcgc cagcgtgaaggtgagctgcaaggccagcggctacaccttcagcgactacgagatgcactgggtgcggcaggcccccggccag ggcctggagtggatgggcgccatccaccccggcagcggcgacaccgcctacaaccagcggttcaagggccgggtgaccatca ccgccgacaagagcaccagcaccgcctacatggagctgagcagcctgcggagcgaggacaccgccgtgtactactgcgcccg gttctacagctacgcctactggggccagggcaccctggtgaccgtgagcgccggtggaggcggttcaggcggaggtggttctgg cggtggcggatcggacatcgtgatgacccagacccccctgagcctgcccgtgacccccggcgagcccgccagcatcagctgcc ggagcagccagagcctggtgcacagcaacggcaacacctacctgcagtggtacctgcagaagcccggccagagcccccagct |
| | | gctgatctacaaggtgagcaaccggttcagcggcgtgcccgaccggttcagcggcagcggcagcggcaccgacttcaccctga agatcagccgggtggaggccgaggacgtgggcgtgtactactgcagccagagcatctacgtgccctacaccttcggccagggc accaagctggagatcaaacgtggacaagatgcaagttctgctggatcaaccaggaataaagatccaacaagacgtccagaacta gaagctggtacaaatggaagaagttcgacaacaagtccctttgtaaaaccaaatccaggttctagcacagacggcaacagcgccg gacattcggggaacaacatcctcggttccgaagtggccttatttgcactcatgtacgtggcagcggccgccttcgtgctcctgttcttt gtgggctgtggggtgctgctgtcccgcaagcgccggcggatgaagctgctgagcagcatcgagcaggcctgtgacatctgccg gctgaagaaactgaagtgcagcaaagaaaagcccaagtgcgccaagtgcctgaagaacaactgggagtgccggtacagcccc aagaccaagagaagccccctgaccagagcccacctgaccgaggtggaaagccggctggaaagactggaacagctgtttctgct gatcttcccacgcgaggacctggacatgatcctgaagatggacagcctgcaggacatcaaggccctgctgaccggcctgttcgtg caggacaacgtgaacaaggacgccgtgaccgacagactggccagcgtggaaaccgacatgcccctgaccctgcggcagcaca gaatcagcgccaccagcagcagcgaggaaagcagcaacaagggccagcggcagctgacagtgtctgctgctgcaggcggaa gcggaggctctggcggatctgatgccctggacgacttcgacctggatatgctgggcagcgacgccctggatgattttgatctggac atgctgggatctgacgctctggacgatttcgatctcgacatgttgggatcagatgcactggatgactttgacctggacatgctcggat ca |
| 60 | GPC3-CAR | evqlvqsgaevkkpgasvkvsckasgytfsdyemhwvrqapgqglewmgaihpgsgdtaynqrfkgrvtitadkststay melsslrsedtavyycarfysyaywgqgtlvtvsaggggsggggsggggsdivmtqtplslpvtpgepasiscrssqslvhsn gntylqwylqkpgqspqlliykvsnrfsgvpdrfsgsgsgtdftlkisrveaedvgvyycsqsiyvpytfgqgtkleikrtttpap rpptpaptiasqplslrpeacrpaaggavhtrgldfacdiyiwaplagtcgvlllslvitlyckrgrkkllyifkqpfmrpvqttqee dgcscrfpeeeeggcelrvkfsrsadapayqqgqnqlynelnlgrreeydvldkrrgrdpemggkpqrrknpqeglynelqk dkmaeayseigmkgerrrgkghdglyqglstatkdtydalhmqalppr |
| 61 | Amino acid sequence of EGFRvIII-EphrinB2EC | diqmtqspsslsasvgdrvtitchasqdinvnigwlqqkpgkafkgliyhgknledgvpsrfsgsgsgtdftltisslqpedfaty ycnqyenipltfgqgtkveikrggggsggggsggggsdvqlvesggglvqpggslrlscavsgysitsdyawnwirqapgk glewlgyisyrgrtqynpslksrisitrdnskntfflqlnslraedtavyycarmgknwdywgqgtlvtvssgqdassagstrnk dptrrpeleagtngrssttspfvkpnpgsstdgnsaghsgnnilgsevalfalmyvaaaafvllffvgcgvllsrkrrrmkllssie qacdicrlkklkcskekpkcakclknnwecryspktkrspltrahltevesrlerleqlfllifpredldmilkmdslqdikalltgl fvqdnvnkdavtdrlasvetdmpltlrqhrisatssseessnkgqrqltvsaaaggsggsggsdalddfdldmlgsdalddfdl dmlgsdalddfdldmlgsdalddfdldmlgs |
| 62 | Amino acid of IL13Ra2-CAR | evqllesggglvqpggslrlscaasgftfrkyamgwvrqapgkglewvsgisgsvgstyyadsvkgrftisrdnskntlylqm nslraedtavyycakvrygwgagafdywgqgtlvtvssggggsggggsggggsdiqmtqspstlsasvgdrvtitcrasqsis swlawyqqkpgkapklliydasslesgvpsrfsgsgsgteftltisslqpddfatyycqqydtyppitfgqgtkveikrtttpapr pptpaptiasqplslrpeacrpaaggavhtrgldfacdfwvlvvvggvlacysllvtvafiifwvrskrsrllhsdymnmtprrp gptrkhyqpyapprdfaayrsrvkfsrsadapayqqgqnqlynelnlgrreeydvldkrrgrdpemggkpqrrknpqegly nelqkdkmaeayseigmkgerrrgkghdglyqglstatkdtydalhmqalppr |
| 63 | Nucleic acid of EGFRvIII-EphrinB2EC -IL13Ra2-CAR | aagcttgatatcgaattcggagcactgtcctccgaacgtcggagcactgtcctccgaacgtcggagcactgtcctccgaacgtcgg agcactgtcctccgaacggagcatgtcctccgaacgtcggagcactgtcctccgaacgactagttaggcgtgtacggtgggaggc ctatataagcagagctcgtttagtgaaccgtcagatcgcctggagacgccatccacgctgttttgacctccatagaagacaccggg accgatccagcctctcgacattcgttggatcatggccttaccagtgaccgccttgctcctgccgctggccttgctgctccacgccgc caggccggaggtgcaattgctggagtctgggggaggcttggtacagcctggggggtccctgagactctcctgtgcagcctccgg attcacctttcgcaagtatgccatgggctgggtccgccaggctccagggaaggggctggagtgggtctcaggtattagtggtagtg ttggtagcacatactacgcagactccgtgaagggccggttcaccatctccagagacaattccaagaacacgctgtatctgcagatg aacagcctgagagccgaggacacggccgtatattactgtgcgaaagttcgttacggttggggtgcaggtgcattcgactactggg gccaaggaaccctggtcaccgtctcgagtggtggaggcggttcaggcggaggtggttctggcggtggcggatcggacatccag atgacccagtctccttccaccctgtctgcatctgtaggagaccgtgtcaccatcacttgccgtgccagtcagagtattagtagctggtt ggcctggtatcagcagaaaccagggaaagcccctaagctcctgatctatgatgcctccagtttggaaagtggggtcccatcacgttt |
| | | cagcggcagtggatccgggacagaattcactctcaccatcagcagcttgcagcctgatgattttgcaacttattactgccaacagta cgatacctacccaccaatcacgtttggccagggcaccaaagtcgagatcaagcgtaccacgacgccagcgccgcgaccaccaa caccggcgcccaccatcgcgtcgcagcccctgtccctgcgcccagaggcgtgccggccagcggcggggggcgcagtgcaca cgagggggctggacttcgcctgtgatttttgggtgctggtggtggttggtggagtcctggcttgctatagcttgctagtaacagtggc ctttattattttctgggtgaggagtaagaggagcaggctcctgcacagtgactacatgaacatgactccccgccgccccgggccaa cccgcaagcattaccagccctatgccccaccacgcgacttcgcagcctatcgctccagagtgaagttcagcaggagcgcagacg cccccgcgtaccagcagggccagaaccagctctataacgagctcaatctaggacgaagagaggagtacgatgttttggacaaga gacgtggccgggaccctgagatggggggaaagccgcagagaaggaagaaccctcaggaaggcctgtacaatgaactgcaga aagataagatggcggaggcctacagtgagattgggatgaaaggcgagcgccggaggggcaaggggcacgatggcctttacca gggtctcagtacagccaccaaggacacctacgacgcccttcacatgcaggccctgccccctcgctagacgcgtggctccggtgc ccgtcagtgggcagagcgcacatcgcccacagtccccgagaagttggggggaggggtcggcaattgaaccggtgcctagaga aggtggcgcggggtaaactgggaaagtgatgtcgtgtactggctccgcctttttcccgagggtgggggagaaccgtatataagtg cagtagtcgccgtgaacgttctttttcgcaacgggtttgccgccagaacacaggtgtcgtgacgcggatccaggcctaagcttacg cgtgccaccatggccttaccagtgaccgccttgctcctgccgctggccttgctgctccacgccgccaggccggatattcagatgac ccagagcccgagcagcctgagcgcgagcgtgggcgaccgtgtgaccattacctgccatgcgagccaggatattaacgtgaacat tggctggctgcagcagaaaccgggcaaagcgtttaaaggcctgatttatcatggcaaaaacctggaagatggcgtgccgagccgt tttagcggcagcggcagcggcaccgattttaccctgaccattagcagcctgcagccggaagattttgcgacctattattgcaatcag tatgaaaatatcccactgacatttggccagggcaccaaagtggaaattaaacgtggtggaggcggttcaggcggaggtggctctg gcggtggcggatcggatgtgcagctggtggaaagcggcggcggcctggtgcagccgggcggcagcctgcgtctgagctgcgc ggtgagcggctatagcattaccagcgattatgcgtggaactggattcgtcaggcgccgggcaaaggcctggaatggctgggctat attagctatcgtggccgcacccagtataacccgagcctgaaaagccgtattagcattacccgtgataacagcaaaaacacctttttcc tgcagctgaacagcctgcgtgcggaagataccgcggtgtattattgcgcgcgcatgggtaagaattgggattactggggccaggg caccctggtgaccgtgagcagcggacaagatgcaagttctgctggatcaaccaggaataaagatccaacaagacgtccagaact agaagctggtacaaatggaagaagttcgacaacaagtccctttgtaaaaccaaatccaggttctagcacagacggcaacagcgcc ggacattcggggaacaacatcctcggttccgaagtggccttatttgcactcatgtacgtggcagcggccgccttcgtgctcctgttct ttgtgggctgtggggtgctgctgtcccgcaagcgccggcggatgaagctgctgagcagcatcgagcaggcctgtgacatctgcc ggctgaagaaactgaagtgcagcaaagaaaagcccaagtgcgccaagtgcctgaagaacaactgggagtgccggtacagccc caagaccaagagaagccccctgaccagagcccacctgaccgaggtggaaagccggctggaaagactggaacagctgtttctgc tgatcttcccacgcgaggacctggacatgatcctgaagatggacagcctgcaggacatcaaggccctgctgaccggcctgttcgt gcaggacaacgtgaacaaggacgccgtgaccgacagactggccagcgtggaaaccgacatgcccctgaccctgcggcagca cagaatcagcgccaccagcagcagcgaggaaagcagcaacaagggccagcggcagctgacagtgtctgctgctgcaggcgg aagcggaggctctggcggatctgatgccctggacgacttcgacctggatatgctgggcagcgacgccctggatgattttgatctgg acatgctgggatctgacgctctggacgatttcgatctcgacatgttgggatcagatgcactggatgactttgacctggacatgctcgg atca |
| 64 | Amino acid of B7H3-28Z | evqllesggglvqpggslrlscaasgftfssyamswvrqapgkglewvsaisgsggstyyadsvkgrftisrdnskntlylqm nslraedtavyycakyravtfdywgqgtlvtvssggggsggggsggggsdiqmtqspstlsasvgdrvtitcrasqsisswla wyqqkpgkapklliydasslesgvpsrfsgsgsgteftltisslqpddfatyycqqfetqirwtfgqgtkveikrtttpaprpptpa ptiasqplslrpeacrpaaggavhtrgldfacdfwvlvvvggvlacysllvtvafiifwvrskrsrllhsdymnmtprrpgptrk hyqpyapprdfaayrsrvkfsrsadapayqqgqnqlynelnlgrreeydvldkrrgrdpemggkpqrrknpqeglynelqk dkmaeayseigmkgerrrgkghdglyqglstatkdtydalhmqalppr |
| 65 | Amino acid of B7H3-BBZ | evqllesggglvqpggslrlscaasgftfssyamswvrqapgkglewvsaisgsggstyyadsvkgrftisrdnskntlylqm nslraedtavyycakyravtfdywgqgtlvtvssggggsggggsggggsdiqmtqspstlsasvgdrvtitcrasqsisswla wyqqkpgkapklliydasslesgvpsrfsgsgsgteftltisslqpddfatyycqqfetqirwtfgqgtkveikrtttpaprpptpa ptiasqplslrpeacrpaaggavhtrgldfacdiyiwaplagtcgvlllslvitlyckrgrkkllyifkqpfmrpvqttqeedgcscr |
| | | fpeeeeggcelrvkfsrsadapayqqgqnqlynelnlgrreeydvldkrrgrdpemggkpqrrknpqeglynelqkdkmae ayseigmkgerrrgkghdglyqglstatkdtydalhmqalppr |
| 66 | Amino acid of 376.96-28Z | divmtqshkfmstsigarvsitckasqdvrtavawyqqkpgqspklliysasyrytgvpdrftgsgsgtdftftissvqaedlav yycqqhygtppwtfgggtkleikggggsggggsggggsevqlvesggglvkpggslklsceasrftfssyamswvrqtpek rlewvaaisgggrytyypdsmkgrftisrdnaknflylqmsslrsedtamyycarhydgyldywgqgttltvssttpaprppt paptiasqplslrpeacrpaaggavhtrgldfacdfwvlvvvggvlacysllvtvafiifwvrskrsrllhsdymnmtprrpgpt rkhyqpyapprdfaayrsrvkfsrsadapayqqgqnqlynelnlgrreeydvldkrrgrdpemggkpqrrknpqeglynelq kdkmaeayseigmkgerrrgkghdglyqglstatkdtydalhmqalppr |
| 67 | Claudin18.2-CAR | qvqlqesgpglikpsqtlsltctvsggsissgynwhwirqppgkglewigyihytgstnynpalrsrvtisvdtsknqfslklssv taadtaiyycariyngnsfpywgqgttvtvssggggsggggsggggsdivmtqspdslavslgeratinckssqslfnsgnqk nyltwyqqkpgqppklliywastresgvpdrfsgsgsgtdftltisslqaedvavyycqnaysfpytfgggtkleikrtttpaprp ptpaptiasqplslrpeacrpaaggavhtrgldfacdiyiwaplagtcgvlllslvitlyckrgrkkllyifkqpfmrpvqttqeed gcscrfpeeeeggcelrvkfsrsadapayqqgqnqlynelnlgrreeydvldkrrgrdpemggkpqrrknpqeglynelqkd kmaeayseigmkgerrrgkghdglyqglstatkdtydalhmqalppr |
| 68 | Mesothelin-synEphrinB 2EC- Claudin18.2-CAR | aagcttgatatcgaattcggagcactgtcctccgaacgtcggagcactgtcctccgaacgtcggagcactgtcctccgaacgtcgg agcactgtcctccgaacggagcatgtcctccgaacgtcggagcactgtcctccgaacgactagttaggcgtgtacggtgggaggc ctatataagcagagctcgtttagtgaaccgtcagatcgcctggagacgccatccacgctgttttgacctccatagaagacaccggg accgatccagcctctcgacattcgttggatcatggccttaccagtgaccgccttgctcctgccgctggccttgctgctccacgccgc caggccgcaggtgcagctgcaggagagcggccccggcctgatcaagcccagccagaccctgagcctgacctgcaccgtgagc ggcggcagcatcagcagcggctacaactggcactggatccggcagccccccggcaagggcctggagtggatcggctacatcc actacaccggcagcaccaactacaaccccgccctgcggagccgggtgaccatcagcgtggacaccagcaagaaccagttcag cctgaagctgagcagcgtgaccgccgccgacaccgccatctactactgcgcccggatctacaacggcaacagcttcccctactg gggccagggcaccaccgtgaccgtgagcagcggtggaggcggttcaggcggaggtggttctggcggtggcggatcggacatc gtgatgacccagagccccgacagcctggccgtgagcctgggcgagcgggccaccatcaactgcaagagcagccagagcctgt tcaacagcggcaaccagaagaactacctgacctggtaccagcagaagcccggccagccccccaagctgctgatctactgggcc agcacccgggagagcggcgtgcccgaccggttcagcggcagcggcagcggcaccgacttcaccctgaccatcagcagcctgc aggccgaggacgtggccgtgtactactgccagaacgcctacagcttcccctacaccttcggcggcggcaccaagctggagatca agcggaccacgacgccagcgccgcgaccaccaacaccggcgcccaccatcgcgtcgcagcccctgtccctgcgcccagagg cgtgccggccagcggcggggggcgcagtgcacacgagggggctggacttcgcctgtgatatctacatctgggcgcccttggcc gggacttgtggggtccttctcctgtcactggttatcaccctttactgcaaacggggcagaaagaaactcctgtatatattcaaacaacc atttatgagaccagtacaaactactcaagaggaagatggctgtagctgccgatttccagaagaagaagaaggaggatgtgaactg agagtgaagttcagcaggagcgcagacgcccccgcgtaccagcagggccagaaccagctctataacgagctcaatctaggacg aagagaggagtacgatgttttggacaagagacgtggccgggaccctgagatggggggaaagccgcagagaaggaagaaccct caggaaggcctgtacaatgaactgcagaaagataagatggcggaggcctacagtgagattgggatgaaaggcgagcgccgga ggggcaaggggcacgatggcctttaccagggtctcagtacagccaccaaggacacctacgacgcccttcacatgcaggccctg ccccctcgctagtaagctagccgatggctccggtgcccgtcagtgggcagagcgcacatcgcccacagtccccgagaagttggg gggaggggtcggcaattgaaccggtgcctagagaaggtggcgcggggtaaactgggaaagtgatgtcgtgtactggctccgcct ttttcccgagggtgggggagaaccgtatataagtgcagtagtcgccgtgaacgttctttttcgcaacgggtttgccgccagaacaca ggtgtcgtgacgcggatccaggcctaagcttacgcgtgccaccatggcgctccctgtcaccgcactgcttcttccgctggcactgc tgctgcacgctgcacggcctcagatgcagctagtgcagtctggggctgaggtgaagaagcctggggcctcagtgaaggtttcctg caaggcatctggatacaccttcaccagctactatatgcactgggtgcgacaggcccctggacaagggcttgagtggatgggaata atcaaccctagtggtggtagcacaagctacgcacagaagttccagggcagagtcaccatgaccagggacacgtccacgagcac agtctacatggagctgagcagcctgagatctgaggacacggccgtgtattactgtgcgagtagtcggagtgggactacggtggta aatcatgatgcttttgatatctgggggaaagggaccacggtcaccgtctcgagtggtggaggcggttcaggcggaggtggttctgg |
| | | cggtggcggatcggacatccagttgacccagtctccatcctccctgtctgcgtctgtaggagacagagtcaccatcacttgccggg caagccaggtcattagccgtgctttagcctggtatcaacaaacaccagggaaacctcctaaactcctgatctatgatgcctccaattt gcagagtggggtcccatcaaggttcagcggcagtggatctgggacagatttcactctcaccatcagccgcctgcagcctgaagatt ttgcaacttattactgtcaacagtttaatagttaccctctcactttcggcggagggaccaagctggagatcaaacgtggacaagatgc aagttctgctggatcaaccaggaataaagatccaacaagacgtccagaactagaagctggtacaaatggaagaagttcgacaaca agtccctttgtaaaaccaaatccaggttctagcacagacggcaacagcgccggacattcggggaacaacatcctcggttccgaag tggccttatttgcactcatgtacgtggcagcggccgccttcgtgctcctgttctttgtgggctgtggggtgctgctgtcccgcaagcg ccggcggatgaagctgctgagcagcatcgagcaggcctgtgacatctgccggctgaagaaactgaagtgcagcaaagaaaagc ccaagtgcgccaagtgcctgaagaacaactgggagtgccggtacagccccaagaccaagagaagcccccctgaccagagccca cctgaccgaggtggaaagccggctggaaagactggaacagctgtttctgctgatcttcccacgcgaggacctggacatgatcctg aagatggacagcctgcaggacatcaaggccctgctgaccggcctgttcgtgcaggacaacgtgaacaaggacgccgtgaccga cagactggccagcgtggaaaccgacatgcccctgaccctgcggcagcacagaatcagcgccaccagcagcagcgaggaaag cagcaacaagggccagcggcagctgacagtgtctgctgctgcaggcggaagcggaggctctggcggatctgatgccctggac gacttcgacctggatatgctgggcagcgacgccctggatgattttgatctggacatgctgggatctgacgctctggacgatttcgatc tcgacatgttgggatcagatgcactggatgactttgacctggacatgctcggatca |
| 69 | Mesothelin-synEphrinB 2EC-Claudin18.2-CAR-IL7 | aagcttgatatcgaattcggagcactgtcctccgaacgtcggagcactgtcctccgaacgtcggagcactgtcctccgaacgtcgg agcactgtcctccgaacggagcatgtcctccgaacgtcggagcactgtcctccgaacgactagttaggcgtgtacggtgggaggc ctatataagcagagctcgtttagtgaaccgtcagatcgcctggagacgccatccacgctgttttgacctccatagaagacaccggg accgatccagcctctcgacattcgttggatcatggccttaccagtgaccgccttgctcctgccgctggccttgctgctccacgccgc caggccgcaggtgcagctgcaggagagcggccccggcctgatcaagcccagccagaccctgagcctgacctgcaccgtgagc ggcggcagcatcagcagcggctacaactggcactggatccggcagccccccggcaagggcctggagtggatcggctacatcc actacaccggcagcaccaactacaaccccgccctgcggagccgggtgaccatcagcgtggacaccagcaagaaccagttcag cctgaagctgagcagcgtgaccgccgccgacaccgccatctactactgcgcccggatctacaacggcaacagcttcccctactg gggccagggcaccaccgtgaccgtgagcagcggtggaggcggttcaggcggaggtggttctggcggtggcggatcggacatc gtgatgacccagagccccgacagcctggccgtgagcctgggcgagcgggccaccatcaactgcaagagcagccagagcctgt tcaacagcggcaaccagaagaactacctgacctggtaccagcagaagcccggccagccccccaagctgctgatctactgggcc agcacccgggagagcggcgtgcccgaccggttcagcggcagcggcagcggcaccgacttcaccctgaccatcagcagcctgc aggccgaggacgtggccgtgtactactgccagaacgcctacagcttcccctacaccttcggcggcggcaccaagctggagatca agcggaccacgacgccagcgccgcgaccaccaacaccggcgcccaccatcgcgtcgcagcccctgtccctgcgcccagagg cgtgccggccagcggcggggggcgcagtgcacacgagggggctggacttcgcctgtgatatctacatctgggcgcccttggcc gggacttgtggggtccttctcctgtcactggttatcaccctttactgcaaacggggcagaaagaaactcctgtatatattcaaacaacc atttatgagaccagtacaaactactcaagaggaagatggctgtagctgccgatttccagaagaagaagaaggaggatgtgaactg agagtgaagttcagcaggagcgcagacgcccccgcgtaccagcagggccagaaccagctctataacgagctcaatctaggacg aagagaggagtacgatgttttggacaagagacgtggccgggaccctgagatggggggaaagccgcagagaaggaagaaccct caggaaggcctgtacaatgaactgcagaaagataagatggcggaggcctacagtgagattgggatgaaaggcgagcgccgga ggggcaaggggcacgatggcctttaccagggtctcagtacagccaccaaggacacctacgacgcccttcacatgcaggccctg ccccctcgcgctactaacttcagcctgctgaagcaggctggagacgtggaggagaaccctggacctatgttccatgtttcttttaggt atatctttggacttcctcccctgatccttgttctgttgccagtagcatcatctgattgtgatattgaaggtaaagatggcaaacaatatga gagtgttctaatggtcagcatcgatcaattattggacagcatgaaagaaattggtagcaattgcctgaataatgaatttaacttttttaaa agacatatctgtgatgctaataaggaaggtatgtttttattccgtgctgctcgcaagttgaggcaatttcttaaaatgaatagcactggt gattttgatctccacttattaaaagtttcagaaggcacaacaatactgttgaactgcactggccaggttaaaggaagaaaaccagctg ccctgggtgaagcccaaccaacaaagagtttggaagaaaataaatctttaaaggaacagaaaaaactgaatgacttgtgtttcctaa agagactattacaagagataaaaacttgttggaataaaattttgatgggcactaaagaacactagtaagctagccgatggctccggt gcccgtcagtgggcagagcgcacatcgcccacagtccccgagaagttggggggaggggtcggcaattgaaccggtgcctaga |
| | | gaaggtggcgcggggtaaactgggaaagtgatgtcgtgtactggctccgcctttttcccgagggtgggggagaaccgtatataag tgcagtagtcgccgtgaacgttctttttcgcaacgggtttgccgccagaacacaggtgtcgtgacgcggatccaggcctaagcttac gcgtgccaccatggcgctccctgtcaccgcactgcttcttccgctggcactgctgctgcacgctgcacggcctcagatgcagctag tgcagtctggggctgaggtgaagaagcctggggcctcagtgaaggtttcctgcaaggcatctggatacaccttcaccagctactat atgcactgggtgcgacaggcccctggacaagggcttgagtggatgggaataatcaaccctagtggtggtagcacaagctacgca cagaagttccagggcagagtcaccatgaccagggacacgtccacgagcacagtctacatggagctgagcagcctgagatctga ggacacggccgtgtattactgtgcgagtagtcggagtgggactacggtggtaaatcatgatgcttttgatatctgggggaaaggga ccacggtcaccgtctcgagtggtggaggcggttcaggcggaggtggttctggcggtggcggatcggacatccagttgacccagt ctccatcctccctgtctgcgtctgtaggagacagagtcaccatcacttgccgggcaagccaggtcattagccgtgctttagcctggt atcaacaaacaccagggaaacctcctaaactcctgatctatgatgcctccaatttgcagagtggggtcccatcaaggttcagcggc agtggatctgggacagatttcactctcaccatcagccgcctgcagcctgaagattttgcaacttattactgtcaacagtttaatagttac cctctcactttcggcggagggaccaagctggagatcaaacgtggacaagatgcaagttctgctggatcaaccaggaataaagatc caacaagacgtccagaactagaagctggtacaaatggaagaagttcgacaacaagtccctttgtaaaaccaaatccaggttctagc acagacggcaacagcgccggacattcggggaacaacatcctcggttccgaagtggccttatttgcactcatgtacgtggcagcgg ccgccttcgtgctcctgttctttgtgggctgtggggtgctgctgtcccgcaagcgccggcggatgaagctgctgagcagcatcgag caggcctgtgacatctgccggctgaagaaactgaagtgcagcaaagaaaagcccaagtgcgccaagtgcctgaagaacaactg ggagtgccggtacagccccaagaccaagagaagccccctgaccagagcccacctgaccgaggtggaaagccggctggaaag actggaacagctgtttctgctgatcttcccacgcgaggacctggacatgatcctgaagatggacagcctgcaggacatcaaggcc ctgctgaccggcctgttcgtgcaggacaacgtgaacaaggacgccgtgaccgacagactggccagcgtggaaaccgacatgcc cctgaccctgcggcagcacagaatcagcgccaccagcagcagcgaggaaagcagcaacaagggccagcggcagctgacagt gtctgctgctgcaggcggaagcggaggctctggcggatctgatgccctggacgacttcgacctggatatgctgggcagcgacgc cctggatgattttgatctggacatgctgggatctgacgctctggacgatttcgatctcgacatgttgggatcagatgcactggatgact ttgacctggacatgctcggatca |
| 70 | Mesothelin-synEphrinB 2EC-Claudin18.2-CAR-IL7-CCL21 | aagcttgatatcgaattcggagcactgtcctccgaacgtcggagcactgtcctccgaacgtcggagcactgtcctccgaacgtcgg agcactgtcctccgaacggagcatgtcctccgaacgtcggagcactgtcctccgaacgactagttaggcgtgtacggtgggaggc ctatataagcagagctcgtttagtgaaccgtcagatcgcctggagacgccatccacgctgttttgacctccatagaagacaccggg accgatccagcctctcgacattcgttggatcatggccttaccagtgaccgccttgctcctgccgctggccttgctgctccacgccgc caggccgcaggtgcagctgcaggagagcggccccggcctgatcaagcccagccagaccctgagcctgacctgcaccgtgagc ggcggcagcatcagcagcggctacaactggcactggatccggcagccccccggcaagggcctggagtggatcggctacatcc actacaccggcagcaccaactacaaccccgccctgcggagccgggtgaccatcagcgtggacaccagcaagaaccagttcag cctgaagctgagcagcgtgaccgccgccgacaccgccatctactactgcgcccggatctacaacggcaacagcttcccctactg gggccagggcaccaccgtgaccgtgagcagcggtggaggcggttcaggcggaggtggttctggcggtggcggatcggacatc gtgatgacccagagccccgacagcctggccgtgagcctgggcgagcgggccaccatcaactgcaagagcagccagagcctgt tcaacagcggcaaccagaagaactacctgacctggtaccagcagaagcccggccagccccccaagctgctgatctactgggcc agcacccgggagagcggcgtgcccgaccggttcagcggcagcggcagcggcaccgacttcaccctgaccatcagcagcctgc aggccgaggacgtggccgtgtactactgccagaacgcctacagcttcccctacaccttcggcggcggcaccaagctggagatca agcggaccacgacgccagcgccgcgaccaccaacaccggcgcccaccatcgcgtcgcagcccctgtccctgcgcccagagg cgtgccggccagcggcggggggcgcagtgcacacgagggggctggacttcgcctgtgatatctacatctgggcgcccttggcc gggacttgtggggtccttctcctgtcactggttatcaccctttactgcaaacggggcagaaagaaactcctgtatatattcaaacaacc atttatgagaccagtacaaactactcaagaggaagatggctgtagctgccgatttccagaagaagaagaaggaggatgtgaactg agagtgaagttcagcaggagcgcagacgcccccgcgtaccagcagggccagaaccagctctataacgagctcaatctaggacg aagagaggagtacgatgttttggacaagagacgtggccgggaccctgagatggggggaaagccgcagagaaggaagaaccct caggaaggcctgtacaatgaactgcagaaagataagatggcggaggcctacagtgagattgggatgaaaggcgagcgccgga ggggcaaggggcacgatggcctttaccagggtctcagtacagccaccaaggacacctacgacgcccttcacatgcaggccctg |
| | | cccccctcgcgctactaacttcagcctgctgaagcaggctggagacgtggaggagaaccctggacctatgttccatgtttcttttaggt atatctttggacttcctcccctgatccttgttctgttgccagtagcatcatctgattgtgatattgaaggtaaagatggcaaacaatatga gagtgttctaatggtcagcatcgatcaattattggacagcatgaaagaaattggtagcaattgcctgaataatgaatttaacttttttaaa agacatatctgtgatgctaataaggaaggtatgtttttattccgtgctgctcgcaagttgaggcaatttcttaaaatgaatagcactggt gattttgatctccacttattaaaagtttcagaaggcacaacaatactgttgaactgcactggccaggttaaaggaagaaaaccagctg ccctgggtgaagcccaaccaacaaagagtttggaagaaaataaatctttaaaggaacagaaaaaactgaatgacttgtgtttcctaa agagactattacaagagataaaaacttgttggaataaaattttgatgggcactaaagaacacgagggcagaggaagtctgctaaca tgcggtgacgtcgaggagaatcctggacctatggctcagtcactggctctgagcctccttatcctggttctggcctttggcatcccca ggacccaaggcagtgatggaggggctcaggactgttgcctcaagtacagccaaaggaagattcccgccaaggttgtccgcagct accggaagcaggaaccaagcttaggctgctccatcccagctatcctgttcttgccccgcaagcgctctcaggcagagctatgtgca gacccaaaggagctctgggtgcagcagctgatgcagcatctggacaagacaccatccccacagaaaccagcccagggctgca ggaaggacaggggggcctccaagactggcaagaaaggaaagggctccaaaggctgcaagaggactgagcggtcacagacc cctaaagggccatagtaagctagccgatggctccggtgcccgtcagtgggcagagcgcacatcgcccacagtccccgagaagtt ggggggaggggtcggcaattgaaccggtgcctagagaaggtggcgcggggtaaactgggaaagtgatgtcgtgtactggctcc gcctttttcccgagggtgggggagaaccgtatataagtgcagtagtcgccgtgaacgttctttttcgcaacgggtttgccgccagaa cacaggtgtcgtgacgcggatccaggcctaagcttacgcgtgccaccatggcgctccctgtcaccgcactgcttcttccgctggca ctgctgctgcacgctgcacggcctcagatgcagctagtgcagtctggggctgaggtgaagaagcctggggcctcagtgaaggttt cctgcaaggcatctggatacaccttcaccagctactatatgcactgggtgcgacaggcccctggacaagggcttgagtggatggg aataatcaaccctagtggtggtagcacaagctacgcacagaagttccagggcagagtcaccatgaccagggacacgtccacgag cacagtctacatggagctgagcagcctgagatctgaggacacggccgtgtattactgtgcgagtagtcggagtgggactacggtg gtaaatcatgatgcttttgatatctgggggaaagggaccacggtcaccgtctcgagtggtggaggcggttcaggcggaggtggttc tggcggtggcggatcggacatccagttgacccagtctccatcctccctgtctgcgtctgtaggagacagagtcaccatcacttgcc gggcaagccaggtcattagccgtgctttagcctggtatcaacaaacaccagggaaacctcctaaactcctgatctatgatgcctcca atttgcagagtggggtcccatcaaggttcagcggcagtggatctgggacagatttcactctcaccatcagccgcctgcagcctgaa gattttgcaacttattactgtcaacagtttaatagttaccctctcactttcggcggagggaccaagctggagatcaaacgtggacaag atgcaagttctgctggatcaaccaggaataaagatccaacaagacgtccagaactagaagctggtacaaatggaagaagttcgac aacaagtccctttgtaaaaccaaatccaggttctagcacagacggcaacagcgccggacattcggggaacaacatcctcggttcc gaagtggccttatttgcactcatgtacgtggcagcggccgccttcgtgctcctgttctttgtgggctgtggggtgctgctgtcccgca agcgccggcggatgaagctgctgagcagcatcgagcaggcctgtgacatctgccggctgaagaaactgaagtgcagcaaagaa aagcccaagtgcgccaagtgcctgaagaacaactgggagtgccggtacagccccaagaccaagagaagccccctgaccagag cccacctgaccgaggtggaaagccggctggaaagactggaacagctgtttctgctgatcttcccacgcgaggacctggacatgat cctgaagatggacagcctgcaggacatcaaggccctgctgaccggcctgttcgtgcaggacaacgtgaacaaggacgccgtga ccgacagactggccagcgtggaaaccgacatgcccctgaccctgcggcagcacagaatcagcgccaccagcagcagcgagg aaagcagcaacaagggccagcggcagctgacagtgtctgctgctgcaggcggaagcggaggctctggcggatctgatgccctg gacgacttcgacctggatatgctgggcagcgacgccctggatgattttgatctggacatgctgggatctgacgctctggacgatttc gatctcgacatgttgggatcagatgcactggatgactttgacctggacatgctcggatca |
| 71 | FAP-synEphrinB2EC-C laudin18.2-CAR | aagcttgatatcgaattcggagcactgtcctccgaacgtcggagcactgtcctccgaacgtcggagcactgtcctccgaacgtcgg agcactgtcctccgaacggagcatgtcctccgaacgtcggagcactgtcctccgaacgactagttaggcgtgtacggtgggaggc ctatataagcagagctcgtttagtgaaccgtcagatcgcctggagacgccatccacgctgttttgacctccatagaagacaccggg accgatccagcctctcgacattcgttggatcatggccttaccagtgaccgccttgctcctgccgctggccttgctgctccacgccgc caggccgcaggtgcagctgcaggagagcggccccggcctgatcaagcccagccagaccctgagcctgacctgcaccgtgagc ggcggcagcatcagcagcggctacaactggcactggatccggcagccccccggcaagggcctggagtggatcggctacatcc actacaccggcagcaccaactacaaccccgccctgcggagccgggtgaccatcagcgtggacaccagcaagaaccagttcag cctgaagctgagcagcgtgaccgccgccgacaccgccatctactactgcgcccggatctacaacggcaacagcttcccctactg |
| | | gggccagggcaccaccgtgaccgtgagcagcggtggaggcggttcaggcggaggtggttctggcggtggcggatcggacatc gtgatgacccagagccccgacagcctggccgtgagcctgggcgagcgggccaccatcaactgcaagagcagccagagcctgt tcaacagcggcaaccagaagaactacctgacctggtaccagcagaagcccggccagccccccaagctgctgatctactgggcc agcacccgggagagcggcgtgcccgaccggttcagcggcagcggcagcggcaccgacttcaccctgaccatcagcagcctgc aggccgaggacgtggccgtgtactactgccagaacgcctacagcttcccctacaccttcggcggcggcaccaagctggagatca agcggaccacgacgccagcgccgcgaccaccaacaccggcgcccaccatcgcgtcgcagcccctgtccctgcgcccagagg cgtgccggccagcggcggggggcgcagtgcacacgagggggctggacttcgcctgtgatatctacatctgggcgcccttggcc gggacttgtggggtccttctcctgtcactggttatcaccctttactgcaaacggggcagaaagaaactcctgtatatattcaaacaacc atttatgagaccagtacaaactactcaagaggaagatggctgtagctgccgatttccagaagaagaagaaggaggatgtgaactg agagtgaagttcagcaggagcgcagacgcccccgcgtaccagcagggccagaaccagctctataacgagctcaatctaggacg aagagaggagtacgatgttttggacaagagacgtggccgggaccctgagatggggggaaagccgcagagaaggaagaaccct caggaaggcctgtacaatgaactgcagaaagataagatggcggaggcctacagtgagattgggatgaaaggcgagcgccgga ggggcaaggggcacgatggcctttaccagggtctcagtacagccaccaaggacacctacgacgcccttcacatgcaggccctg ccccctcgctagtaagctagccgatggctccggtgcccgtcagtgggcagagcgcacatcgcccacagtccccgagaagttggg gggaggggtcggcaattgaaccggtgcctagagaaggtggcgcggggtaaactgggaaagtgatgtcgtgtactggctccgcct ttttcccgagggtgggggagaaccgtatataagtgcagtagtcgccgtgaacgttctttttcgcaacgggtttgccgccagaacaca ggtgtcgtgacgcggatccaggcctaagcttacgcgtgccaccatggcgctccctgtcaccgcactgcttcttccgctggcactgc tgctgcacgctgcacggcctcaggtgcaattggtgcagtctggggctgaggtgaagaagcctgggtcctcggtgaaggtctcctg caaggcctccggaggcacattcagcagctacgctataagctgggtgcgacaggcccctggacaagggctcgagtggatgggag ggatcatccctatctttggtacagcaaactacgcacagaagttccagggcagggtcaccattactgcagacaaatccacgagcaca gcctacatggagctgagcagcctgagatctgaggacaccgccgtgtattactgtgcgagagatgctgctgatagggactactggg gccaagggaccaccgtgaccgtctcctcaggtggaggcggttcaggcggaggtggttctggcggtggcggatcggatattgttat gactcaatctccactgtctctgccggtgactccaggcgaaccggcgagcatttcttgccgttccagccagtctctgcttcaccccaac ggcttcaaccatctctattggtacctgcaaaaaccgggtcagagccctcagctgctgatctacgtggggggtaaccgcgcttccgg tgtaccggaccgtttcagcggctctggatccggcaccgatttcacgttgaaaatcagccgtgttgaagcagaagacgtgggcgttta ttactgtcagcagcgtaataataagaatcgtacttttggtcaaggcaccaaggtcgaaattaaacgtggacaagatgcaagttctgct ggatcaaccaggaataaagatccaacaagacgtccagaactagaagctggtacaaatggaagaagttcgacaacaagtccctttg taaaaccaaatccaggttctagcacagacggcaacagcgccggacattcggggaacaacatcctcggttccgaagtggccttattt gcactcatgtacgtggcagcggccgccttcgtgctcctgttctttgtgggctgtggggtgctgctgtcccgcaagcgccggcggat gaagctgctgagcagcatcgagcaggcctgtgacatctgccggctgaagaaactgaagtgcagcaaagaaaagcccaagtgcg ccaagtgcctgaagaacaactgggagtgccggtacagccccaagaccaagagaagccccctgaccagagcccacctgaccga ggtggaaagccggctggaaagactggaacagctgtttctgctgatcttcccacgcgaggacctggacatgatcctgaagatggac agcctgcaggacatcaaggccctgctgaccggcctgttcgtgcaggacaacgtgaacaaggacgccgtgaccgacagactggc cagcgtggaaaccgacatgcccctgaccctgcggcagcacagaatcagcgccaccagcagcagcgaggaaagcagcaacaa gggccagcggcagctgacagtgtctgctgctgcaggcggaagcggaggctctggcggatctgatgccctggacgacttcgacct ggatatgctgggcagcgacgccctggatgattttgatctggacatgctgggatctgacgctctggacgatttcgatctcgacatgttg ggatcagatgcactggatgactttgacctggacatgctcggatca |
| 72 | Claudin18.2-synEphrin B2EC-Mesothelin-CAR | aagcttgatatcgaattcggagcactgtcctccgaacgtcggagcactgtcctccgaacgtcggagcactgtcctccgaacgtcgg agcactgtcctccgaacggagcatgtcctccgaacgtcggagcactgtcctccgaacgactagttaggcgtgtacggtgggaggc ctatataagcagagctcgtttagtgaaccgtcagatcgcctggagacgccatccacgctgttttgacctccatagaagacaccggg accgatccagcctctcgacattcgttggatcatggccttaccagtgaccgccttgctcctgccgctggccttgctgctccacgccgc caggccgcagatgcagctagtgcagtctggggctgaggtgaagaagcctggggcctcagtgaaggtttcctgcaaggcatctgg atacaccttcaccagctactatatgcactgggtgcgacaggcccctggacaagggcttgagtggatgggaataatcaaccctagtg gtggtagcacaagctacgcacagaagttccagggcagagtcaccatgaccagggacacgtccacgagcacagtctacatggag |
| | | ctgagcagcctgagatctgaggacacggccgtgtattactgtgcgagtagtcggagtgggactacggtggtaaatcatgatgctttt gatatctgggggaaagggaccacggtcaccgtctcgagtggtggaggcggttcaggcggaggtggttctggcggtggcggatc ggacatccagttgacccagtctccatcctccctgtctgcgtctgtaggagacagagtcaccatcacttgccgggcaagccaggtca ttagccgtgctttagcctggtatcaacaaacaccagggaaacctcctaaactcctgatctatgatgcctccaatttgcagagtggggt cccatcaaggttcagcggcagtggatctgggacagatttcactctcaccatcagccgcctgcagcctgaagattttgcaacttattac tgtcaacagtttaatagttaccctctcactttcggcggagggaccaagctggagatcaaacgtaccacgacgccagcgccgcgac caccaacaccggcgcccaccatcgcgtcgcagcccctgtccctgcgcccagaggcgtgccggccagcggcggggggcgcag tgcacacgagggggctggacttcgcctgtgatatctacatctgggcgcccttggccgggacttgtggggtccttctcctgtcactgg ttatcaccctttactgcaaacggggcagaaagaaactcctgtatatattcaaacaaccatttatgagaccagtacaaactactcaaga ggaagatggctgtagctgccgatttccagaagaagaagaaggaggatgtgaactgagagtgaagttcagcaggagcgcagacg cccccgcgtaccagcagggccagaaccagctctataacgagctcaatctaggacgaagagaggagtacgatgttttggacaaga gacgtggccgggaccctgagatggggggaaagccgcagagaaggaagaaccctcaggaaggcctgtacaatgaactgcaga aagataagatggcggaggcctacagtgagattgggatgaaaggcgagcgccggaggggcaaggggcacgatggcctttacca gggtctcagtacagccaccaaggacacctacgacgcccttcacatgcaggccctgccccctcgctagtaagctagccgatggctc cggtgcccgtcagtgggcagagcgcacatcgcccacagtccccgagaagttggggggaggggtcggcaattgaaccggtgcc tagagaaggtggcgcggggtaaactgggaaagtgatgtcgtgtactggctccgcctttttcccgagggtgggggagaaccgtata taagtgcagtagtcgccgtgaacgttctttttcgcaacgggtttgccgccagaacacaggtgtcgtgacgcggatccaggcctaag cttacgcgtcctagcgctaccggtcgccaccatggccttaccagtgaccgccttgctcctgccgctggccttgctgctccacgccg ccaggccgcaggtgcagctgcaggagagcggccccggcctgatcaagcccagccagaccctgagcctgacctgcaccgtgag cggcggcagcatcagcagcggctacaactggcactggatccggcagccccccggcaagggcctggagtggatcggctacatc cactacaccggcagcaccaactacaaccccgccctgcggagccgggtgaccatcagcgtggacaccagcaagaaccagttca gcctgaagctgagcagcgtgaccgccgccgacaccgccatctactactgcgcccggatctacaacggcaacagcttcccctact ggggccagggcaccaccgtgaccgtgagcagcggtggaggcggttcaggcggaggtggttctggcggtggcggatcggacat cgtgatgacccagagccccgacagcctggccgtgagcctgggcgagcgggccaccatcaactgcaagagcagccagagcctg ttcaacagcggcaaccagaagaactacctgacctggtaccagcagaagcccggccagccccccaagctgctgatctactgggcc agcacccgggagagcggcgtgcccgaccggttcagcggcagcggcagcggcaccgacttcaccctgaccatcagcagcctgc aggccgaggacgtggccgtgtactactgccagaacgcctacagcttcccctacaccttcggcggcggcaccaagctggagatca agcggggacaagatgcaagttctgctggatcaaccaggaataaagatccaacaagacgtccagaactagaagctggtacaaatg gaagaagttcgacaacaagtccctttgtaaaaccaaatccaggttctagcacagacggcaacagcgccggacattcggggaaca acatcctcggttccgaagtggccttatttgcactcatgtacgtggcagcggccgccttcgtgctcctgttctttgtgggctgtggggtg ctgctgtcccgcaagcgccggcggatgaagctgctgagcagcatcgagcaggcctgtgacatctgccggctgaagaaactgaa gtgcagcaaagaaaagcccaagtgcgccaagtgcctgaagaacaactgggagtgccggtacagccccaagaccaagagaagc cccctgaccagagcccacctgaccgaggtggaaagccggctggaaagactggaacagctgtttctgctgatcttcccacgcgag gacctggacatgatcctgaagatggacagcctgcaggacatcaaggccctgctgaccggcctgttcgtgcaggacaacgtgaac aaggacgccgtgaccgacagactggccagcgtggaaaccgacatgcccctgaccctgcggcagcacagaatcagcgccacca gcagcagcgaggaaagcagcaacaagggccagcggcagctgacagtgtctgctgctgcaggcggaagcggaggctctggcg gatctgatgccctggacgacttcgacctggatatgctgggcagcgacgccctggatgattttgatctggacatgctgggatctgacg ctctggacgatttcgatctcgacatgttgggatcagatgcactggatgactttgacctggacatgctcggatca |
| 73 | Amino acid sequence of CLL1-synEphrinB2EC | evqlvqsgaevkkpgasvkvsckasgytfnsyymhwvrqapgqglewmgifnpgggstsyaqkfqgrvtmtrdtststvy melsslrsedtavyycargttsdafdiwgqgtmvtvssggggsggggsggggsdiqmtqspsslsasvgdrvtitcrasqsiss ylnwyqqkpgkapklliydvasslqsgvpsrfsgsgsgtdftltisslqpedfatyycqqsystpltfgggtkveikrgqdassags trnkdptrrpeleagtngrssttspfvkpnpgsstdgnsaghsgnnilgsevalfalmyvaaaafvllffvgcgvllsrkrrrmkll ssieqacdicrlkklkcskekpkcakclknnwecryspktkrspltrahltevesrlerleqlfllifpredldmilkmdslqdika llltglfvqdnvnkdavtdrlasvetdmpltlrqhrisatssseessnkgqrqltvsaaaggsggsggsdalddfdldmlgsdald |
| | | dfdldmlgsdalddfdldmlgsdalddfdldmlgs |
| 74 | CLL1-synEphrinB2EC-NKG2D-CAR | aagcttgatatcgaattcggagcactgtcctccgaacgtcggagcactgtcctccgaacgtcggagcactgtcctccgaacgtcgg agcactgtcctccgaacggagcatgtcctccgaacgtcggagcactgtcctccgaacgactagttaggcgtgtacggtgggaggc ctatataagcagagctcgtttagtgaaccgtcagatcgcctggagacgccatccacgctgttttgacctccatagaagacaccggg accgatccagcctctcgacattcgttggatcatgagagtgaagttcagcaggagcgcagacgcccccgcgtaccagcagggcca gaaccagctctataacgagctcaatctaggacgaagagaggagtacgatgttttggacaagagacgtggccgggaccctgagat ggggggaaagccgcagagaaggaagaaccctcaggaaggcctgtacaatgaactgcagaaagataagatggcggaggccta cagtgagattgggatgaaaggcgagcgccggaggggcaaggggcacgatggcctttaccagggtctcagtacagccaccaag gacacctacgacgcccttcacatgcaggccctgccccctcgcgggtggattcgtggtcggaggtctcgacacagctgggagatg agtgaatttcataattataacttggatctgaagaagagtgatttttcaacacgatggcaaaagcaaagatgtccagtagtcaaaagca aatgtagagaaaatgcatctccattttttttctgctgcttcatcgctgtagccatgggaatccgtttcattattatggtagctatatggagtg ctgtattcctaaactcattattcaaccaagaagttcaaattcccttgaccgaaagttactgtggcccatgtcctaaaaactggatatgtta caaaaataactgctaccaattttttgatgagagtaaaaactggtatgagagccaggcttcttgtatgtctcaaaatgccagccttctgaa agtatacagcaaagaggaccaggatttacttaaactggtgaagtcatatcattggatgggactagtacacattccaacaaatggatct tggcagtgggaagatggctccattctctcacccaacctactaacaataattgaaatgcagaagggagactgtgcactctatgcctcg agctttaaaggctatatagaaaactgttcaactccaaatacgtacatctgcatgcaaaggactgtgtaagctagcatcgatggctccg gtgcccgtcagtgggcagagcgcacatcgcccacagtccccgagaagttggggggaggggtcggcaattgaaccggtgccta gagaaggtggcgcggggtaaactgggaaagtgatgtcgtgtactggctccgcctttttcccgagggtgggggagaaccgtatata agtgcagtagtcgccgtgaacgttctttttcgcaacgggtttgccgccagaacacaggtgtcgtgacgcggatccaggcctaagctt acgcgtcctagcgctaccggtcgccaccatggcgctccctgtcaccgcactgcttcttccgctggcactgctgctgcacgctgcac ggcctgaggtgcagctggtgcagtctggggctgaggtgaagaagcctggggcctcagtgaaggtttcctgcaaggcatctggat acaccttcaacagctactatatgcactgggtgcgacaggcccctggacaagggcttgagtggatgggaatattcaaccctggtggt ggtagcacaagctacgcacagaagttccagggcagagtcaccatgaccagggacacgtccacgagcacagtctacatggagct gagcagcctgagatctgaggacacggccgtgtattactgtgcgagaggaactacatccgatgcttttgatatctggggccaaggga caatggtcaccgtctcgagtggtggaggcggttcaggcggaggtggttctggcggtggcggatcggacatccagatgacccagt ctccatcctccctgtctgcatctgtaggagacagagtcaccatcacttgccgggcaagtcagagcattagcagctatttaaattggtat cagcagaaaccagggaaagcccctaagctcctgatcgatgtcgcatccagtttgcaaagtggggtcccatcaaggttcagtggca gtggatctgggacagatttcactctcaccatcagcagtctgcaacctgaagattttgcaacttactactgtcaacagagttacagtacc cctctcactttcggcggagggaccaaggtggagatcaaacgtggacaagatgcaagttctgctggatcaaccaggaataaagatc caacaagacgtccagaactagaagctggtacaaatggaagaagttcgacaacaagtccctttgtaaaaccaaatccaggttctagc acagacggcaacagcgccggacattcggggaacaacatcctcggttccgaagtggccttatttgcactcatgtacgtggcagcgg ccgccttcgtgctcctgttctttgtgggctgtggggtgctgctgtcccgcaagcgccggcggatgaagctgctgagcagcatcgag caggcctgtgacatctgccggctgaagaaactgaagtgcagcaaagaaaagcccaagtgcgccaagtgcctgaagaacaactg ggagtgccggtacagccccaagaccaagagaagccccctgaccagagcccacctgaccgaggtggaaagccggctggaaag actggaacagctgtttctgctgatcttcccacgcgaggacctggacatgatcctgaagatggacagcctgcaggacatcaaggcc ctgctgaccggcctgttcgtgcaggacaacgtgaacaaggacgccgtgaccgacagactggccagcgtggaaaccgacatgcc cctgaccctgcggcagcacagaatcagcgccaccagcagcagcgaggaaagcagcaacaagggccagcggcagctgacagt gtctgctgctgcaggcggaagcggaggctctggcggatctgatgccctggacgacttcgacctggatatgctgggcagcgacgc cctggatgattttgatctggacatgctgggatctgacgctctggacgatttcgatctcgacatgttgggatcagatgcactggatgact ttgacctggacatgctcggatca |
| 75 | CD8 hinge region | tttpaprpptpaptiasqplslrpeacrpaaggavhtrgldfacd |
| 76 | CD8 transmembrane region | iyiwaplagtcgvlllslvitlyc |
| 77 | CD28 transmembrane region | fwvlvvvggvlacysllvtvafiifwv |
| 78 | CD28 intracellular region | rskrsrllhsdymnmtprrpgptrkhyqpyapprdfaayrs |
| 79 | 4-1BB | krgrkkllyifkqpfmrpvqttqeedgcscrfpeeeeggcel |
| 80 | CD3ζ | rvkfsrsadapayqqgqnqlynelnlgrreeydvldkrrgrdpemggkpqrrknpqeglynelqkdkmaeayseigmkge rrrgkghdglyqglstatkdtydalhmqalppr |
| 81 | CD8α signaling domain | malpvtalllplalllhaarp |
| 82 | 28Z | tttpaprpptpaptiasqplslrpeacrpaaggavhtrgldfacdfwvlvvvggvlacysllvtvafiifwvrskrsrllhsdymn mtprrpgptrkhyqpyapprdfaayrsrvkfsrsadapayqqgqnqlynelnlgrreeydvldkrrgrdpemggkpqrrkn pqeglynelqkdkmaeayseigmkgerrrgkghdglyqglstatkdtydalhmqalppr |
| 83 | BBZ | tttpaprpptpaptiasqplslrpeacrpaaggavhtrgldfacdiyiwaplagtcgvlllslvitlyckrgrkkllyifkqpfmrpv qttqeedgcscrfpeeeeggcelrvkfsrsadapayqqgqnqlynelnlgrreeydvldkrrgrdpemggkpqrrknpqegl ynelqkdkmaeayseigmkgerrrgkghdglyqglstatkdtydalhmqalppr |
| 84 | 28BBZ | tttpaprpptpaptiasqplslrpeacrpaaggavhtrgldfacdfwvlvvvggvlacysllvtvafiifwvrskrsrllhsdymn mtprrpgptrkhyqpyapprdfaayrskrgrkkllyifkqpfmrpvqttqeedgcscrfpeeeeggcelrvkfsrsadapayq qgqnqlynelnlgrreeydvldkrrgrdpemggkpqrrknpqeglynelqkdkmaeayseigmkgerrrgkghdglyqgl statkdtydalhmqalppr |
| 85 | Receptor regulatory domain of synEphrinB2EC-8 | gqdassagstrnkdptrrpeleagtngrssttspfvkpnpgsstdgnsaghsgnnilgsevalfafmyvaaaafvllffvgcgvll srkrrr |
| 86 | anti CD123-scFv | evqllesggglvqpggslrlscaasgftfssyamswvrqapgkglewvsaisgsggstyyadsvkgrftisrdnskntlylqm nslraedtavyycakddsfyggfdywgqgtlvtvssggggsggggsggggsdiqmtqspstlsasvgdrvtitcrasqsissw lawyqqkpgkapklliydasslesgvpsrfsgsgsgteftltisslqpddfatyycqqyssykytfgqgtkveikr |
| 87 | Amino acid sequence of GPC3-EphrinB2EC ( comprsing human synotch transmembrane region) | eqkliseedlevqlvqsgaevkkpgasvkvsckasgytfsdyemhwvrqapgqglewmgaihpgsgdtaynqrfkgrvti tadkststaymelsslrsedtavyycarfysyaywgqgtlvtvsaggggsggggsggggsdivmtqtplslpvtpgepasiscr ssqslvhsngntylqwylqkpgqspqlliykvsnrfsgvpdrfsgsgsgtdftlkisrveaedvgvyycsqsiyvpytfgqgtkl eikrgqdassagstrnkdptrrpeleagtngrssttspfvkpnpgsstdgnsaghsgnnilgsevalfafmyvaaaafvllffvgc gvllsrkrrrmkllssieqacdicrlkklkcskekpkcakclknnwecryspktkrspltrahltevesrlerleqlfllifpredld milkmdslqdikalltglfvqdnvnkdavtdrlasvetdmpltlrqhrisatssseessnkgqrqltvsaaaggsggsggsdald dfdldmlgsdalddfdldmlgsdalddfdldmlgsdalddfdldmlgs |
| 88 | CD123-synEphrinB2E C-NKG2D-CAR | aagcttgatatcgaattcggagcactgtcctccgaacgtcggagcactgtcctccgaacgtcggagcactgtcctccgaacgtcgg agcactgtcctccgaacggagcatgtcctccgaacgtcggagcactgtcctccgaacgactagttaggcgtgtacggtgggaggc ctatataagcagagctcgtttagtgaaccgtcagatcgcctggagacgccatccacgctgttttgacctccatagaagacaccggg accgatccagcctctcgacattcgttggatcatgagagtgaagttcagcaggagcgcagacgcccccgcgtaccagcagggcca gaaccagctctataacgagctcaatctaggacgaagagaggagtacgatgttttggacaagagacgtggccgggaccctgagat ggggggaaagccgcagagaaggaagaaccctcaggaaggcctgtacaatgaactgcagaaagataagatggcggaggccta cagtgagattgggatgaaaggcgagcgccggaggggcaaggggcacgatggcctttaccagggtctcagtacagccaccaag gacacctacgacgcccttcacatgcaggccctgccccctcgcgggtggattcgtggtcggaggtctcgacacagctgggagatg agtgaatttcataattataacttggatctgaagaagagtgatttttcaacacgatggcaaaagcaaagatgtccagtagtcaaaagca aatgtagagaaaatgcatctccattttttttctgctgcttcatcgctgtagccatgggaatccgtttcattattatggtagctatatggagtg ctgtattcctaaactcattattcaaccaagaagttcaaattcccttgaccgaaagttactgtggcccatgtcctaaaaactggatatgtta caaaaataactgctaccaattttttgatgagagtaaaaactggtatgagagccaggcttcttgtatgtctcaaaatgccagccttctgaa agtatacagcaaagaggaccaggatttacttaaactggtgaagtcatatcattggatgggactagtacacattccaacaaatggatct tggcagtgggaagatggctccattctctcacccaacctactaacaataattgaaatgcagaagggagactgtgcactctatgcctcg |
| | | agctttaaaggctatatagaaaactgttcaactccaaatacgtacatctgcatgcaaaggactgtgtaagctagcatcgatggctccg gtgcccgtcagtgggcagagcgcacatcgcccacagtccccgagaagttggggggaggggtcggcaattgaaccggtgccta gagaaggtggcgcggggtaaactgggaaagtgatgtcgtgtactggctccgcctttttcccgagggtgggggagaaccgtatata agtgcagtagtcgccgtgaacgttctttttcgcaacgggtttgccgccagaacacaggtgtcgtgacgcggatccaggcctaagctt acgcgtcctagcgctaccggtcgccaccatggccttaccagtgaccgccttgctcctgccgctggccttgctgctccacgccgcca ggccggaggtgcaattgctggagtctgggggaggcttggtacagcctggggggtccctgagactctcctgtgcagcctccggatt cacctttagcagttatgccatgagctgggtccgccaggctccagggaaggggctggagtgggtctcagctattagtggtagtggtg gtagcacatactacgcagactccgtgaagggccggttcaccatctccagagacaattccaagaacacgctgtatctgcagatgaac agcctgagagccgaggacacggccgtatattactgtgcgaaagatgattctttctacggtggtttcgactactggggccaaggaac cctggtcaccgtctcgagtggtggaggcggttcaggcggaggtggttctggcggtggcggatcggacatccagatgacccagtc tccttccaccctgtctgcatctgtaggagaccgtgtcaccatcacttgccgtgccagtcagagtattagtagctggttggcctggtatc agcagaaaccagggaaagcccctaagctcctgatctatgatgcctccagtttggaaagtggggtcccatcacgtttcagcggcagt ggatccgggacagaattcactctcaccatcagcagcttgcagcctgatgattttgcaacttattactgccaacagtactcttcttacaa atacacgtttggccagggcaccaaagtcgagatcaagcgtggacaagatgcaagttctgctggatcaaccaggaataaagatcca acaagacgtccagaactagaagctggtacaaatggaagaagttcgacaacaagtccctttgtaaaaccaaatccaggttctagcac agacggcaacagcgccggacattcggggaacaacatcctcggttccgaagtggccttatttgcactcatgtacgtggcagcggcc gccttcgtgctcctgttctttgtgggctgtggggtgctgctgtcccgcaagcgccggcggatgaagctgctgagcagcatcgagca ggcctgtgacatctgccggctgaagaaactgaagtgcagcaaagaaaagcccaagtgcgccaagtgcctgaagaacaactggg agtgccggtacagccccaagaccaagagaagccccctgaccagagcccacctgaccgaggtggaaagccggctggaaagact ggaacagctgtttctgctgatcttcccacgcgaggacctggacatgatcctgaagatggacagcctgcaggacatcaaggccctg ctgaccggcctgttcgtgcaggacaacgtgaacaaggacgccgtgaccgacagactggccagcgtggaaaccgacatgcccct gaccctgcggcagcacagaatcagcgccaccagcagcagcgaggaaagcagcaacaagggccagcggcagctgacagtgtc tgctgctgcaggcggaagcggaggctctggcggatctgatgccctggacgacttcgacctggatatgctgggcagcgacgccct ggatgattttgatctggacatgctgggatctgacgctctggacgatttcgatctcgacatgttgggatcagatgcactggatgactttg acctggacatgctcggatca |
| 89 | antiEGFRvIII-scFv2 | dvqlqsgpslvkpsqslsltctvtgysitsdfawnwirqfpgnklewmgyisysgntrynpslksrisitrdtsknqfflqlnsvti edtatyycvtagrgfpywgqgtlvtvsaggggsggggsggggsdilmtqspssmsvslgdtvsitchssqdinsnigwlqqr pgksfkgliyhgtnlddevpsrfsgsgsgadysltisslesedfadyycvqyaqfpwtfgggtkleikr |
| 90 | antiMesothelin-scFv2 | mqvqlqqsgpelekpgasvklsckasgysftgytmnwvkqshgkslewiglitpyngassynqkfrgkatltvdkssstay mdllsltsedsavyfcarggydgrgfdywgqgttvtvssggggsggggsggggsdieltqspaimsaspgekvtmtcsasss vsymhwyqqksgtspkrwiydtsklasgvpgrfsgsgsgnsysltissveaeddatyycqqwgshpltfgagtkleik |
| 91 | antiFAP-scFv2 | qvqlvqsgaevkkpgasvkvscktsrytfteytihwvrqapgqrlewigginpnngipnynqkfkgrvtitvdtsastaymel sslrsedtavyycarrriaygydeghamdywgqgtlvtvssggggsggggsggggsdivmtqspdslavslgeratinckss qsllysrnqknylawyqqkpgqppklliifwastresgvpdrfsgsgfgtdftltisslqaedvavyycqqyfsypltfgqgtkve ik |
| 92 | antiCLL1-scFv2 | evqlqqsgpelvkpgasvkmsckasgytftsyfihwvkqkpgqglewikgkatltadkssstaymelssltsedsavyyctr ddgyygyamdywgqgtsvtvssggggsggggsggggsdiqmtqspsslsaslgervsltcratqelsgylswlqqkpdgti krliyaastldsgvpkrfsgnrsgsdysltisslesedfadyyclqyaiypytfgggtkleikr |
| 93 | antiIL13Ra2-scFv1 | evqllesggglvqpggslrlscaasgftfrkyamgwvrqapgkglewvsgisgsvgstyyadsvkgrftisrdnskntlylqm nslraedtavyycakvrygwgagafdywgqgtlvtvssggggsggggsggggsdiqmtqspstlsasvgdrvtitcrasqsis swlawyqqkpgkapklliydasslesgvpsrfsgsgsgteftltisslqpddfatyycqqydtyppitfgqgtkveikr |
| 94 | antiIL13Ra2-scFv2 | gpelkkpgetvkiyckasgysfrdysvhwvkqapgkglkwmgwintetgeptyvdefkgrfaffleasantvylqisnlkne dtatyfcdyrftywgqgtlvtvsaakggggsggggsggggseivmtqtplilsvtigqpasisckssqsvlysngktylnwllqr pgqspkrliylvskldsgvpdrftgsgsgtdftlkisrveaedlgvyycvqgshfpytfgggtkleikrad |
| 95 | antiGPC3-scFv-2 | dvvmtqsplslpvtpgepasiscrssqslvhsnantylhwylqkpgqspqlliykvsnrfsgvpdrfsgsgsgtdftlkisrvea edvgvyycsqnthvpptfgqgtkleikrggggsggggsggggsqvqlvqsgaevkkpgasvkvsckasgytftdyemhw vrqapgqglewmgaldpktgdtaysqkfkgrvtltadeststaymelsslrsedtavyyctrfysytywgqgtlvtvss |
| 96 | anti Claudin18.2 scFv-2 | qvqlqqpgaelvrpgasvklsckasgytftsywinwvkqrpgqglewigniypsdsytnynqkfkdkatltvdkssstaym qlssptsedsavyyctrswrgnsfdywgqgttltvssggggsggggsggggsdivmtqspssltvtagekvtmsckssqslln sgnqknyltwyqqkpgqppklliywastresgvpdrftgsgsgtdftltissvqaedlavyycqndysypftfgsgtkleik |
| 97 | anti Claudin18.2 scFv-3 | qiqlvqsgpelkkpgetvkisckasgytftnygmnwvkqapgkglkwmgwintntgeptyaeefkgrfafsletsastaylq innlknedtatyfcarlgfgnamdywgqgtsvtvssggggsggggsggggsdivmtqspssltvtagekvtmscksssqslln sgnqknyltwyqqkpgqppklliywastresgvpdrftgsgsgtdftltissvqaedlavyycqndysypltfgagtklelkr |
| 98 | anti Claudin18.2 scFv-4 | qvqlqqpgaelvrpgasvklsckasgytftsywinwvkqrpgqglewigniypsdsytnynqkfkdkatltvdkssstaym qlssptsedsavyyctrswrgnsfdywgqgttltvssggggsggggsggggsdivmtqspssltvtagekvtmsckssqslln sgnqknyltwyqqkpgqppklliywastresgvpdrftgsgsgtdftltissvqaedlavyycqndysypftfgsgtkleikr |
| 99 | antiB7H3-scFv1 | evqllesggglvqpggslrlscaasgftfssyamswvrqapgkglewvsaisgsggstyyadsvkgrftisrdnskntlylqm nslraedtavyycakyravtfdywgqgtlvtvssggggsggggsggggsdiqmtqspstlsasvgdrvtitcrasqsisswla wyqqkpgkapklliydasslesgvpsrfsgsgsgteftltisslqpddfatyycqqfetqirwtfgqgtkveikr |
| 100 | 376.96-scFv | divmtqshkfmstsigarvsitckasqdvrtavawyqqkpgqspklliysasyrytgvpdrftgsgsgtdftftissvqaedlav yycqqhygtppwtfgggtkleikggggsggggsggggsevqlvesggglvkpggslklsceasrftfssyamswvrqtpek rlewvaaisgggrytyypdsmkgrftisrdnaknflylqmsslrsedtamyycarhydgyldywgqgttltvss |
| 101 | antiB7H3-scFv2 | evqlvesggglvqpggslrlscaasgftfssfgmhwvrqapgkglewvayissdssaiyyadtvkgrftisrdnaknslylqm nslrdedtavyycgrgreniyygsrldywgqgttvtvssggggsggggsggggsdiqltqspsflsasvgdrvtitckasqnvd tnvawyqqkpgkapkaliysasyrysgvpsrfsgsgsgtdftltisslqpedfatyycqqynnypftfgqgtkleik |
| 102 | Notch1 transmembrane region (Notch2STS) | Lmyvaaaafvllffvgcgvllskrkrkh |
| 103 | Notch1 transmembrane region (Notch3STS) | Lmyvaaaafvllffvgcgvllsrrkreh |
| 104 | Notch1 transmembrane region (Notch4STS) | Lmyvaaaafvllffvgcgvllsrrrrreh |
| 105 | Amino acid sequence of Mesothelin-synEphrinB 2EC | qmqlvqsgaevkkpgasvkvsckasgytftsyymhwvrqapgqglewmgiinpsggstsyaqkfqgrvtmtrdtststvy melsslrsedtavyycassrsgttvvnhdafdiwgkgttvtvssggggsggggsggggsdiqltqspsslsasvgdrvtitcras qvisralawyqqtpgkppklliydasnlqsgvpsrfsgsgsgtdftltisrlqpedfatyycqqfnsypltfgggtkleikrgqdas sagstrnkdptrrpeleagtngrssttspfvkpnpgsstdgnsaghsgnnilgsevalfalmyvaaaafvllffvgcgvllsrkrrr mkllssieqacdicrlkklkcskekpkcakclknnwecryspktkrspltrahltevesrlerleqlfllifpredldmilkmdslq dikalltglfvqdnvnkdavtdrlasvetdmpltlrqhrisatssseessnkgqrqltvsaaaggsggsggsdalddfdldmlgs dalddfdldmlgsdalddfdldmlgsdalddfdldmlgs |
| 106 | Amino acid sequence of FAP-synEphrinB2EC | qvqlvqsgaevkkpgssvkvsckasggtfssyaiswvrqapgqglewmggiipifgtanyaqkfqgrvtitadkststayme lssl rsedtavyycardaadrdywgqgttvtvssggggsggggsggggsdivmtqsplslpvtpgepasiscrsssqsllhpngf nhlywylqkpgqspqlliyvggnrasgvpdrfsgsgsgtdftlkisrveaedvgvyycqqrnnknrtfgqgtkveikrgqdas sagstrnkdptrrpeleagtngrssttspfvkpnpgsstdgnsaghsgnnilgsevalfalmyvaaaafvllffvgcgvllsrkrrr mkllssieqacdicrlkklkcskekpkcakclknnwecryspktkrspltrahltevesrlerleqlfllifpredldmilkmdslq dikalltglfvqdnvnkdavtdrlasvetdmpltlrqhrisatssseessnkgqrqltvsaaaggsggsggsdalddfdldmlgs dalddfdldmlgsdalddfdldmlgsdalddfdldmlgs |
| 107 | Mesothelin-CAR | qmqlvqsgaevkkpgasvkvsckasgytftsyymhwvrqapgqglewmgiinpsggstsyaqkfqgrvtmtrdtststvy melsslrsedtavyycassrsgttvvnhdafdiwgkgttvtvssggggsggggsggggsdiqltqspsslsasvgdrvtitcras |
| | | qvisralawyqqtpgkppklliydasnlqsgvpsrfsgsgsgtdftltisrlqpedfatyycqqfnsypltfgggtkleikrtttpap rpptpaptiasqplslrpeacrpaaggavhtrgldfacdiyiwaplagtcgvlllslvitlyckrgrkkllyifkqpfmrpvqttqee dgcscrfpeeeeggcelrvkfsrsadapayqqgqnqlynelnlgrreeydvldkrrgrdpemggkpqrrknpqeglynelqk dkmaeayseigmkgerrrgkghdglyqglstatkdtydalhmqalppr |
| 108 | Claudin18.2-synEphrin B2EC | qvqlqesgpglikpsqtlsltctvsggsissgynwhwirqppgkglewigyihytgstnynpalrsrvtisvdtsknqfslklssv taadtaiyycariyngnsfpywgqgttvtvssggggsggggsggggsdivmtqspdslavslgeratinckssqslfnsgnqk nyltwyqqkpgqppklliywastresgvpdrfsgsgsgtdftltisslqaedvavyycqnaysfpytfgggtkleikrgqdassa gstrnkdptrrpeleagtngrssttspfvkpnpgsstdgnsaghsgnnilgsevalfalmyvaaaafvllffvgcgvllsrkrrrm kllssieqacdicrlkklkcskekpkcakclknnwecryspktkrspltrahltevesrlerleqlfllifpredldmilkmdslqdi kalltglfvqdnvnkdavtdrlasvetdmpltlrqhrisatssseessnkgqrqltvsaaaggsggsggsdalddfdldmlgsdal ddfdldmlgsdalddfdldmlgsdalddfdldmlgs |
| 109 | NKG2D-CAR | Mrvkfsrsadapayqqgqnqlynelnlgrreeydvldkrrgrdpemggkpqrrknpqeglynelqkdkmaeayseigmk gerrrgkghdglyqglstatkdtydalhmqalpprgwirgrrsrhswemsefhnynldlkksdfstrwqkqrcpvvkskcre naspfffccfiavamgirfiimvaiwsavflnslfnqevqipltesycgpcpknwicyknncyqffdesknwyesqascmsq nasllkvyskedqdllklvksyhwmglvhiptngswqwedgsilspnlltiiemqkgdcalyassfkgyiencstpntyicm qrtv |
| 110 | Amino acid sequence of CLL 1 -synEphrinB2EC-APLP2 (TM) | evqlvqsgaevkkpgasvkvsckasgytfnsyymhwvrqapgqglewmgifnpgggstsyaqkfqgrvtmtrdtststvy melsslrsedtavyycargttsdafdiwgqgtmvtvssggggsggggsggggsdiqmtqspsslsasvgdrvtitcrasqsiss ylnwyqqkpgkapklliydvasslqsgvpsrfsgsgsgtdftltisslqpedfatyycqqsystpltfgggtkveikrgqdassags trnkdptrrpeleagtngrssttspfvkpnpgsstdgnsaghsgnnilgsevalfasaligllviavaiatvivislvmlrkrqmkll ssieqacdicrlkklkcskekpkcakclknnwecryspktkrspltrahltevesrlerleqlfllifpredldmilkmdslqdika lltglfvqdnvnkdavtdrlasvetdmpltlrqhrisatssseessnkgqrqltvsaaaggsggsggsdalddfdldmlgsdald dfdldmlgsdalddfdldmlgsdalddfdldmlgs |

## Claims

1. A chimeric polypeptide, comprising:
a) a binding domain capable of specifically binding a target molecule;
b) a receptor regulatory domain comprising one or more cleavage sites,
wherein the receptor regulatory domain comprises an extracellular region and a transmembrane region, which are not both derived from a Notch protein; and
c) an intracellular domain,
wherein binding of the binding domain to the target molecule can induce cleavage of the receptor regulatory domain, thereby releasing the intracellular domain.

2. The chimeric polypeptide according to claim 1, wherein the extracellular region comprises an extracellular region derived from Jagged2, EphrinB2, APLP1, APLP2, APP, CD44, CSF1R, CXCL16, CX3CL1, Delta1, E-cadherin, EphB2, EphrinB1 , Growth hormone receptor, HLA-A2, IFNaR2, IL1R2, L1, LRP, LRP2, LRP6, N-cadherin, Nectin1α, NRADD, p75-NTR, Pcdhα4, Pcdhγ-C3, PTPκ, PTP-LAR, SorCS1b, SorLA, Sortilin, ApoER2, PKHD1, ErbB4, IFNaR2, VEGF-R1, or VLDLR, or a combination thereof, or a fragment of the extracellular region of any one of the above proteins or a combination thereof, or a variant of the extracellular region of any one of the above proteins or a combination thereof, or a truncated structure of the extracellular region of any one of the above proteins or a combination thereof.

3. The chimeric polypeptide according to claim 1 or 2, wherein the transmembrane region further comprises a stop transfer sequence (STS).

4. The chimeric polypeptide according to any one of claims 1-3, wherein the binding domain comprises an antigen binding domain capable of binding to a target molecule on surface of a target cell, or the binding domain comprises a ligand moiety capable of binding to a receptor.

5. The chimeric polypeptide according to claim 4, wherein the target cell is a pathogen.

6. The chimeric polypeptide according to claim 4, wherein the target cell is a human cell.

7. The chimeric polypeptide according to claim 6, wherein the human cell is a tumor cell.

8. The chimeric polypeptide according to any one of claims 1-7, wherein the target molecule is selected from the group consisting of: a differentiation marker cluster, a cell surface receptor, an adhesion protein, an integrin, a mucin, a lectin, and a tumor antigen.

9. The chimeric polypeptide according to any one of claims 1-7, wherein the target molecule is a tumor antigen.

10. The chimeric polypeptide according to any one of claims 1-9, wherein the antigen binding domain is selected from: an antibody, a receptor, a cell adhesion molecule, a non-antibody molecular scaffold, or a combination thereof.

11. The chimeric polypeptide according to claim 10, wherein the antibody is a single-domain antibody, a single-chain antibody, a double-chain antibody, a triple-chain antibody, a miniantibody, a F(ab')₂ fragment, a F(ab) v fragment, scFv, a single domain antibody (sdAb) and a functional fragment thereof, or combinations thereof.

12. The chimeric polypeptide according to any one of claims 4-11, wherein the antigen binding domain specifically binds to a tumor antigen selected from the group consisting of: Mesothelin, FAP, Claudin18.2, CLL1, CD19, GPC3, WT1, EGFR, BCMA, CD7, NKG2D-Ligand, CD19, B7H3, ALPPL2, CD123, CD171, CD179a, CD20, CD213A2, CD22, CD24, CD246, CD272, CD30, CD33, CD38, CD44v6, CD46, CD71, CD97, CEA, CLDN6, CLECL1, CS-1, EGFR, EGFRvIII, ELF2M, EpCAM, EphA2, FLT3, GD2, GD3, GM3, GPRC5D, HER2 (ERBB2), IGLL1, IL 11Ra, IL13Ra2, CD 117, MUC1, NCAM, PAP, PDGFR-b, PRSS21, PSCA, PSMA, ROR1, SIRPa, SSEA-4, TAG72, TEM1/CD248, TEM7R, TSHR, VEGFR2, ALPI, cMet, and Axl.

13. The chimeric polypeptide according to any one of claims 1-12, wherein the transmembrane region of the receptor regulatory domain comprises a γ-secretase cleavage site.

14. The chimeric polypeptide according to claim 1, wherein the intracellular domain comprises a transcription factor, a site-specific nuclease, a recombinase, an inhibitory immune receptor, an activating immune receptor, or a combination thereof.

15. The chimeric polypeptide according to claim 14, wherein the transcription factor is selected from: Gal4-VP16, Gal4-VP64, tetR-VP64, ZFHD1-VP64, Gal4-KRAB, HAP1-VP16 or a combination thereof.

16. The chimeric polypeptide according to any one of claims 1-15, further comprising an additional proteolytic cleavage site, a signal sequence, a detectable label, a tumor-specific cleavage site, a disease-specific cleavage site, and a combination thereof.

17. The chimeric polypeptide according to any one of claims 1-16, wherein the extracellular region of the receptor regulatory domain comprises an amino acid sequence having at least 80% sequence homology to any one of SEQ ID NOs: 1, 3, 5, 7, 9, 11.

18. The chimeric polypeptide according to any one of claims 1-17, wherein the STS comprises an amino acid sequence having at least 80% sequence homology to any one of SEQ ID NOs: 22, 23, 24, 25, 26.

19. The chimeric polypeptide according to any one of claims 1-18, wherein the transmembrane region of the receptor regulatory domain comprises an amino acid sequence having at least 80% sequence homology to any one of SEQ ID NOs: 13, 14, 15, 16, 17, 18, 19, 20, 21, 102, 103, 104.

20. The chimeric polypeptide according to any one of claims 1-19, wherein the receptor regulatory domain comprises an amino acid sequence having at least 80% sequence homology to any one of SEQ ID NOs: 28, 29, 30, 31, 32, 33, 34, 35, 36, 37, 38, 39, 40, 41 or 85.

21. The chimeric polypeptide according to any one of claims 1-20, wherein the chimeric polypeptide comprises an amino acid sequence having at least 80% sequence homology to any one of SEQ ID NOs: 50, 51, 52, 53, 54, 55, 56, 57, 61, 73, 87, 105, 106, 108 or 110.

22. A nucleic acid molecule, comprising a nucleotide sequence encoding the chimeric polypeptide according to any one of claims 1-21.

23. The nucleic acid molecule according to claim 22, wherein the nucleic acid molecule is constructed in an expression cassette or an expression vector.

24. The nucleic acid molecule according to claim 23, wherein the expression vector comprises a viral vector or a transposon vector.

25. The nucleic acid molecule according to claim 24, wherein the viral vector is a lentiviral vector, an adenoviral vector, an adeno-associated viral vector or a retroviral vector.

26. An engineered cell comprising the chimeric polypeptide according to any one of claims 1-21, and/or the nucleic acid molecule according to any one of claims 22-25.

27. The engineered cell according to claim 26, wherein the engineered cell is an immune cell, a neuron, an epithelial cell, an endothelial cell or a stem cell.

28. The engineered cell according to claim 27, wherein the immune cell is a B cell, a monocyte, a natural killer cell, a basophil, an eosinophil, a neutrophil, a dendritic cell, a macrophage, a regulatory T cell, a helper T cell, a cytotoxic T cell, other T cells, or a combination thereof.

29. The engineered cell according to any one of claims 26-28, further comprising an expression cassette encoding an exogenous gene operably linked to the intracellular domain of the chimeric polypeptide, the intracellular domain of the chimeric polypeptide modulats expression of the exogenous gene.

30. The engineered cell according to claim 29, wherein expression of the exogenous gene is regulated by a promoter modulated by GAL-4, tetR, ZFHD1, HNF1A or HAP1.

31. The engineered cell according to claim 29 or 30, wherein an expression product of the exogenous gene is selected from: a non-coding RNA, a cytokine, a cytotoxin, a chemokine, an immunomodulator, a pro-apoptotic factor, an anti-apoptotic factor, a hormone, a differentiation factor, a dedifferentiation factor, a modifying TCR, a CAR, a reporter gene, or a combination thereof.

32. The engineered cell according to claim 31, wherein the binding domain of the chimeric polypeptide specifically binds to a first target molecule, the expression product of the exogenous gene is CAR which specifically binds to a second target molecule which is different from the first target molecule, and the first and second target molecules are respectively selected from the group consisting of: Mesothelin, FAP, Claudin18.2, CLL1, CD19, GPC3, WT1, EGFR, BCMA, CD7, NKG2D-Ligand, MOG, CD19, B7H3, ALPPL2, CD123, CD171, CD179a, CD20, CD213A2, CD22, CD24, CD246, CD272, CD30, CD33, CD38, CD44v6, CD46, CD71, CD97, CEA, CLDN6, CLECL1, CS-1, EGFR, EGFRvIII, ELF2M, EpCAM, EphA2, Ephrin B2 , FAP, FLT3, GD2, GD3, GM3, GPRC5D, HER2 (ERBB2), IGLL1, IL 1Ra, IL13Ra2, CD 117, MUC1, NCAM, PAP, PDGFR-b, PRSS21, PSCA, PSMA, ROR1, SIRPa, SSEA- 4, TAG72, TEM1/CD248, TEM7R, TSHR, VEGFR2, ALPI, cMet and Axl.

33. The engineered cell according to claim 32, wherein the first target molecule and the second target molecule are respectively any one selected from the following combinations: ASGR1 and GPC3, EGFRvIII and IL13Ra2, EGFRvIII and B7H3, Mesothelin and Claudin18.2, Claudin18.2 and Mesothelin, FAP and Claudin18.2, CLL1 and NKG2D, or CD123 and NKG2D.

34. The engineered cell according to claim 32, wherein the chimeric polypeptide and the CAR respectively comprise sequences shown in SEQ ID NOs: 61 and 62; or sequences shown in SEQ ID NOs: 61 and 64; or sequences shown in SEQ ID NOs: 61 and 65, or sequences shown in SEQ ID NOs: 61 and 66, or sequences shown in SEQ ID NOs: 105 and 67, or sequences shown in SEQ ID NOs: 108 and 107, or sequences shown in SEQ ID NOs: 73 and 109, or sequences shown in SEQ ID NOs: 50 and 109.

35. The engineered cell according to claim 31, wherein the cytokine is IL-2, IL-7, IL-9, IL-12, IL-15, IL-18, CCL21, or a combination thereof.

36. The engineered cell according to claim 31, wherein the engineered cell comprises a nucleic acid sequence having at least 80% sequence homology to any one of SEQ ID NOs: 59, 63, 68, 69, 70, 71, 72, 74, 88, or an amino acid sequence translated therefrom.

37. A pharmaceutical composition, comprising: a pharmaceutically acceptable carrier; and the nucleic acid molecule according to any one of claims 22-25 and/or the engineered cell according to any one of claims 26-36.

38. A method for modulating an activity of an engineered cell, comprising:
a) providing the engineered cell according to any one of claims 26-36; and
b) contacting the engineered cell with a target molecule, wherein binding of the target molecule to the binding domain of the chimeric polypeptide on the engineered cell induces cleavage of the proteolytic cleavage site of the chimeric polypeptide and release of the transcription factor in the intracellular domain of the chimeric polypeptide, to modulate the activity of the engineered cell.

39. The method according to claim 38, wherein the activity of the engineered cell is selected from any one of: cell proliferation, cell apoptosis, cell non-apoptotic death, cell differentiation, cell dedifferentiation, cell migration, cell adhesion and/or cytolytic activity.

40. The method according to claim 38, wherein the transcription factor modulates expression of an exogenous gene, which is selected from the group consisting of: a chemokine, a chemokine receptor, a chimeric antigen receptor, a cytokine, a cytokine receptor, a differentiation factor, a growth factor, a growth factor receptor, a hormone, a metabolic enzyme, a pathogen-derived protein, a proliferation inducer, a receptor, a RNA-guided nuclease, a site-specific nuclease, a T-cell receptor, a toxin, a toxin-derived protein, a transcriptional regulator, a transcriptional activator, a transcriptional repressor, a translational regulator, a translational activator, a translational repressor, an activating immune receptor, an antibody, an apoptosis inhibitor, an apoptosis inducer, a modifying T cell receptor, an immune activator, an immunosuppressant, and an inhibitory immune receptor.

41. The method according to claim 38, wherein the released transcription factor regulates differentiation of the engineered cell, which is an immune cell, a stem cell, a progenitor cell or a precursor cell.

42. The method according to claim 38, wherein the method is a method of treating a tumor.

43. A method for activating an engineered cell, comprising:
contacting the engineered cell according to any one of claims 26-36 with a target molecule, wherein the binding domain comprised in the chimeric polypeptide comprises an antibody that specifically binds to a first target molecule, and wherein the contacting results in the release of a transcription factor activating protein in the intracellular domain of the chimeric polypeptide to regulate expression of a CAR and/or modifying TCR in the engineered cell, wherein the engineered cell is activated after the CAR and/or modifying TCR specifically binds to a second target molecule which is different from the first target molecule.

44. The method according to claim 43, wherein the first target molecule and the second target molecule are different tumor antigens; or the first target molecule is a tissue-specific molecule and the second target molecule is a tumor antigen.

45. A method for inhibiting an activity of a target cell in a subject, comprising: administering to the subject a therapeutic effective amount of the engineered cell of any one of claims 26-36, the engineered cell inhibits the activity of the target cell in the subject.

46. The method according to claim 45, wherein the target cell is a tumor cell.

47. The method according to claim 45, wherein the target cell is an acute myeloma leukemia cell, an anaplastic lymphoma cell, an astrocytoma cell, a B cell cancer cell, a breast cancer cell, a colon cancer cell, an ependymoma cell, an esophageal cancer cell, a glioblastoma cell, a glioma cell, a leiomyosarcoma cell, a liposarcoma cell, a hepatoma cell, a lung a cancer cell, a mantle cell lymphoma cell, a melanoma cell, a neuroblastoma cell, a non-small cell lung cancer cell, an oligodendroglioma cell, an ovarian cancer cell, a pancreatic cancer cell, a peripheral T-cell lymphoma cell, a kidney cancer cell, a sarcoma cell, a gastric cancer cell, a liver cancer cell, a mesothelioma cell, or a sarcoma cell.

48. The method according to claim 45, wherein the target cell expresses a low level of the target molecule, and the binding domain of the chimeric polypeptide specifically binds to the target molecule.

49. The method according to claim 45, wherein the engineered cell further comprise a CAR, a modifying TCR, an exogenous cytokine and/or a therapeutic monoclonal antibody whose transcriptional activation is triggered by the chimeric polypeptide binding to a target molecule.

50. The method according to claim 49, wherein the target molecule comprises a first target molecule and a second target molecule, the chimeric polypeptide specifically binds to the first target molecule, and the CAR and/or modifying TCR specifically binds to the second target molecule, the first target molecule and the second target molecule express heterogeneous tumor antigens in the target cell, or the first target molecule is a tissue-specific molecule and the second target molecule is a tumor antigen.

51. The method according to claim 50, wherein the positive rate of the first target molecule in the target cell is lower than the positive rate of the second target molecule in the target cell.

52. The method according to claim 50, wherein the method can further improve the anti-tumor specificity of the engineered cell.

53. A system or medicine box or kit for regulating cell activity, inhibiting a target cell or treating the health condition of a subject in need thereof, comprising: one or more of the following:
a) the chimeric polypeptide according to any one of claims 1-21;
b) the nucleic acid molecule according to any one of claims 22-25;
c) the cell according to any one of claims 26-36; and
d) the pharmaceutical composition according to claim 37.

54. Use of one or more of the following a), b), c), d) in treating a tumor:
a) the chimeric polypeptide according to any one of claims 1-21;
b) the nucleic acid molecule according to any one of claims 22-25;
c) the cell according to any one of claims 26-36; and
d) the composition according to claim 37.

55. The use according to claim 54, wherein the tumor is a solid tumor, a blood tumor, a soft tissue tumor or a metastatic lesion.

56. The use of the chimeric polypeptide according to any one of claims 1-21, the nucleic acid molecule according to any one of claims 22-25, the cell according to any one of claims 26-36 and the pharmaceutical composition according to claim 37 in the manufacture of a medicament for treating health condition.
